# EUROPEAN PATENT APPLICATION

(11) **EP 2 495 323 A1**
(43) Date of publication of application: **05.09.2012**
(21) Application number: 10826845.9
(22) Date of filing: 29.10.2010
(51) Int. Cl.: C12N 15/113, A61K 31/713, A61K 48/00, A61P 43/00, C07H 21/02, C07H 21/04

(54) **MODIFIED DOUBLE-STRANDED POLYNUCLEOTIDE**

(30) Priority: 30.10.2009 JP 2009249998; 28.12.2009 JP 2009298719
(71) Applicant: Daiichi Sankyo Company, Limited, Toyko 103-8426 (JP)
(72) Inventor: KOIZUMI, Makoto, Tokyo 134-8630 (JP); HIROTA, Yasuhide, Tokyo 134-8630 (JP)
(74) Representative: Arends, William Gerrit
(86) International application number: PCT/JP2010/069266
(87) International publication number: WO 2011/052715

(57) **Abstract**

The present invention provides a double-stranded polynucleotide having a sense strand polynucleotide consisting of a nucleotide sequence complementary to a target sequence in a target gene, and an antisense strand polynucleotide having a nucleotide sequence complementary to the sense strand polynucleotide, wherein an aryl or heteroaryl compound is bound to a phosphate group at the 5'-end of the antisense strand polynucleotide.

## Description

### Technical Field

The present invention relates to a double-stranded polynucleotide that has an RNA interference effect and/or a gene expression inhibitory effect, use of the double-stranded polynucleotide, a method for inhibiting gene expression using the double-stranded polynucleotide, a pharmaceutical composition comprising the double-stranded polynucleotide, etc.

### Background Art

Methods for inhibiting the expression of a target gene in cells, tissues, or individuals include an approach in which double-stranded RNA is introduced into the cells, tissues, or individuals. By this introduction of double-stranded RNA, mRNA having homology to the sequence is degraded such that the expression of the target gene is inhibited. This effect is called "RNA interference" or "RNAi". RNA interference was originally reported in *C.elegans* (see e.g., Non Patent Reference 1) and then also reported in plants (see e.g., Non Patent Reference 2).

Double-stranded RNA consisting of 21-nucleotide sense and antisense strands having a 2-nucleotide overhang at the 3'-end (small interfering RNA: siRNA) has been reported to have an RNA interference effect in cultured cells of vertebrates (see e.g., Non Patent Reference 3) . siRNA is considered to be useful for the identification of gene functions, screening of cell strains suitable for useful substance production, regulation of genes involved in disease, etc., but, however, it is predisposed to be degraded easily by RNase (see e.g., Non Patent Reference 4).

Some reports have been made on the modification of the 5'-ends of sense and antisense strands in siRNA. It has been reported that siRNA having a 6-aminohexyl phosphate group at the 5'-end of the sense or antisense strand has inhibitory activity against the expression of target mRNA (see Non Patent Reference 5). On the other hand, it has been reported that siRNA having this 6-aminohexyl phosphate group at the 5'-end of the antisense strand has no inhibitory activity against the expression of target mRNA (see e.g., Non Patent Reference 6). It has also been reported that siRNA having a 3-aminopropyl phosphate group at the 5'-end of the sense strand has inhibitory activity against the expression of target mRNA, whereas siRNA having a 3-aminopropyl phosphate group at the 5'-end of the antisense strand has no inhibitory activity against the expression of target mRNA (see e.g., Non Patent Reference 7). It has been reported that inhibitory activity against the expression of target mRNA in siRNA having the 6-aminohexyl phosphate group or the 3-aminopropyl phosphate group at the 5'-end of the antisense strand is observably lower than that in unmodified siRNA, but is not completely lost. (see e.g., Non Patent Reference 8).

It has been reported that siRNA having fluorescein at the 5'-end of the sense or antisense strand also has inhibitory activity against the expression of target mRNA (see e.g., Non Patent Reference 9). It has been reported that of siRNAs having a steroid or lipid structure at the 5'-end of the sense or antisense strand, siRNA having a steroid or lipid structure at the 5'-end of the sense strand has inhibitory activity against the expression of target mRNA (see e.g., Non Patent Reference 8). It has been reported that when siRNA has an ortho-nitrobenzyl derivative, which can be eliminated by UV irradiation, at the 5'-end of the antisense strand, its inhibitory activity against the expression of target mRNA can be controlled using UV irradiation (see e.g., Non Patent Reference 10).

The present inventors have conducted diligent studies to obtain a polynucleotide that has an RNA interference effect and/or a gene expression inhibitory effect, and have consequently completed the present invention by finding a double-stranded polynucleotide having an RNA interference effect and/or a gene expression inhibitory effect in which an aromatic substituent (in the present specification, also abbreviated as "Ar") is bound to the 5'-end of an antisense strand in a double-stranded polynucleotide.

### Reference

### Non Patent Reference

Non Patent Reference 1: Nature, 1998, Vol. 391, p. 806-811
Non Patent Reference 2: Science, 1999, Vol. 286, p. 950-952
Non Patent Reference 3: Nature, 2001, Vol. 411, p. 494-498
Non Patent Reference 4: Clinical Chemistry, 2002, Vol. 48, p. 1647-1653
Non Patent Reference 5: Molecular Cell, 2002, Vol. 10, p. 537-548
Non Patent Reference 6: Nucleic Acids Research, 2003, Vol. 31, p. 2705-2716
Non Patent Reference 7: Molecular Cell, 2002, Vol. 10, p. 549-561
Non Patent Reference 8: Oligonucleotides, 2007, Vol. 176, p. 35-43
Non Patent Reference 9: Antisense Nucleic Acid Drug Development, 2003, Vol. 13, p. 83-105
Non Patent Reference 10: Biochimica Biophysica Acta, 2006, Vol. 1758, p. 394-403

### Disclosure of the Invention

### Object of the Invention

An object of the present invention is to provide a double-stranded polynucleotide that has an RNA interference effect and/or a gene expression inhibitory effect.

A further object of the present invention is to provide a double-stranded polynucleotide that is resistant to RNase and has an RNA interference effect and/or a gene expression inhibitory effect.

A further object of the present invention is to provide a method for inhibiting gene expression using the double-stranded polynucleotide.

A further object of the present invention is to provide a pharmaceutical composition comprising the double-stranded polynucleotide.

### Means for Achieving the Object

Specifically, the present invention consists of:
(1) A double-stranded polynucleotide, or a salt thereof, having a sense strand polynucleotide consisting of a nucleotide sequence complementary to a target sequence in a target gene, and an antisense strand polynucleotide having a nucleotide sequence complementary to the sense strand polynucleotide, wherein a substituent represented by X is bound to a phosphate group at the 5'-end of the antisense strand polynucleotide to form a phosphodiester structure, wherein
   X represents:
   (a) a substituent represented by the formula (I):

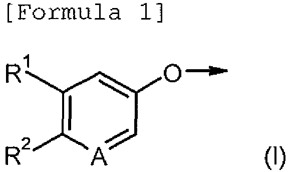

wherein A represents a nitrogen atom or C-R³,
R¹ and R² each independently represent
a hydrogen atom,
an alkyl group having 1 to 8 carbon atoms and optionally having a substituent,
an alkoxy group having 1 to 8 carbon atoms and optionally having a substituent,
a cycloalkyl group having 3 to 6 carbon atoms and optionally having a substituent,
a halogen atom,
an alkylcarbonyl group containing an alkyl group having 1 to 8 carbon atoms and optionally having a substituent,
a phenyl group optionally having a substituent,
a phenyloxy group optionally having a substituent,
a saturated or unsaturated 5- or 6-membered heterocyclic group containing 1 to 3 heteroatoms selected from the heteroatom group consisting of a nitrogen atom, an oxygen atom, and a sulfur atom and optionally having a substituent,
an aralkyl group optionally having a substituent in the phenyl group moiety,
an aralkyloxy group optionally having a substituent in the phenyl group moiety,
an alkylsulfonyl group having 1 to 6 carbon atoms, a hydroxy group,
an alkylcarbonylamino group having an alkyl group having 1 to 9 carbon atoms,
a hydroxyalkylcarbonylamino group having an alkyl group having 1 to 9 carbon atoms wherein the alkyl group is substituted by a hydroxy group on the terminal carbon atom thereof,
an N-alkylcarbamoyl group having an alkyl group having 1 to 8 carbon atoms,
an N-(hydroxyalkyl)carbamoyl group having an alkyl group having 1 to 9 carbon atoms wherein the alkyl group is substituted by a hydroxy group on the terminal carbon atom thereof,
an N-alkylcarbamoylalkylene group having an alkyl group having 1 to 8 carbon atoms and being bonded to the aromatic ring via an alkylene group containing 1 to 4 carbon atoms,
an N-(hydroxyalkyl)carbamoylalkylene group having an alkyl group having 1 to 9 carbon atoms wherein the alkyl group is substituted by a hydroxy group on the terminal carbon atom thereof, and being bonded to the aromatic ring via an alkylene group containing 1 to 4 carbon atoms, a carboxy group, or
a group represented by the formula: -(CH₂)ₖ-CONR⁴R⁵, wherein R⁴ and R⁵ each independently represent a hydrogen atom, an alkyl group having 1 to 6 carbon atoms and optionally having a substituent, or an aralkyl group optionally having a substituent in the phenyl group moiety, and k represents an integer of 0 to 3, or R¹ and R² are optionally combined to form, together with the aromatic ring bonded to R¹ and R², a saturated or unsaturated bicyclic or tricyclic structure optionally having a substituent, optionally further containing 1 or 1 or more heteroatoms as constituent atoms in the ring, and optionally having an oxo group, and
R³ represents
a halogen atom,
an alkyl group having 1 to 6 carbon atoms,
an alkoxy group having 1 to 6 carbon atoms,
a halogenomethyl group,
a hydroxy group, or
a hydrogen atom; or
(b) a tyrosine residue optionally having a substituent on the amino group wherein a hydroxy group on the phenyl group is the phosphate group binding site;
   (2) The double-stranded polynucleotide or salt thereof according to (1), wherein in formula (I), A is C-R³;
   (3) The double-stranded polynucleotide or salt thereof according to (2), wherein R³ is a hydrogen atom;
   (4) The double-stranded polynucleotide or salt thereof according to any one of (1) to (3), wherein the sense strand polynucleotide consists of a polynucleotide represented by the following formula (II), the antisense strand polynucleotide consists of a polynucleotide represented by the following formula (III), and the double-stranded polynucleotide further has the following features (a) to (d):

      5'- (γ-β)₉-γ-λₘ-3' (II)

      5'-X-β-(γ-β)₉-υₙ-3' (III),
(a) γ represents an RNA, β represents a 2'-OMeRNA, and λ and υ each represent a DNA;
(b) m and n identically or differently represent any integer from 0 to 5;
(c) (γ-β)₉-γ in the polynucleotide represented by formula (II) has a nucleotide sequence identical to the target gene; and
(d) (γ-β)₉-γ in formula (II) and β-(γ-β)₉ in formula (III) have nucleotide sequences complementary to each other;
   (5) The double-stranded polynucleotide or salt thereof according to any one of (1) to (3), wherein the sense strand polynucleotide consists of a polynucleotide represented by the following formula (IV), the antisense strand polynucleotide consists of a polynucleotide represented by the following formula (V), and the double-stranded polynucleotide further has the following features (a) to (d):

      5'-(α-β)₉-αₚ-λₘ-3' (IV)

      51 -X-δₛ- (α-β)₉-υₙ-3' (V),
(a) α and β differently represent a DNA or a 2'-OMeRNA, δ and λ identically or differently represent a DNA or a 2'-OMeRNA, and u identically or differently represents any nucleotide selected from a DNA, an RNA, and a 2'-OMeRNA;
(b) p represents an integer of 0 or 1, m is 0 when p is 0 and represents any integer from 0 to 5 when p is 1, s represents an integer of 0 or 1, and n represents any integer from 0 to 5;
(c) (α-β)₉-αₚ in the polynucleotide represented by formula (IV) has a nucleotide sequence identical to the target gene; and
(d) (α-β) in formula (IV) and (α-β)₉ in formula (V) have nucleotide sequences complementary to each other;
   (6) The double-stranded polynucleotide or salt thereof according to any one of (1) to (3), wherein the sense strand polynucleotide consists of a polynucleotide represented by the following formula (VI), the antisense strand polynucleotide consists of a polynucleotide represented by the following formula (VII), and the double-stranded polynucleotide further has the following features (a) to (d):

      5'-β-(α-β)₈-αₚ-λₘ-3' (VI)

      5'-X-δₛ-(α-β)₈-(α-β)-υₙ-3' (VII),
(a) α and β differently represent a DNA or a 2'-OMeRNA, δ and λ identically or differently represent a DNA or a 2'-OMeRNA, and u identically or differently represents any nucleotide selected from a DNA, an RNA, and a 2'-OMeRNA;
(b) p represents an integer of 0 or 1, m is 0 when p is 0 and represents any integer from 0 to 5 when p is 1, s represents an integer of 0 or 1, and n represents any integer from 0 to 5;
(c) β-(α-β)₈-αₚ in the polynucleotide represented by formula (VI) has a nucleotide sequence identical to the target gene; and
(d) (α-β)₈ in formula (VI) and (α-β)₈ in formula (VII) have nucleotide sequences complementary to each other;
   (7) The double-stranded polynucleotide or salt thereof according to (5) or (6), wherein α is a DNA, and β is a 2'-OMeRNA;
   (8) The double-stranded polynucleotide or salt thereof according to any one of (4) to (7), wherein λₘ and υₙ are identically or differently any of: DNAs having a thymine base, an adenine base, or a guanine base; or 2'-OMeRNAs having a uracil base, an adenine base, or a guanine base;
   (9) The double-stranded polynucleotide or salt thereof according to any one of (4) to (8), wherein m is 0, and n is 2;
   (10) The double-stranded polynucleotide or salt thereof according to any one of (5) to (9), wherein p and m are 0, s is 1, and n is 2;
   (11) The double-stranded polynucleotide or salt thereof according to any one of (5) to (9), wherein p and m are 0, s is 0 or 1, n is 2, and υ₂ is a DNA or a 2'-OMeRNA;
   (12) The double-stranded polynucleotide or salt thereof according to any one of (4) to (11), wherein between 1 and 4 2'-OMeRNA nucleotides are substituted by an ENA or a 2',4'-BNA/LNA;
   (13) The double-stranded polynucleotide or salt thereof according to any one of (4) to (12), wherein between 1 and 4 DNA nucleotides are substituted by an RNA, an ENA or a 2',4'-BNA/LNA;
   (14) The double-stranded polynucleotide or salt thereof according to any one of (1) to (13), wherein the nucleotides are bonded to each other via a phosphodiester or phosphorothioate bond;
   (15) A pharmaceutical composition comprising a double-stranded polynucleotide or salt thereof according to any one of (1) to (14) as an active ingredient;
   (16) A method for inhibiting the expression of a target gene, comprising administering a double-stranded polynucleotide or salt thereof selected from (1) to (15) to a mammal.

### Effects of the Invention

The present invention has provided a double-stranded polynucleotide that has an RNA interference effect and/or a gene expression inhibitory effect. The present invention has also provided a double-stranded polynucleotide that is resistant to at least one enzyme selected from RNase, phosphatase, and exonuclease and has an RNA interference effect and/or a gene expression inhibitory effect. The present invention has further provided a double-stranded polynucleotide that is resistant to RNase, phosphatase, and exonuclease and has an RNA interference effect and/or a gene expression inhibitory effect. The present invention allows functional analysis of various genes using the polynucleotide and provides a pharmaceutical composition comprising the double-stranded polynucleotide.

The present invention allows functional analysis of various genes using the polynucleotide and provides a pharmaceutical composition comprising the double-stranded polynucleotide.

### Brief Description of Drawings

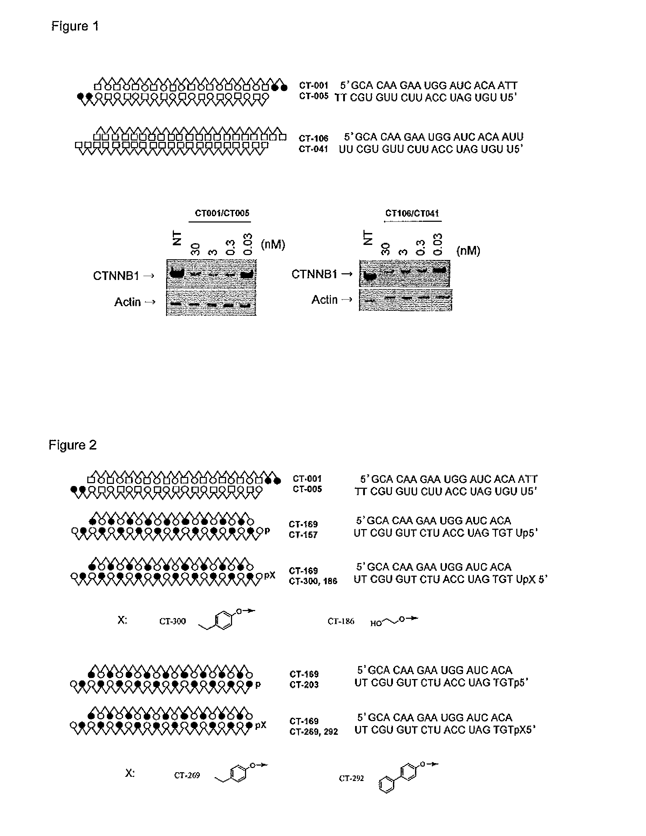

[Figure 1] Figure 1 is a diagram showing double-stranded polynucleotides against the human β-catenin gene (hereinafter, examples of combinations of polynucleotides as sense and antisense strands are shown in each diagram; as symbols, an open square (□) represents an RNA, a filled circle (●) represents a DNA, an open circle (O) represents a 2'-O-methyl RNA, and an open rhombus (◇) represents an ENA. In the diagram, p represents a phosphate group, s represents a phosphate group or a phosphodiester bond in a thiophosphate group or a phosphorothioate bond, respectively. The same holds true for the diagrams shown below).
[Figure 2] Figure 2 is a diagram showing double-stranded polynucleotides against the human β-catenin gene. The structure of X in the sequences is also shown. The same holds true for the diagrams shown below.

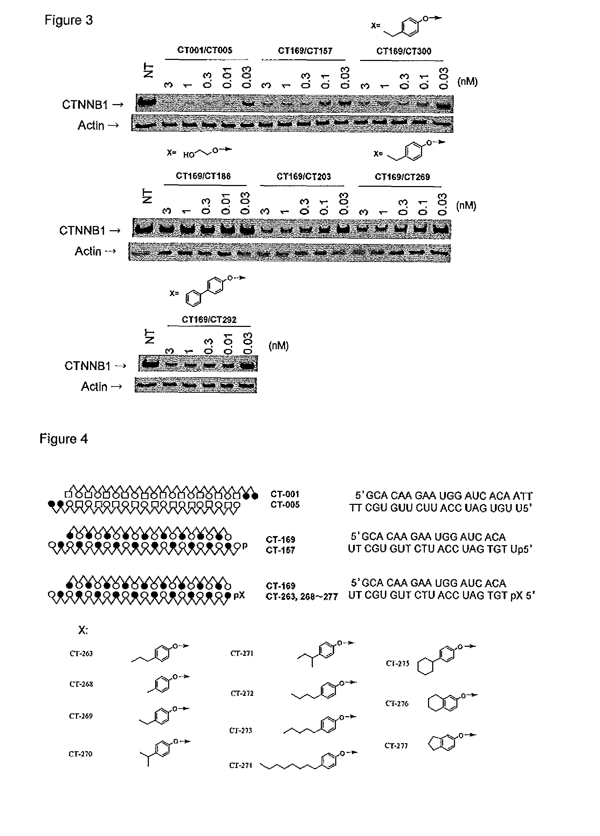

[Figure 3] Figure 3 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene. In the diagram, the notation "CT001/CT005" means "CT-001/CT-005"; thus, the "hyphen: -" in the notations of double-stranded polynucleotides may be omitted. The structure of a compound bound to the 5'-end of the antisense strand is also shown. The same holds true for the diagrams shown below.
[Figure 4] Figure 4 is a diagram showing double-stranded polynucleotides against the human β-catenin gene.
[Figure 5] Figure 5 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 6] Figure 6 is a diagram showing double-stranded polynucleotides against the human β-catenin gene.
[Figure 7] Figure 7 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 8] Figure 8 is a diagram showing double-stranded polynucleotides against the human β-catenin gene.
[Figure 9] Figure 9 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 10] Figure 10 is a diagram showing double-stranded polynucleotides against the human β-catenin gene.
[Figure 11] Figure 11 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 12] Figure 12 is a diagram showing the effect of phosphatase on CT-203, and a liquid chromatogram of CT-203 (A), a chromatogram after phosphatase treatment (B), and a chromatogram of CT-328 (C).
[Figure 13] Figure 13 is a diagram showing the effect of phosphatase on CT-292, and a liquid chromatogram of CT-292 (A), a chromatogram after phosphatase treatment (B), and a chromatogram of CT-328 (C).
[Figure 14] Figure 14 is a diagram showing the effect of 5'-3'-exonuclease on CT-203, and liquid chromatograms of the sample at reaction times of 0 minutes (A), 15 minutes (B), 30 minutes (C), and 45 minutes (D).
[Figure 15] Figure 15 is a diagram showing the effect of 5'-3'-exonuclease on CT-292, and liquid chromatograms of the sample at reaction times of 0 minutes (A), 15 minutes (B), 30 minutes (C), and 45 minutes (D).
[Figure 16] Figure 16 is a diagram showing double-stranded polynucleotides against the human β-catenin gene.
[Figure 17] Figure 17 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 18] Figure 18 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 19] Figure 19 is a diagram showing double-stranded polynucleotides against the human β-catenin gene.
[Figure 20] Figure 20 is a diagram showing the outline of Method A.
[Figure 21] Figure 21 is a diagram showing the outline of Method B.
[Figure 22] Figure 22 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 23] Figure 23 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 24] Figure 24 is a diagram showing double-stranded polynucleotides against the human β-catenin gene.
[Figure 25] Figure 25 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 26] Figure 26 is a diagram showing double-stranded polynucleotides against the human β-catenin gene.
[Figure 27] Figure 27 is a diagram showing double-stranded polynucleotides against the MCL1 gene.
[Figure 28] Figure 28 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 29] Figure 29 is a diagram showing double-stranded polynucleotides against the human β-catenin gene.
[Figure 30] Figure 30 is a diagram showing the results of western blot analysis of the intensities of inhibitory activities of various double-stranded polynucleotides on the expression of Myeloid Cell Leukemia Sequence 1 (MCL1) gene. MCL1 represents the expression of MCL1 proteins, and CTNNB1 represents the expression of CTNNB1 proteins used as a control. The number represents the concentration of the double-stranded polynucleotide added. A lighter-colored band means stronger inhibitory activity on the expression of the MCL1 gene.
[Figure 31] Figure 31 is a diagram showing double-stranded polynucleotides against the human β-catenin gene.
[Figure 32] Figure 32 is a diagram showing double-stranded polynucleotides against the human β-catenin gene.
[Figure 33] Figure 33 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 34] Figure 34 is a diagram showing double-stranded polynucleotides against the human β-catenin gene.
[Figure 35] Figure 35 is a diagram showing the results of western blot analysis of the intensities of inhibitory activities of various double-stranded polynucleotides on the expression of the human β-catenin gene. CTNNB1 represents the expression of human β-catenin proteins, and Actin represents the expression of β-actin proteins used as a control. The number represents the concentration of the double-stranded polynucleotide added. A lighter-colored band means stronger inhibitory activity on the expression of the human β-catenin gene.
[Figure 36] Figure 36 is a diagram showing the structures of compounds described in Reference Examples 22 to 33, 36, and 40 to 42.
[Figure 37] Figure 37 is a diagram showing double-stranded polynucleotides against the human β-catenin gene.
[Figure 38] Figure 38 is a diagram showing the gene inhibitory activities of double-stranded polynucleotides analyzed by real-time PCR.
[Figure 39] Figure 39 is a diagram showing double-stranded polynucleotides against the human β-catenin gene.
[Figure 40] Figure 40 is a diagram showing the gene inhibitory activities of double-stranded polynucleotides analyzed by real-time PCR.

### Description of Embodiments

### 1. Description of terms

In the present specification, the "target gene" is not particularly limited as long as it can be translated to produce mRNA and/or protein in cells, tissues, or individuals to which or to whom this gene is introduced (hereinafter, they may be referred to as "recipients"). Specifically, the target gene may be endogenous to the recipients for introduction or may be exogenous and introduced thereto by a method such as gene transfer. It may also be a gene present on the chromosome or an extrachromosomal gene. Examples of the exogenous gene include, but are not limited to, those derived from viruses, bacteria, fungi, and protozoans, which can infect the recipients. The function of a gene may be known or unknown.

Examples of such a target gene can include genes whose expression is specifically increased and/or which are specifically mutated in patients having a particular disease. Examples of the disease can include central nervous system disease (e.g., Alzheimer's disease, dementia, and eating disorders), inflammatory disease (e.g., allergy, rheumatism, osteoarthritis, and lupus erythematosus), cardiovascular disease (e.g., hypertension, cardiomegaly, angina pectoris, and arteriosclerosis), cancer (e.g., non-small cell lung cancer, ovarian cancer, prostatic cancer, gastric cancer, bladder cancer, breast cancer, uterine cervix cancer, colon cancer, and rectal cancer), respiratory disease (e.g., pneumonia, bronchitis, asthma, and pulmonary fibrosis), diabetes mellitus, immunological disease (e.g., Crohn's disease, atopic dermatitis, autoimmune disease, immunodeficiency, and leukemia), liver/gallbladder disease (e.g., liver cirrhosis, hepatitis, liver failure, cholestasis, and calculus), gastrointestinal disease (e.g., an ulcer, enteritis, and malabsorption), infection, and adiposity. Examples of causative genes of these diseases can include, but are not limited to, kinesin spindle protein (KSP), vascular endothelial growth factor, (VEGF), transthyretin (TTR), proprotein convertase subtilisin/kexin type 9 (PCSK9), polo-like kinase(PLK), ApoB-100, ribonucleotide reductase M2 subunit (RRM2), clusterin, heat shock protein 27 (Hsp27), survivin, eukaryotic initiation factor-4E (eIF-4E), intercellular adhesion molecule 1 (ICAM-1), alpha subunit of the interleukin 4 receptor (IL-4R-alpha), Factor XI, Factor VII, N-ras, H-ras, K-ras, bcl-2, bc1-xL, Her-1, Her-2, Her-3, Her-4, MDR-1, human β-catenin gene, DDX3(DEAD (Asp-Glu-Ala-Asp) box polypeptide 3, X-linked), Myeloid Cell Leukemia Sequence 1 (MCL1) gene

In the present specification, the term "natural nucleoside" refers to a 2'-deoxynucleoside such as 2'-deoxyadenosine, 2'-deoxyguanosine, 2'-deoxycytidine, 2'-deoxy-5-methylcytidine, and thymidine or a ribonucleoside such as adenosine, guanosine, cytidine, 5-methylcytidine, and uridine. Moreover, the term "oligonucleotide" refers to an oligonucleotide composed of a compound in which the sugar moiety of the nucleoside forms an ester with phosphoric acid.

In the present specification, 2'-deoxyadenosine may be referred to as A^{t}; 2'-deoxyguanosine may be referred to as G^{t}; 2'-deoxycytidine may be referred to as C^{t}; 2'-deoxy-5-methylcytidine may be referred to as 5meC^{t}; thymidine may be referred to as T^{t}; 2'-deoxyuridine may be referred to as U^{t}; adenosine may be referred to as A^{rt}; guanosine may be referred to as G^{rt}; cytidine may be referred to as C^{rt}; 5-methylcytidine may be referred to as SmeG^{rt}; and uridine may be referred to as U^{rt}. Moreover, in the present specification, 2'-deoxyadenosine nucleotide may be referred to as A^{p}; 2'-deoxyguanosine nucleotide may be referred to as G^{p}; 2'-deoxycytidine nucleotide may be referred to as C^{p}; 2'-deoxy-5-methylcytidine nucleotide may be referred to as 5meC^{p}; a thymidine nucleotide may be referred to as T^{p}; a 2'-deoxyuridine nucleotide may be referred to as U^{p}; an adenosine nucleotide may be referred to as A^{rp}; a guanosine nucleotide may be referred to as G^{rp}; a cytidine nucleotide may be referred to as C^{rp}; a 5-methylcytidine nucleotide may be referred to as 5meC^{rp}; and a uracil nucleotide may be referred to as U^{rp}.

In the present specification, where there are phosphorothioate ester forms instead of phosphoester forms of a nucleotide, a counterpart of A^{p} may be referred to as A^{s}; a counterpart of G^{p} may be referred to as G^{s}; a counterpart of C^{p} may be referred to as C^{s}; a counterpart of 5meC^{p} may be referred to as 5meC^{s}; a counterpart of T^{p} may be referred to as T^{s}; a counterpart of U^{p} may be referred to as U^{s}; a counterpart of A^{rp} may be referred to as A^{rs}; a counterpart of G^{rp} may be referred to as G^{rs}; a counterpart of C^{rp} may be referred to as C^{rs}; a counterpart of 5meC^{rp} may be referred to as 5meC^{rs}; and a counterpart of U^{rp} may be referred to as C^{rs}.

In the present specification, the term "sugar-modified nucleoside" refers to a nucleoside whose sugar moiety has been modified.

As particular examples of 2'-O-methyl modification, a counterpart of A^{rt} may be referred to as A^{m1t}; a counterpart of G^{rt} may be referred to as G^{m1t}; a counterpart of C^{rt} may be referred to as C^{m1t}; a counterpart of 5meC^{rt} may be referred to as 5meC^{m1t}; a counterpart of U^{rt} may be referred to as U^{m1t}; a counterpart of A^{rp} may be referred to as A^{m1p}; a counterpart of G^{rp} may be referred to as G^{m1p}; a counterpart of C^{rp} may be referred to as C^{m1p}; a counterpart of 5meC^{rp} may be referred to as 5meC^{m1p}; a counterpart of U^{rp} may be referred to as U^{m1p}; a counterpart of A^{rs} may be referred to as A^{m1s}; a counterpart of G^{rs} may be referred to as G^{m1s}; a counterpart of C^{rs} may be referred to as C^{m1s}; a counterpart of 5meC^{s} may be referred to as 5meC^{m1s}; and a counterpart of U^{rs} may be referred to as U^{m1s}.

In the present specification, the 2'-O,4'-C-ethylene nucleotide unit and the "ENA unit" refer to those nucleosides and nucleotides having an ENA and also refer to nucleosides and nucleotides having an ENA unit: a counterpart of A^{t} may be referred to as A^{2t}; a counterpart of A^{p} may be referred to as A^{e2p}; a counterpart of A^{s} may be referred to as A^{e2s}; a counterpart of G^{t} may be referred to as G^{2t}; a counterpart of G^{p} may be referred to as G^{e2p}; a counterpart of G^{s} may be referred to as G^{e2s}; a counterpart of 5meC^{t} may be referred to as C^{2t}; a counterpart of 5meC^{p} may be referred to as C^{e2p}; a counterpart of 5meC^{s} may be referred to as C^{e2s}; a counterpart of T^{t} may be referred to as T^{2t}; a counterpart of T^{p} may be referred to as T^{e2p}; and a counterpart of T^{s} may be referred to as T^{e2s}.

In the present specification, the 2'-O,4'-C-methylene nucleotide unit and the "2',4'-BNA/LNA unit" refer to those nucleosides and nucleotides having a 2',4'-BNA/LNA and also refer to nucleosides and nucleotides having a 2',4'-BNA/LNA unit: a counterpart of A^{t} may be referred to as A^{1t}; a counterpart of A^{p} may be referred to as A^{e1p}; a counterpart of A^{s} may be referred to as A^{e1s}; a counterpart of G^{t} may be referred to as G^{1t}; a counterpart of G^{p} may be referred to as G^{e1p}; a counterpart of G^{s} may be referred to as G^{e1s}; a counterpart of 5meC^{t} may be referred to as C^{1t}; a counterpart of 5meC^{p} may be referred to as C^{e1p}; a counterpart of 5meC^{s} may be referred to as C^{e1s}; a counterpart of T^{t} may be referred to as T^{1t}; a counterpart of T^{p} may be referred to as T^{e1p}; and a counterpart of T^{s} may be referred to as T^{e1s}.

Hereinafter, the structural formula of each nucleotide is shown.

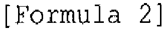

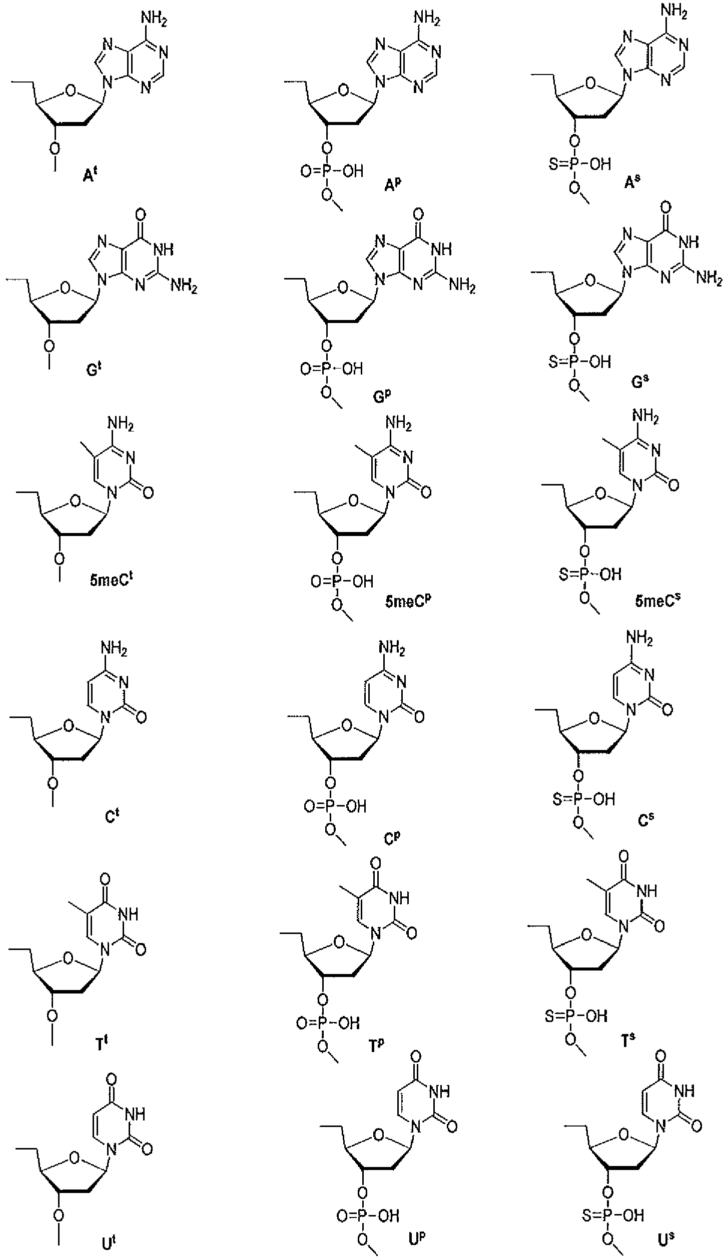

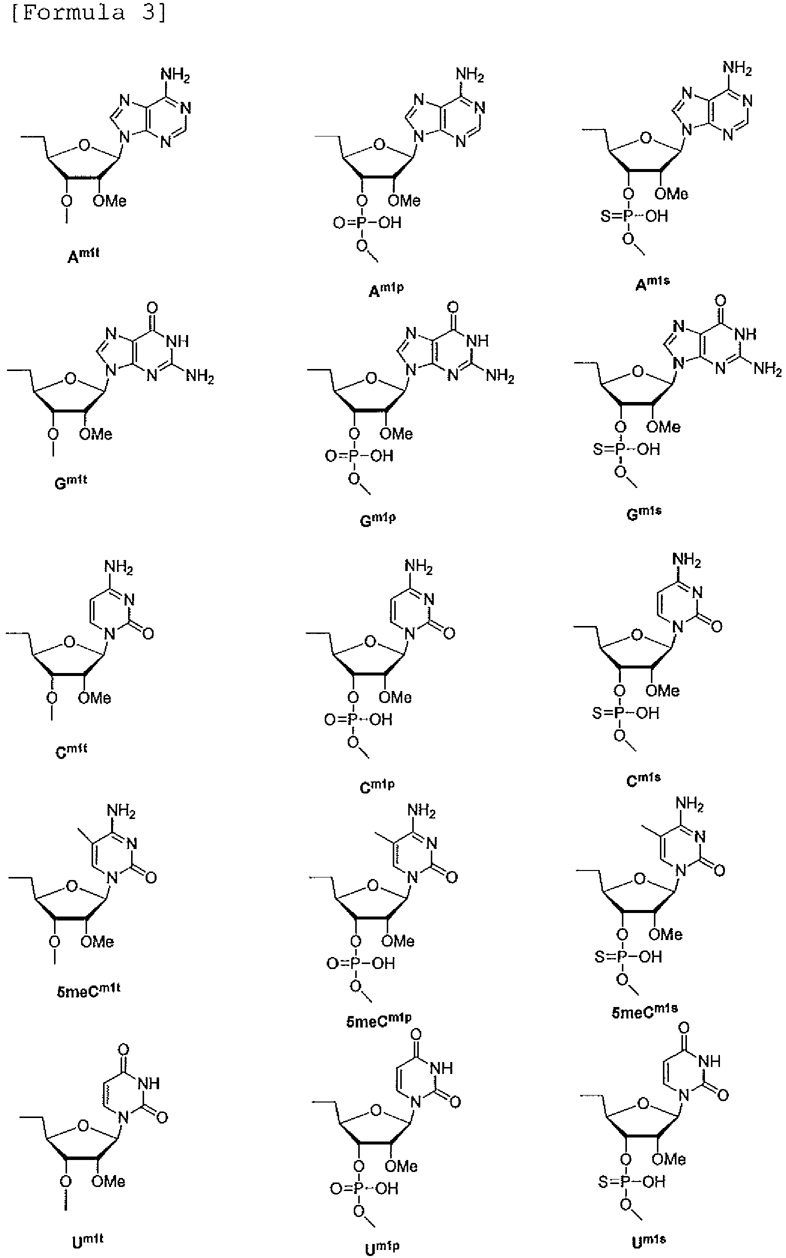

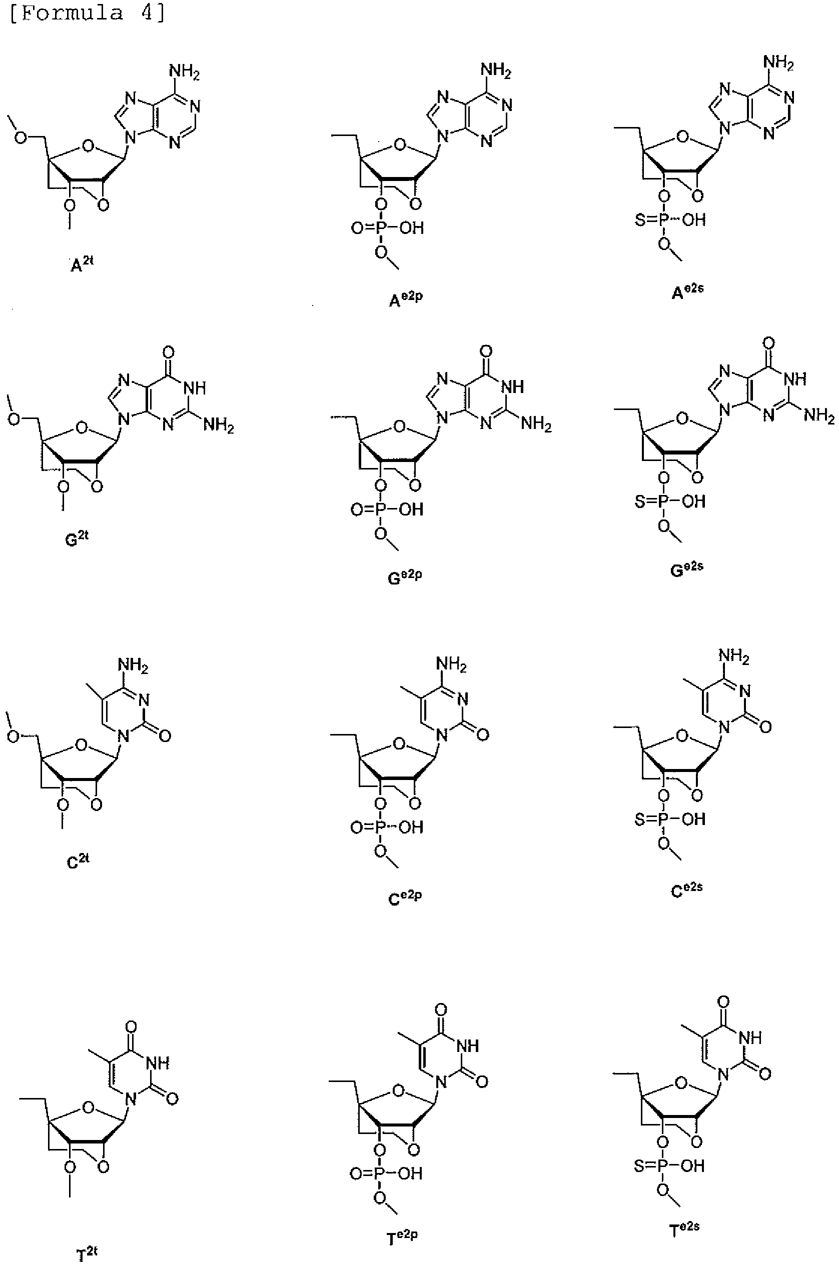

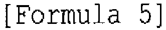

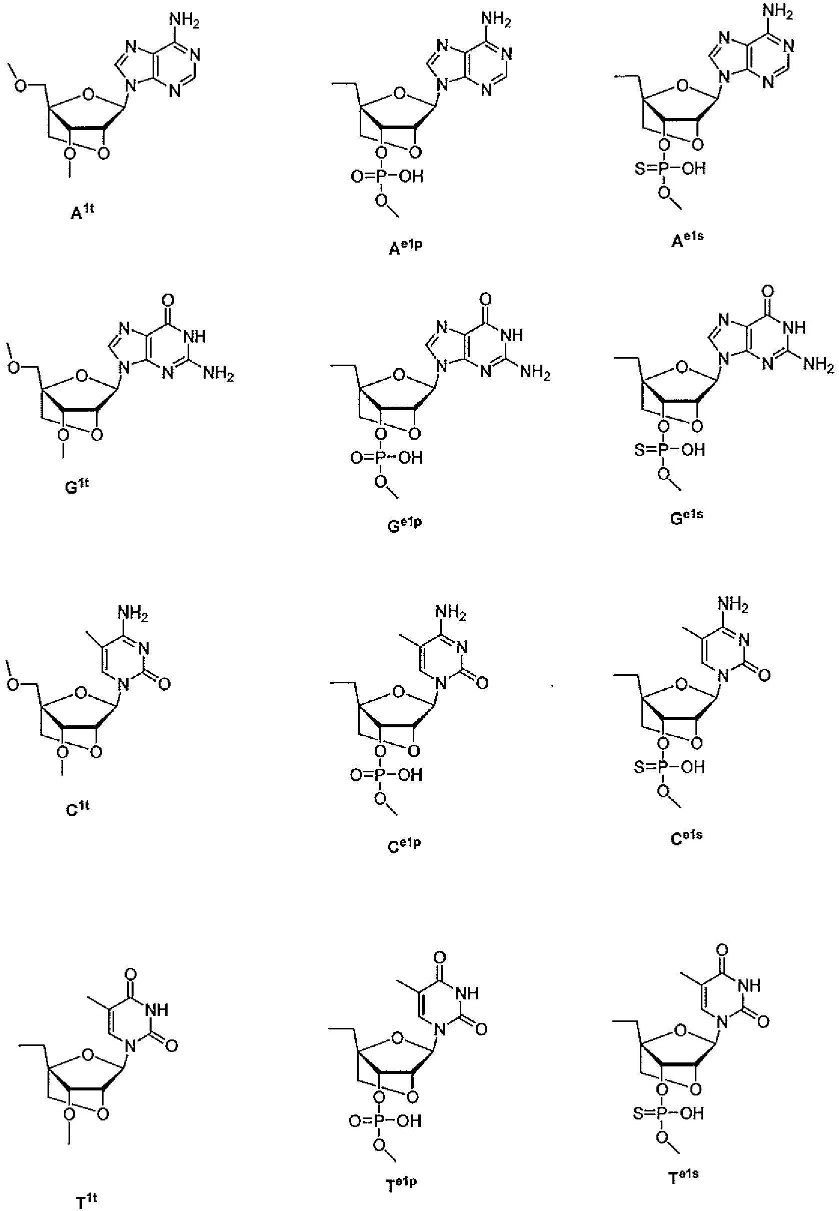

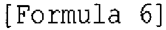

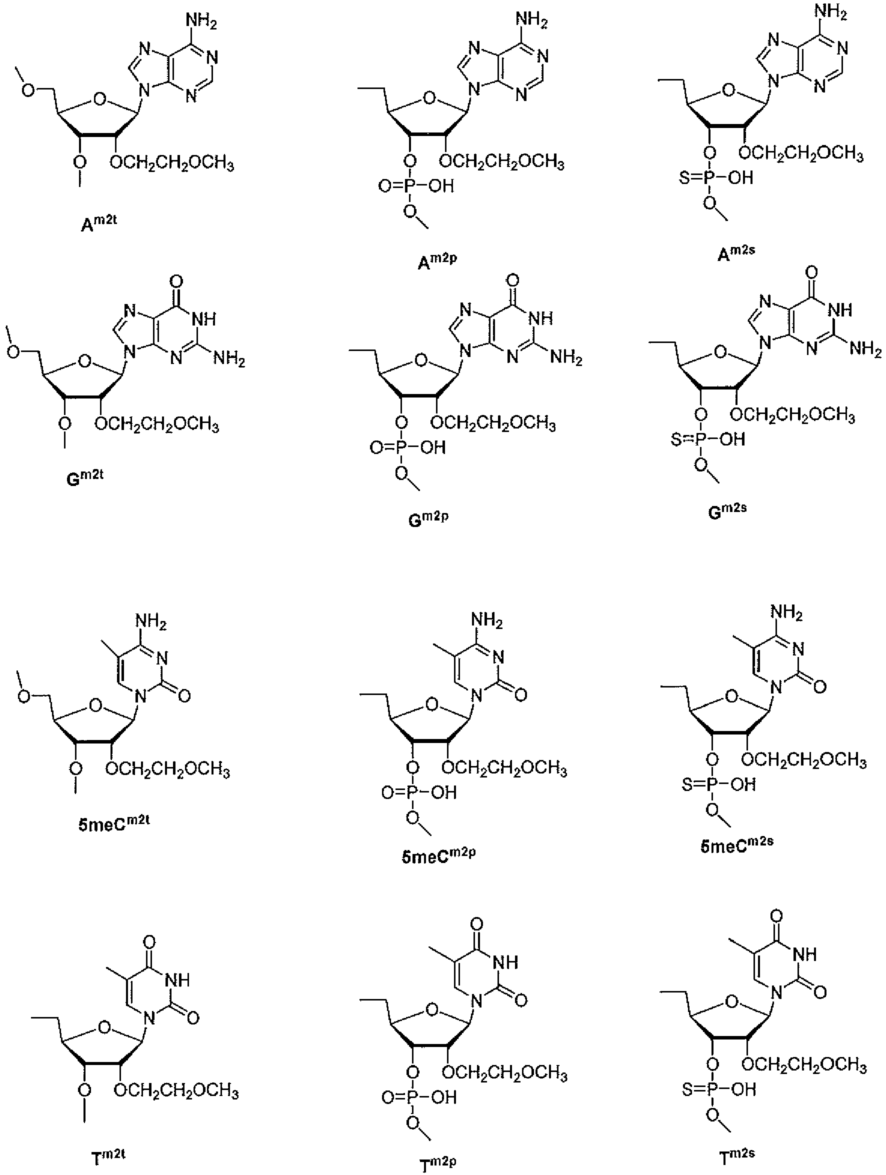

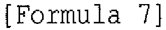

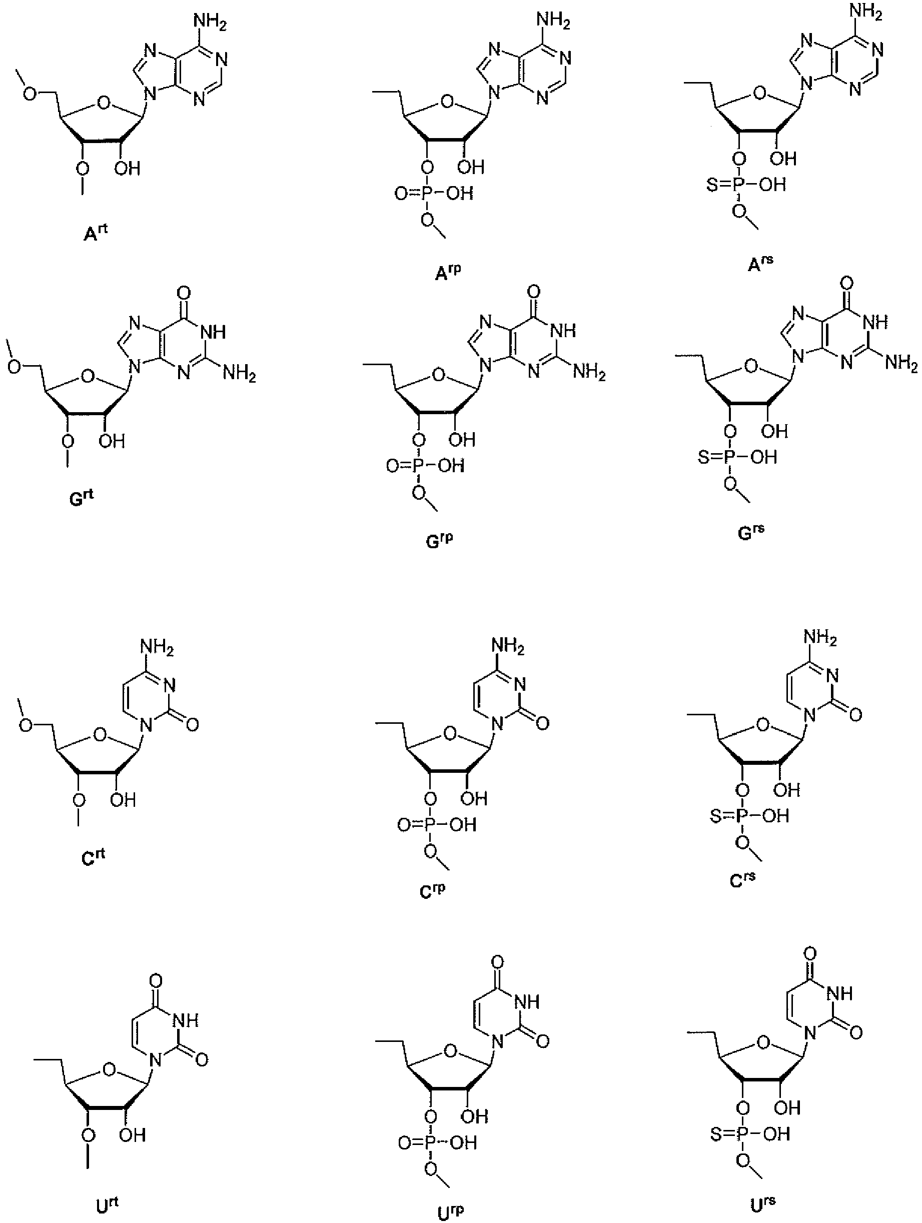

In the present specification, a double-stranded polynucleotide having an antisense strand modified at its 5'-end with phenylphosphate contains an aromatic substituent X (shown below) bound via a phosphodiester bond to the phosphate at the 5'-end of the antisense strand in the double-stranded polynucleotide, i.e., forms an X-P(=O)(OH)-5'-oligonucleotide structure.

In this context, X represents an aromatic group which may be heteroaryl wherein a hydroxy group moiety on the ring is a binding site. X is specifically as follows:
(a) a substituent represented by the formula (I):

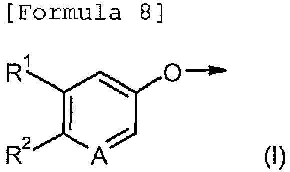

wherein A represents a nitrogen atom or C-R³,
R¹ and R² each independently represent
a hydrogen atom,
an alkyl group having 1 to 8 carbon atoms and optionally having a substituent,
an alkoxy group having 1 to 8 carbon atoms and optionally having a substituent,
a cycloalkyl group having 3 to 6 carbon atoms and optionally having a substituent,
a halogen atom,
an alkylcarbonyl group containing an alkyl group having 1 to 8 carbon atoms and optionally having a substituent,
a phenyl group optionally having a substituent,
a phenyloxy group optionally having a substituent,
a saturated or unsaturated 5- or 6-membered heterocyclic group containing 1 to 3 heteroatoms selected from the heteroatom group consisting of a nitrogen atom, an oxygen atom, and a sulfur atom and optionally having a substituent,
an aralkyl group optionally having a substituent in the phenyl group moiety,
an aralkyloxy group optionally having a substituent in the phenyl group moiety,
an alkylsulfonyl group having 1 to 6 carbon atoms, a hydroxy group,
an alkylcarbonylamino group having an alkyl group having 1 to 9 carbon atoms,
a hydroxyalkylcarbonylamino group having an alkyl group having 1 to 9 carbon atoms wherein the alkyl group is substituted by a hydroxy group on the terminal carbon atom thereof,
an N-alkylcarbamoyl group having an alkyl group having 1 to 8 carbon atoms,
an N-(hydroxyalkyl)carbamoyl group having an alkyl group having 1 to 9 carbon atoms wherein the alkyl group is substituted by a hydroxy group on the terminal carbon atom thereof,
an N-alkylcarbamoylalkylene group having an alkyl group having 1 to 8 carbon atoms and being bonded to the aromatic ring via an alkylene group containing 1 to 4 carbon atoms,
an N-(hydroxyalkyl)carbamoylalkylene group having an alkyl group having 1 to 9 carbon atoms wherein the alkyl group is substituted by a hydroxy group on the terminal carbon atom thereof, and being bonded to the aromatic ring via an alkylene group containing 1 to 4 carbon atoms, a carboxy group, or
a group represented by the formula: -(CH₂)ₖ-CONR⁴R⁵, wherein R⁴ and R⁵ each independently represent a hydrogen atom, an alkyl group having 1 to 6 carbon atoms and optionally having a substituent, or an aralkyl group optionally having a substituent in the phenyl group moiety, and k represents an integer of 0 to 3, or R¹ and R² are optionally combined to form, together with the aromatic ring bonded to R¹ and R², a saturated or unsaturated bicyclic or tricyclic structure optionally having a substituent, optionally further containing 1 or 1 or more heteroatoms as constituent atoms in the ring, and optionally having an oxo group, and
R³ represents
a halogen atom,
an alkyl group having 1 to 6 carbon atoms,
an alkoxy group having 1 to 6 carbon atoms,
a halogenomethyl group,
a hydroxy group, or
a hydrogen atom; or
(b) a tyrosine residue optionally having a substituent on the amino group wherein a hydroxy group on the phenyl group is the phosphate group binding site.

X represents the aspect (a) or (b), as described above. When X has the aspect (a), the moiety A represents the partial structure C-R³ or a nitrogen atom. When A is C-R³, X represents a phenyl group-containing substituent having a structure represented by the following formula (Ia):

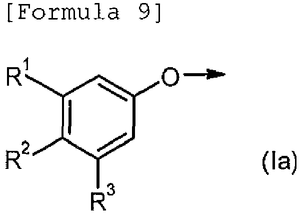

When A is a nitrogen atom, X represents a pyridinyl group-containing substituent represented by the following formula (Ib):

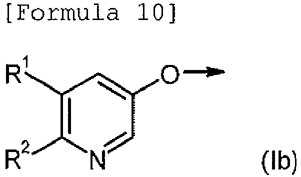

The substituents R¹ and R² in the formula (I) will now be described.

When R¹ and R² are each independently an alkyl group having 1 to 8 carbon atoms and optionally having a substituent, the alkyl group may be straight-chain or branched-chain. Examples thereof can include a methyl group, an ethyl group, a propyl group, an isopropyl group, a butyl group, an isobutyl group, a secondary butyl group, a pentyl group, a hexyl group, a heptyl group, and an octyl group. When the alkyl group has a substituent, the alkyl group may have, as the substituent, 1 or 1 or more groups selected from the substituent group consisting of a hydroxy group, an amino group, a halogen atom, an alkylthio group having 1 to 6 carbon atoms, an alkoxy group having 1 to 6 carbon atoms, a carboxy group, and an alkoxycarbonyl group containing an alkoxy group having 1 to 6 carbon atoms. Any 1 or more substituents may be the same or different. When a hydroxy group or an amino group is used as the substituent in the alkyl group, it is more preferred that the alkyl group should be substituted by this group on the terminal carbon atom thereof. The alkyl group having a hydroxy group is preferably a hydroxymethyl group, a 2-hydroxyethyl group, a 2-hydroxypropyl group, or a 3-hydroxypropyl group. When a halogen atom is used as the substituent in the alkyl group, the alkyl group can be any of a linear or branched alkyl group having 1 to 6 carbon atoms and is more preferably a methyl group or an ethyl group, particularly preferably a methyl group, having a halogen atom. When a halogen atom is used as the substituent in the alkyl group, the halogen atom is preferably a fluorine atom. The number of the fluorine atom may be mono-substitution or perfluoro-substitution. Examples of such an alkyl group can include a monofluoromethyl group, a difluoromethyl group, a trifluoromethyl group, and a 2,2,2-trifluoroethyl group. A monofluoromethyl group, a difluoromethyl group, and a trifluoromethyl group are preferable. The alkylthio group having 1 to 6 carbon atoms and the alkoxy group having 1 to 6 carbon atoms may be straight-chain or branched-chain, and examples thereof can include a methyl group, an ethyl group, a propyl group, an isopropyl group, a butyl group, an isobutyl group, and a secondary butyl group. When a carboxy group or an alkoxycarbonyl group containing an alkoxy group having 1 to 6 carbon atoms is used as the substituent in the alkyl group, the alkyl group substituted by this group on the terminal carbon atom thereof is more preferable. The alkyl group in the alkoxycarbonyl group containing an alkoxy group having 1 to 6 carbon atoms may be a straight-chain or a branched-chain, and examples thereof can include a methyl group, an ethyl group, a propyl group, an isopropyl group, a butyl group, an isobutyl group, and a secondary butyl group.

When R¹ and R² are each independently an alkoxy group having 1 to 8 carbon atoms and optionally having a substituent, this alkoxy group can be any alkoxy group composed of the alkyl group shown above and an oxygen atom.

When R¹ and R² are each independently a cycloalkyl group having 3 to 6 carbon atoms and optionally having a substituent, the cycloalkyl group is any of a cyclopropyl group, a cyclobutyl group, a cyclopentyl group, and a cyclohexyl group. When this cycloalkyl group has a substituent, the cycloalkyl group can have, as the substituent, the same substituent as that exemplified for the alkyl group or the alkoxy group.

When R¹ and R² are each independently a halogen atom, the halogen atom is a fluorine atom, a chlorine atom, a bromine atom, or an iodine atom.

When R¹ and R² are each independently an alkylcarbonyl group (aliphatic acyl group) containing an alkyl group having 1 to 8 carbon atoms and optionally having a substituent, this alkyl moiety can be any alkyl group having 1 to 8 carbon atoms shown above. The alkylcarbonyl group can be composed of such an alkyl group and a carbonyl group.

When R¹ and R² are each independently a phenyl group optionally having a substituent, the phenyl group may have, as the substituent, 1 or 1 or more groups selected from the substituent group consisting of a hydroxy group, an amino group, a halogen atom, an alkylthio group having 1 to 6 carbon atoms, an alkoxy group having 1 to 6 carbon atoms, a carboxy group, and an alkoxycarbonyl group containing an alkoxy group having 1 to 6 carbon atoms. Any 1 or more substituents may be the same or different. The number of the substituent can be 1 to 3.

When R¹ and R² are each independently a phenyloxy group optionally having a substituent, this phenyloxy group can be a phenyloxy group composed of the phenyl group described above and an oxygen atom.

When R¹ and R² are each independently a heterocyclic group optionally having a substituent, this heterocyclic group can be a 5- or 6-membered ring. The heteroatom can be 1 to 3 heteroatoms selected from the heteroatom group consisting of a nitrogen atom, an oxygen atom, and a sulfur atom. Of them, a nitrogen atom is preferable, and one containing 1 or 2 nitrogen atoms is more preferable. In addition, a heterocyclic substituent containing an oxygen atom or a sulfur atom may be used. These heterocyclic groups may be saturated or unsaturated.

When R¹ and R² are each independently an aralkyl group optionally having a substituent in the phenyl group moiety, this phenyl group moiety can be the phenyl group shown above. An alkylene group bound to this phenyl group can be a methylene group or a polymethylene group having 2 to 4 carbon atoms and may be further substituted by an alkyl group having 1 to 6 carbon atoms. Examples of such an aralkyl group can include a benzyl group and an α-methylbenzyl group. The aralkyl group may have, as the substituent in the phenyl group moiety, 1 or 1 or more groups selected from the substituent group consisting of a hydroxy group, an amino group, a halogen atom, an alkylthio group having 1 to 6 carbon atoms, an alkoxy group having 1 to 6 carbon atoms, a carboxy group, and an alkoxycarbonyl group containing an alkoxy group having 1 to 6 carbon atoms. Any 1 or more substituents may be the same or different. The number of the substituent can be 1 to 3.

When R¹ and R² are each independently an aralkyloxy group optionally having a substituent in the phenyl group moiety, this aralkyloxy group can be any aralkyloxy group composed of the aralkyl group described above and an oxygen atom.

When R¹ and R² are each independently an alkylsulfonyl group [Alkyl-SO₂-] having 1 to 6 carbon atoms, the alkyl group having 1 to 6 carbon atoms may be a straight-chain or a branched-chain. Examples thereof can include a methyl group, an ethyl group, a propyl group, an isopropyl group, a butyl group, an isobutyl group, a secondary butyl group, a pentyl group, and a hexyl group. Moreover, the alkyl group may be not only linear but alternatively cyclic. The same alkyl group having 1 to 8 carbon atoms as above can be used except that the number of carbon atoms is 1 to 6.

When R¹ and R² are each independently an alkylcarbonylamino group [Alkyl-(C=O)-NH-] having an alkyl group having 1 to 9 carbon atoms, the alkyl group moiety can be any alkyl group having up to 9 carbon atoms, including the alkyl group having up to 8 carbon atoms described above. Examples thereof can include a methyl group, an ethyl group, and a propyl group.

When R¹ and R² are each independently a hydroxyalkylcarbonylamino group [HO-Alkyl-(C=O)-NH-] having an alkyl group having 1 to 9 carbon atoms wherein the alkyl group is substituted by a hydroxy group on the terminal carbon atom thereof, the alkyl group moiety may be a straight-chain or a branched-chain. The alkyl group moiety can be any alkyl group having up to 9 carbon atoms, including the alkyl group having up to 8 carbon atoms described above. Examples thereof can include a methyl group, an ethyl group, a propyl group, an isopropyl group, a butyl group, an isobutyl group, a secondary butyl group, a pentyl group, a hexyl group, a heptyl group, and an octyl group. This alkyl group is substituted by the hydroxy group on the terminal carbon atom thereof.

When R¹ and R² are each independently an N-alkylcarbamoyl group [Alkyl-NH-(C=O)-] having an alkyl group having 1 to 8 carbon atoms, this group has a structure in which the carbamoyl group is substituted by the alkyl group on the nitrogen atom thereof. This alkyl group moiety may be a straight-chain or a branched-chain, as described above. Examples thereof can include a methyl group, an ethyl group, a propyl group, an isopropyl group, a butyl group, an isobutyl group, a secondary butyl group, a pentyl group, a hexyl group, a heptyl group, and an octyl group.

When R¹ and R² are each independently an N-(hydroxyalkyl)carbamoyl group [HO-Alkyl-NH-(C=O)-] having an alkyl group having 1 to 9 carbon atoms wherein the alkyl group is substituted by a hydroxy group on the terminal carbon atom thereof, this group has a structure in which the carbamoyl group is substituted by a hydroxyalkyl group on the nitrogen atom thereof. This hydroxyalkyl group moiety can be the hydroxyalkyl group described above in which the alkyl group is substituted by a hydrogen atom on the terminal carbon atom thereof.

When R¹ and R² are each independently an N-alkylcarbamoylalkylene group [Alkyl-NH-(C=O)-(CH2)ᵢ-; i represents an integer of 1 to 4] having an alkyl group having 1 to 8 carbon atoms, and being bonded to the aromatic ring via an alkylene group containing 1 to 4 carbon atoms, this group has a structure in which the N-alkylcarbamoyl group is bonded to the aromatic ring via the alkylene group. The N-alkylcarbamoyl group may be the same as described above. The alkylene group at the binding site can have 1 to 4 carbon atoms, preferably 2 carbon atoms.

When R¹ and R² are each independently an N-(hydroxyalkyl)carbamoylalkylene group [HO-Alkyl-NH-(C=O)-(CH₂)ⱼ-; j represents an integer of 1 to 4] having an alkyl group having 1 to 9 carbon atoms wherein the alkyl group is substituted by a hydroxy group on the terminal carbon atom thereof, and being bonded to the aromatic ring via an alkylene group containing 1 to 4 carbon atoms, this group has a structure in which the N-hydroxyalkylcarbamoyl group is bonded to the aromatic ring via the alkylene group. The N-hydroxyalkylcarbamoyl group and the alkylene group at the binding site can be any of those described above.

When R¹ and R² are each independently a group represented by the formula: -(CH₂)ₖ-CONR⁴R⁵, R⁴ and R⁵ each independently represent a hydrogen atom, an alkyl group having 1 to 6 carbon atoms and optionally having a substituent, or an aralkyl group optionally having a substituent in the phenyl group moiety. The alkyl group having 1 to 6 carbon atoms and optionally having a substituent or the aralkyl group optionally having a substituent in the phenyl group moiety can be the same as those defined in R¹ or R². R⁴ and R⁵ are preferably a hydrogen atom and/or an aralkyl group optionally having a substituent in the phenyl group moiety. Moreover, k can be any integer of 0 to 3. When k is 0, R⁴ and R⁵ are preferably a combination of hydrogen atoms. When k is an integer of 1 or larger, particularly, 2, R⁴ and R⁵ are preferably a combination of a hydrogen atom and a benzyl group.

Alternatively, R¹ and R² may be combined to form a bicyclic or tricyclic structure optionally having a substituent. The cyclic structure thus formed may be saturated or unsaturated. Furthermore, this cyclic structure may contain 1 or 1 or more heteroatoms as constituent atoms in the ring.

When R¹ and R² are combined to form a bicyclic structure, R¹ and R² can be, for example, a polymethylene chain having 3 to 4 carbon atoms. Specifically, they can have a structure in which a saturated alicyclic compound is condensed with a benzene ring. Moreover, they can also be an aromatic naphthalene group as a bicyclic structure. Thus, the bicyclic structure can be the form of a 6-5 or 6-6 condensed ring. This bicyclic structure may have, as the substituent, 1 or 1 or more groups selected from the substituent group consisting of a hydroxy group, an amino group, a halogen atom, an alkylthio group having 1 to 6 carbon atoms, an alkoxy group having 1 to 6 carbon atoms, a carboxy group, an alkoxycarbonyl group containing an alkoxy group having 1 to 6 carbon atoms, and an oxo group. Any 1 or more substituents may be the same or different. The number of the substituent can be 1 to 3. The heteroatom can be 1 or 1 or more heteroatoms selected from a nitrogen atom, an oxygen atom, and a sulfur atom, and the number thereof is 1 to 4.

When R¹ and R² are combined to form a tricyclic structure, R¹ and R² can form a cyclic structure, as in the bicyclic structure, and can form, for example, a 6-5-6, 6-6-5, or 6-6-6 condensed ring structure.

This bicyclic or tricyclic structure may have, as the substituent, 1 or 1 or more groups selected from the substituent group consisting of a hydroxy group, an amino group, a halogen atom, an alkylthio group having 1 to 6 carbon atoms, an alkoxy group having 1 to 6 carbon atoms, a carboxy group, an alkoxycarbonyl group containing an alkoxy group having 1 to 6 carbon atoms, and an oxo group. Any 1 or more substituents may be the same or different. The number of the substituent can be 1 to 3. The heteroatom can be 1 or 1 or more heteroatoms selected from a nitrogen atom, an oxygen atom, and a sulfur atom, and the number thereof is 1 to 4. Examples thereof can include an anthracenyl group, a fluorenyl group, and a dibenzofuranyl group.

The substituent R³ in formula (I) will now be described.

When R³ is a halogen atom, examples of the halogen atom can include a chlorine atom and a fluorine atom.

When R³ is an alkyl group having 1 to 6 carbon atoms, the alkyl group may be a straight-chain or a branched-chain. Examples thereof can include a methyl group, an ethyl group, a propyl group, an isopropyl group, a butyl group, an isobutyl group, a secondary butyl group, a pentyl group, and a hexyl group.

When R³ is an alkoxyl group having 1 to 6 carbon atoms, the alkyl group in the alkoxycarbonyl group may be a straight-chain or a branched-chain, and examples thereof can include a methyl group, an ethyl group, a propyl group, an isopropyl group, a butyl group, an isobutyl group, and a secondary butyl group.

When R³ is a halogenomethyl group, examples of the halogen can include fluorine. A substituent therefor can be that exemplified for R¹ and R².

When R³ is a group other than a hydrogen atom, R¹ and R² are preferably a group selected from a hydrogen atom, a halogen atom, a methyl group, and a hydroxy group. More preferably, all of R¹, R² and R³ are a halogen atom.

When R³ is a hydrogen atom, R¹ and R² are preferably a group selected from a hydrogen atom, a hydroxy group, a halogen atom, a halogenoalkoxy group, an acyl group, and a phenyl group. The halogenoalkoxy group may be a trifluoromethoxy group, and the acyl group may be an acetyl group.

X may be the aspect (b), i.e., a tyrosine residue wherein a hydroxy group on the phenyl group is the phosphate group binding site. This tyrosine residue may have a substituent on the amino group. The tyrosine residue as X having no substituent on the amino group is shown below.

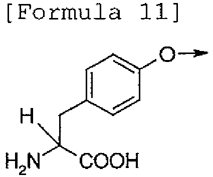

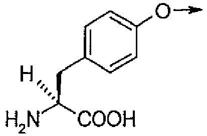

The tyrosine residue may or may not be optically active.

Examples of the substituent on the amino group can include an alkyl group having 1 to 6 carbon atoms, an arylcarbonyl group, a heteroarylcarbonyl group, and an aralkyloxycarbonyl group.

When the substituent on the amino group is an alkyl group having 1 to 6 carbon atoms, this alkyl group may be a straight-chain or a branched-chain. Examples thereof can include a methyl group, an ethyl group, a propyl group, an isopropyl group, a butyl group, an isobutyl group, a secondary butyl group, a pentyl group, and a hexyl group.

When the substituent on the amino group is an arylcarbonyl group, this aryl group can be a phenyl group. This phenyl group may have a substituent. The phenyl group may have, as the substituent, 1 or 1 or more groups selected from the substituent group consisting of a hydroxy group, an amino group, a halogen atom, an alkylthio group having 1 to 6 carbon atoms, an alkoxy group having 1 to 6 carbon atoms, a carboxy group, and an alkoxycarbonyl group containing an alkoxy group having 1 to 6 carbon atoms. Any 1 or more substituents may be the same or different. There can be from 1 to 3 substituents.

When the substituent on the amino group is a heteroarylcarbonyl group, the heteroaryl group is a 5- or 6-membered ring. The heteroatom can be 1 or 1 or more heteroatoms selected from a nitrogen atom, an oxygen atom, and a sulfur atom, and the number thereof is 1 to 2. Examples thereof can include a pyridyl group, a furyl group, and a thienyl group.

When the substituent on the amino group is an aralkyloxycarbonyl group, this aralkyloxycarbonyl group can be a carbonyl group bound to an aralkyloxy group composed of an aralkyl group (composed of a phenyl group moiety having a substituent and an alkylene group which is a methylene group or a polymethylene group having 2 to 4 carbon atoms) and an oxygen atom. Examples of the aralkyl group can include a benzyl group and an α-methylbenzyl group. The phenyl group moiety may have, as the substituent, 1 or 1 or more groups selected from the substituent group consisting of a hydroxy group, an amino group, a halogen atom, an alkylthio group having 1 to 6 carbon atoms, an alkoxy group having 1 to 6 carbon atoms, a carboxy group, and an alkoxycarbonyl group containing an alkoxy group having 1 to 6 carbon atoms. Any 1 or more substituents may be the same or different. The number of the substituent can be 1 to 3.

In the present specification, a double-stranded polynucleotide having an antisense strand in which the substituent represented by X is added to the 5'-end of the antisense strand is also referred to as a "5'-Ar-double-stranded polynucleotide".

Specific examples of the substituent represented by X can include compounds represented by the following chemical formulas:

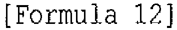

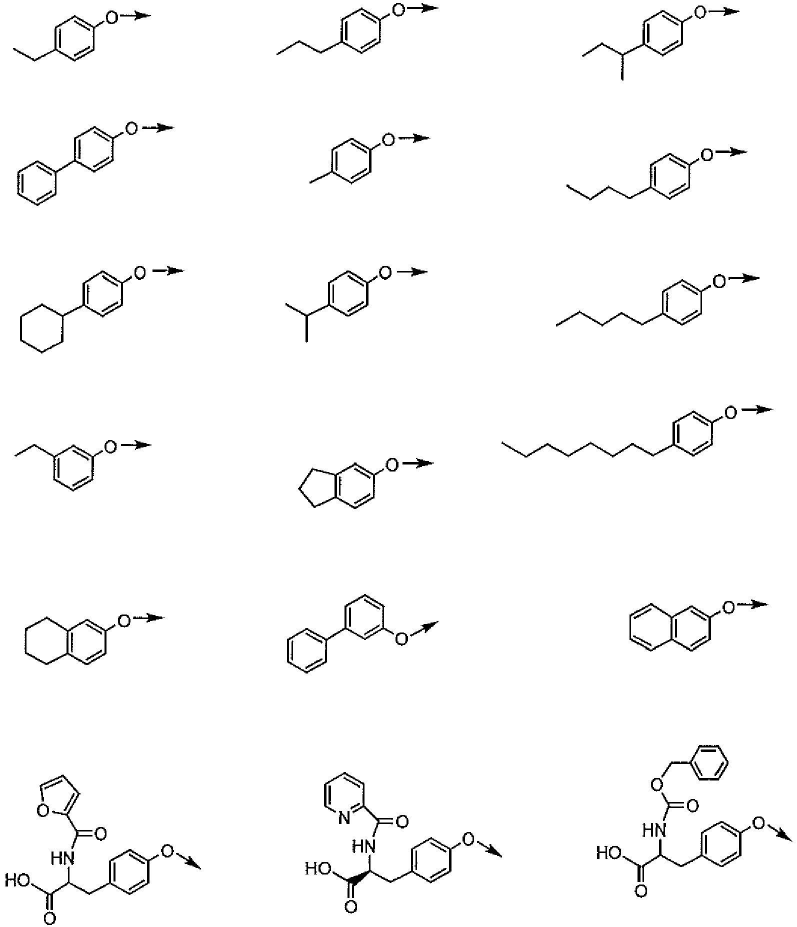

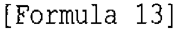

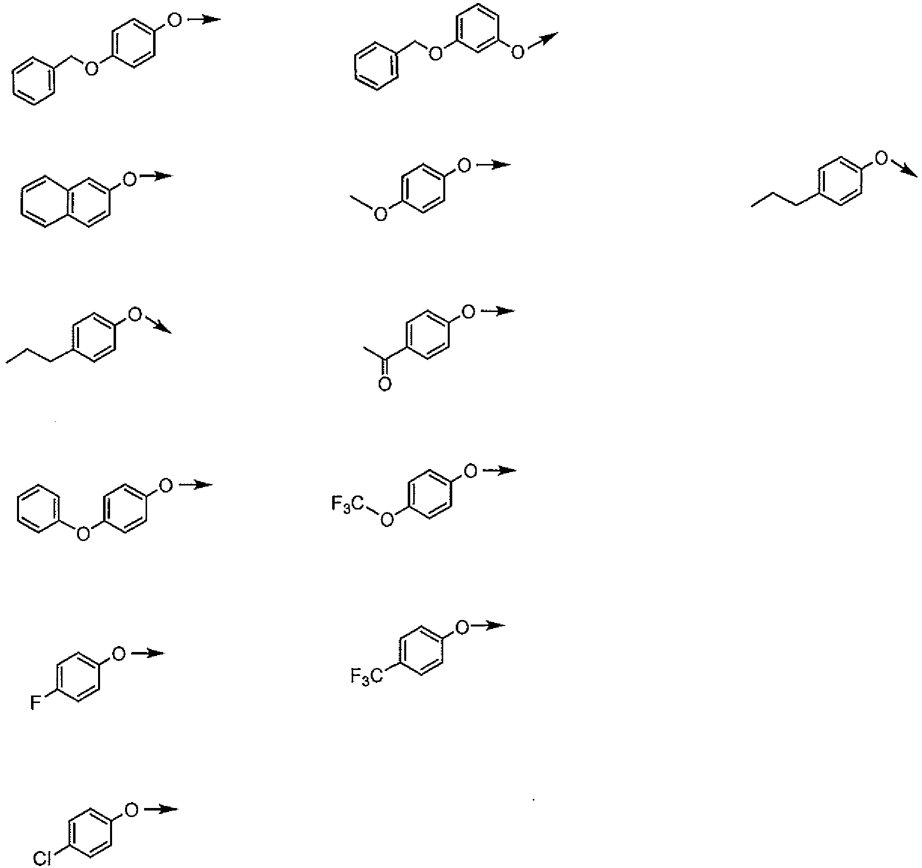

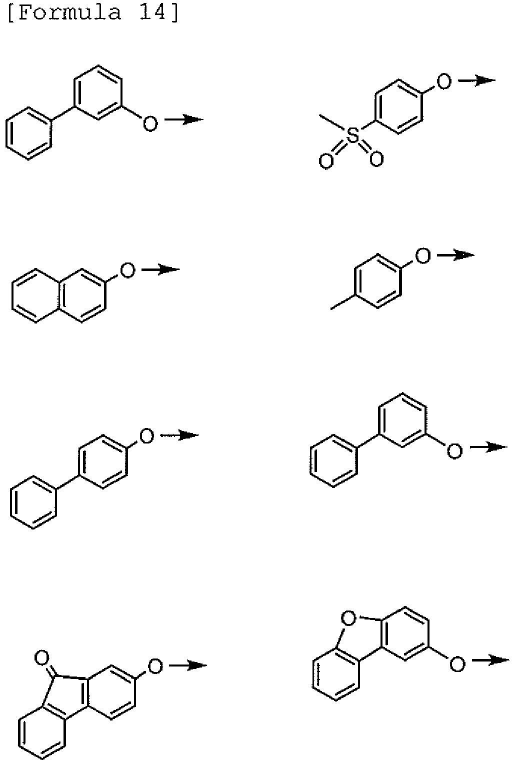

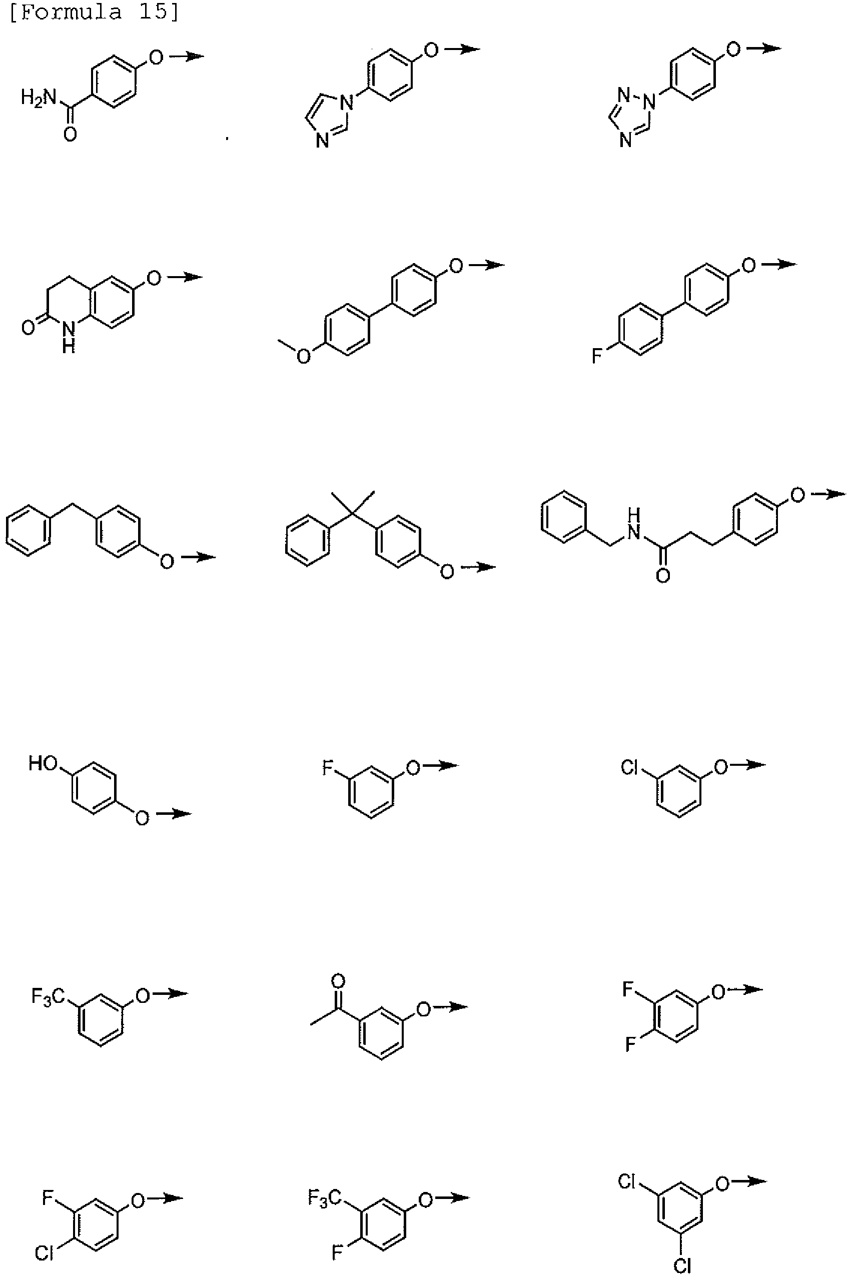

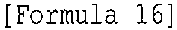

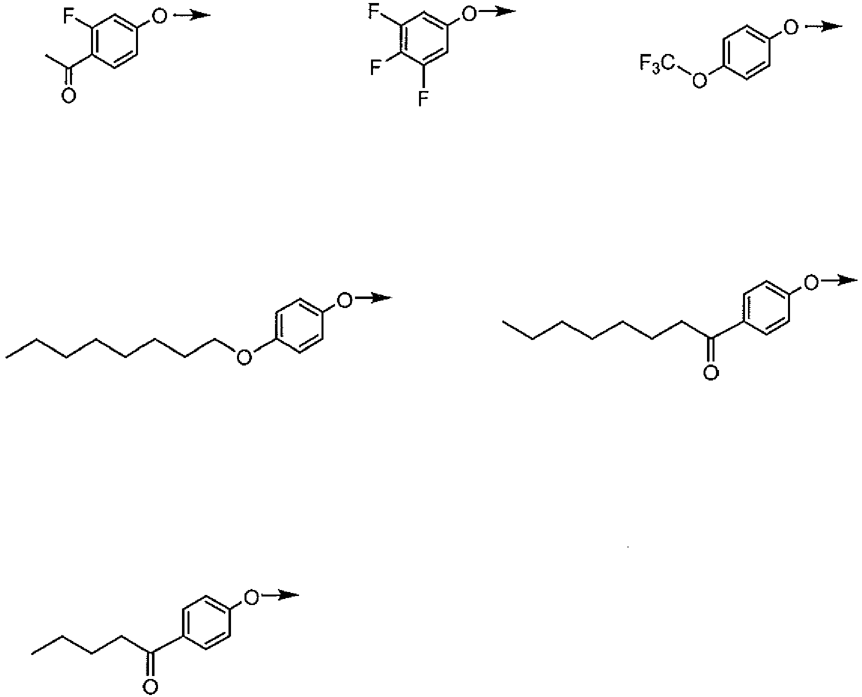

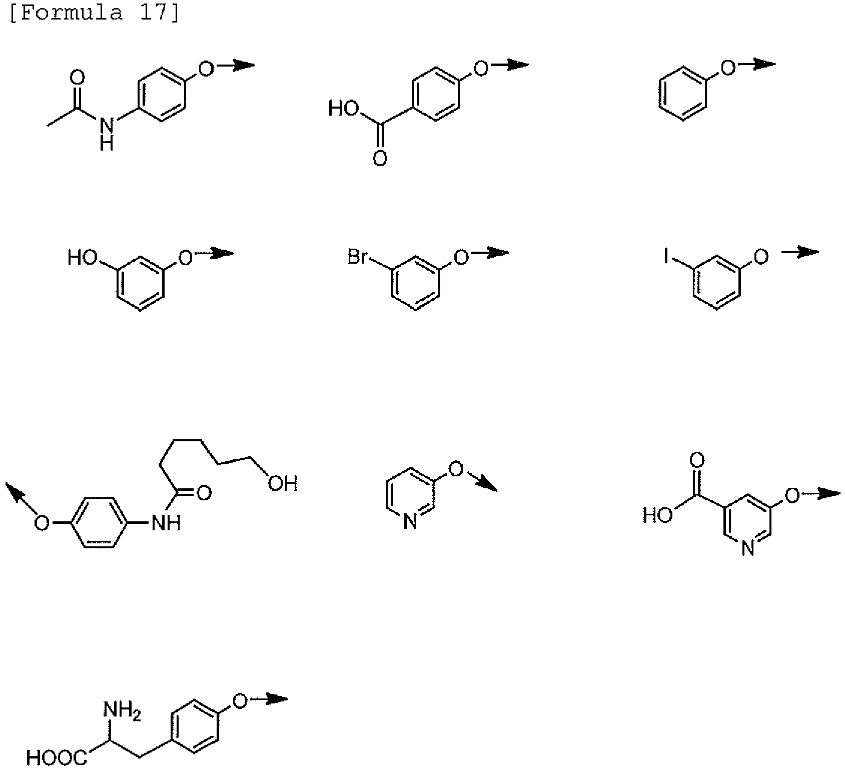

In the present specification, the term "complementary nucleotides" refers to nucleotides whose base moieties are complementary to each other and, for example, nucleotide pairs comprising adenine and thymine, guanine and cytosine, guanine and 5-methylcytosine, and adenine and uracil as base moieties are nucleotides complementary to each other.

In the present specification, a "complementary nucleotide sequence" includes a nucleotide sequence consisting of nucleotides, all of which are complementary to a target nucleotide sequence, and also includes a nucleotide sequence forming base pairs with a target oligonucleotide and/or polynucleotide, although one or more nucleotides are not complementary.

In the present specification, the 5'-Ar-double-stranded polynucleotide is a polynucleotide forming a duplex by way of forming Watson-Crick base pairs between complementary nucleotides, though not all the nucleotides in the polynucleotide may form Watson-Crick base pairs.

In the present specification, the chain length of the 5'-Ar-double-stranded polynucleotide refers to the number of nucleotides in a polynucleotide constituting the sense or antisense strand.

In the present specification, in polynucleotides constituting the 5'-Ar-double-stranded polynucleotide, a strand comprising a nucleotide sequence identical to a target gene is called a passenger strand or sense strand, whereas a strand comprising a nucleotide sequence complementary to the target gene is called a guide strand or antisense strand.

In the present specification, the phrase "having a nucleotide sequence identical to a target gene" refers to having a sequence identical to at least a partial nucleotide sequence of the target gene. It includes a completely identical sequence, and a substantially identical sequence as long as the resulting double-stranded polynucleotide has an RNA interference effect and/or gene expression inhibitory effect on the target gene. Moreover, when the target gene is known to have SNPs or the like, a sequence having these variations is also included as an identical nucleotide sequence. In the present specification, a double-stranded polynucleotide that comprises a nucleotide sequence identical to a target gene and has RNA interference effect and/or gene expression inhibitory effect on the target gene is referred to as a double-stranded polynucleotide against the target gene.

The nucleotide sequence of the 5'-Ar-double-stranded polynucleotide against the target gene is not particularly limited as long as it has RNA interference effect and/or gene expression inhibitory effect on the target gene. For example, it can be determined on the basis of a sequence predicted to have RNA interference effect on the target gene using computer software (e.g., GENETYX (registered trademark): manufactured by GENETYX CORPORATION), and can also be determined by further confirming the RNA interference effect and/or gene expression inhibitory effect of a 5'-Ar-double-stranded polynucleotide prepared on the basis of the selected sequence.

In the present specification, the gene expression inhibitory effect includes the effect of completely inhibiting gene expression and also includes the effect of reducing gene expression, compared with a control. Moreover, in the present specification, gene expression inhibitory effect is used with the same meaning as gene expression inhibitory activity.

The RNA interference effect and/or the gene expression inhibitory effect can be confirmed by methods usually performed by those skilled in the art and can be confirmed, for example, by: administering a double-stranded polynucleotide against a target gene to cells expressing the target gene; after a lapse of a given period of time, quantifying a protein, which is a translation product of the target gene, by Western blot analysis; and comparing the protein expression level with a control. Moreover, the RNA interference effect and/or the gene expression inhibitory effect can also be confirmed by measuring in real time the expression level of the target gene after administration of the double-stranded polynucleotide against the target gene by the method of real-time PCR.

A polynucleotide having a sequence identical or substantially identical to at least a partial nucleotide sequence of the target gene is a polynucleotide having a sequence identical or substantially identical to any 18 or more -nucleotide sequence in the nucleotide sequence of the target gene. In this context, a "substantially identical sequence" refers to a sequence having 70% or higher, preferably 80% or higher, more preferably 90% or higher homology, to the nucleotide sequence of the target gene. The homology of the nucleotide sequence can be calculated using gene analysis software known in the art such as BLAST (registered trademark).

In the item <223> for each sequence in the Sequence Listing attached to the present specification, "cm" represents 2'-O-methylcytidine; "um" represents 2'-O-methyluridine; and "gm" represents 2'-O-methylguanosine.

### 2. 5'-Ar-double-stranded polynucleotide

The chain length of the 5'-Ar-double-stranded polynucleotide according to the present invention may be any length from 18 nucleotides to the full length of the open reading frame (ORF) of the target gene as long as it has an RNA interference effect and/or gene expression inhibitory effect. The sense strand is preferably 18 to 21 nucleotides, more preferably 18 or 19 nucleotides, in chain length. The antisense strand is preferably 19 to 21 nucleotides, more preferably 21 nucleotides, in chain length. The 5'-Ar-double-stranded polynucleotide does not have to be a duplex in its entirety and includes those partially overhanging at the 5' and/or 3'-ends. The overhanging end has 1 to 5 nucleotides, preferably 1 to 3 nucleotides, more preferably 2 nucleotides. Moreover, the most preferable examples thereof include those having a structure in which the 3'-end of the polynucleotide of the antisense strand overhangs by 2 nucleotides (overhang structure).

The 5'-Ar-double-stranded polynucleotide has at least one property selected from the following (a) to (h):
(a) having an RNA interference effect and/or a gene expression inhibitory effect on the target gene;
(b) being resistant to RNase and having an RNA interference effect and/or a gene expression inhibitory effect on the target gene;
(c) being resistant to phosphatase and having an RNA interference effect and/or a gene expression inhibitory effect on the target gene;
(d) being resistant to exonuclease and having an RNA interference effect and/or a gene expression inhibitory effect on the target gene;
(e) being resistant to RNase, phosphatase, and exonuclease and having an RNA interference effect and/or a gene expression inhibitory effect on the target gene;
(f) having an antisense strand that is resistant to phosphatase and having an RNA interference effect and/or a gene expression inhibitory effect on the target gene;
(g) having an antisense strand that is resistant to exonuclease and having an RNA interference effect and/or a gene expression inhibitory effect on the target gene; and
(h) having an antisense strand that is resistant to 5'-3'-exonuclease and having an RNA interference effect and/or a gene expression inhibitory effect on the target gene.

### 2-1.

An example of the 5'-Ar-double-stranded polynucleotide can include a double-stranded polynucleotide having a sense strand polynucleotide consisting of a nucleotide sequence complementary to a target sequence in a target gene, and an antisense strand polynucleotide having a nucleotide sequence complementary to the sense strand polynucleotide, wherein the substituent X represented by the formula (I) in the present specification is added to a phosphate group at the 5'-end of the antisense strand polynucleotide.

A further example of the 5'-Ar-double-stranded polynucleotide can include an isolated double-stranded RNA molecule being of 19 to 23 bases in length and having RNA strands, each of which is 19 to 23 bases in length and at least one of which has a 3'-overhang consisting of 1 to 3 bases, wherein the RNA molecule is a double-stranded polynucleotide and is capable of target-specific RNA interference and has one strand consisting of a sequence 100% identical to the predetermined target mRNA molecule except for the 3'-overhang, the target mRNA molecule being present in a cell or an organism, and wherein the substituent X represented by the formula (I) in the present specification is added to a phosphate group at the 5'-end of the antisense strand.

A further example of the 5'-Ar-double-stranded polynucleotide can include the double-stranded polynucleotide or salt thereof according to (1), wherein the sense strand polynucleotide consists of a polynucleotide represented by the following formula (II), the antisense strand polynucleotide consists of a polynucleotide represented by the following formula (III), and the double-stranded polynucleotide further has the following features (a) to (d):

5'-(γ-β)₉-γ-γₘ-3' (II)

and

5'-X-β-(γ-β)₉-υₙ-3' (III),

(a) γ represents an RNA, β represents a 2'-OMeRNA, and λ and u each represent a DNA;
(b) m and n identically or differently represent any integer from 0 to 5;
(c) (γ-β)₉-γ in the polynucleotide represented by the formula (III) has a nucleotide sequence identical to the target gene; and
(d) (γ-β)₉-γ in the formula (II) and β-(γ-β)₉ in the formula (III) have nucleotide sequences complementary to each other.

A further example of the 5'-Ar-double-stranded polynucleotide can include the double-stranded polynucleotide or salt thereof according to (1), wherein the sense strand polynucleotide consists of a polynucleotide represented by the following formula (IV), the antisense strand polynucleotide consists of a polynucleotide represented by the following formula (V), and the double-stranded polynucleotide further has the following features (a) to (d):

5'-(α-β)₉-αₚ-λₘ-3' (IV)

5'-X-δₛ-(α-β)₉-υₙ-3' (V),

(a) α and β differently represent a DNA or a 2'-OMeRNA, δ and λ identically or differently represent a DNA or a 2'-OMeRNA, and u identically or differently represents any nucleotide selected from a DNA, an RNA, and a 2'-OMeRNA;
(b) p represents an integer of 0 or 1, m is 0 when p is 0 and represents any integer from 0 to 5 when p is 1, s represents an integer of 0 or 1, and n represents any integer from 0 to 5;
(c) (α-β)₉-αₚ in the polynucleotide represented by the formula (IV) has a nucleotide sequence identical to the target gene; and
(d) (α-β)₉ in the formula (IV) and (α-β)₉ in the formula (V) have nucleotide sequences complementary to each other.

A further example of the 5'-Ar-double-stranded polynucleotide can include the double-stranded polynucleotide or a salt thereof according to (1), wherein the sense strand polynucleotide consists of a polynucleotide represented by the following formula (VI), the antisense strand polynucleotide consists of a polynucleotide represented by the following formula (VII), and the double-stranded polynucleotide further has the following features (a) to (d):

5'-β-(α-β)₈-αₚ-λₘ-3' (VI)

5'-X-δₛ-(α-β)₈-(α-β)-υₙ-3' (VII),

(a) α and β differently represent a DNA or a 2'-OMeRNA, δ and λ identically or differently represent a DNA or a 2'-OMeRNA, and u identically or differently represents any nucleotide selected from a DNA, an RNA, and a 2'-OMeRNA;
(b) p represents an integer of 0 or 1, m is 0 when p is 0 and represents any integer from 0 to 5 when p is 1, s represents an integer of 0 or 1, and n represents any integer from 0 to 5;
(c) β-(α-β)₈-αₚ in the polynucleotide represented by the formula (VI) has a nucleotide sequence identical to the target gene; and
(d) (α-β)₈ in the formula (VI) and (α-β)₈ in the formula (VII) have nucleotide sequences complementary to each other.

The sugar-modified nucleotide encompasses all manner of sugar modification known in the technical field of the present invention. The sugar-modified nucleotide can retain every heterocyclic base site and internucleoside bond and further includes sugar-modified nucleotides different from the sugar modifications described above. The group of sugar-modified nucleotides includes 2'-modified nucleosides, 4'-thio-modified nucleosides, 4'-thio-2'-modified nucleosides, and bicyclic sugar-modified nucleosides.

The 2'-modified nucleotides are, for example, halo, allyl, amino, azide, 0-allyl, O-C₁-C₁₀ alkyl, OCF₃, O-(CH2)₂-O-CH₃, 2'-O(CH₂)₂SCH₃, O-(CH₂)₂-O-N(Rₘ) (Rₙ), and O-CH₂-C(=O)-N(Rₘ) (Rₙ), wherein Rₘ and Rₙ are each individually H, an amino protective group, or substituted or unsubstituted C₁-C₁₀ alkyl. A preferable 2'-modification is -F, -OCH₃, or -O-(CH₂)₂-O-CH₃, more preferably -OCH₃.

Examples of the 4'-thio-modified nucleosides can include β-D-ribonucleosides in which the 4'-oxygen atom has been substituted by a sulfur atom (Hoshika, S. et al. FEBS Lett. 579, p. 3115-3118, (2005); Dande, P. et al. J. Med. Chem. 49, p. 1624-1634 (2006); and Hoshika, S. et al. ChemBioChem. 8, p. 2133-2138, (2007)).

Examples of the 4'-thio-2'-modified nucleosides can include 4'-thio-2'-modified nucleosides retaining 2'-H or 2'-O-methyl (Matsugami, et al. Nucleic Acids Res. 36, 1805 (2008)).

Examples of the bicyclic sugar-modified nucleosides can include nucleosides retaining the second ring formed by bridging two atoms of the ribose ring. Examples of such nucleosides can include: 2',4'-BNAs/LNAs (bridged nucleic acids/locked nucleic acids) in which the 2'-oxygen atom and the 4'-carbon atom are bridged by a methylene chain (Obika, S. et al. Tetrahedron Lett., 38, p. 8735- (1997).; Obika, S. et al., Tetrahedron Lett., 39, p. 5401- (1998).; A.A. Koshkin, A.A. et al. Tetrahedron, 54, p. 3607 (1998).; and Obika, S. Bioorg. Med. Chem., 9, p. 1001 (2001).); and ENAs (2'-O,4'-C-ethylene-bridged nucleic acids) bridged by an ethylene chain longer by one carbon than the methylene chain of the 2',4'-BNA/LNA (Morita, K, et al. Bioorg. Med. Chem. Lett., 12, p. 73(2002).; and Morita, K. et al. Bioorg. Med. Chem., 11, p. 2211 (2003).).

When arbitrary 1 to 4 2'-OMeRNA nucleotides in the 5'-Ar-double-stranded polynucleotide are substituted by sugar-modified nucleotides, more preferable sugar-modified nucleotides are, of the sugar-modified nucleotides described above, either identically or differently ENA or 2',4'-BNA/LNA counterparts of the nucleotides.

The 5'-Ar-double-stranded polynucleotide also includes a double-stranded polynucleotide in which 1 to 4 DNA nucleotides in the polynucleotide are identically or differently substituted by an RNA, an ENA, or a 2',4'-BNA/LNA.

### 2-2 Method for synthesizing sense strand in 5'-Ar-double-stranded polynucleotide

The method for preparing the sense strand polynucleotide constituting the 5'-Ar-double-stranded polynucleotide is not particularly limited as long as a desired polynucleotide can be synthesized. A known chemical synthesis method can be used, for example, a phosphotriester, phosphoramidite, or H-phosphonate method. For example, it can be synthesized using a commercially available nucleic acid synthesizer and commercially available reagents used in DNA/RNA synthesis.

### 2-3 Method for synthesizing 5'-phenylphosphate-modified antisense strand in 5'-Ar-double-stranded polynucleotide

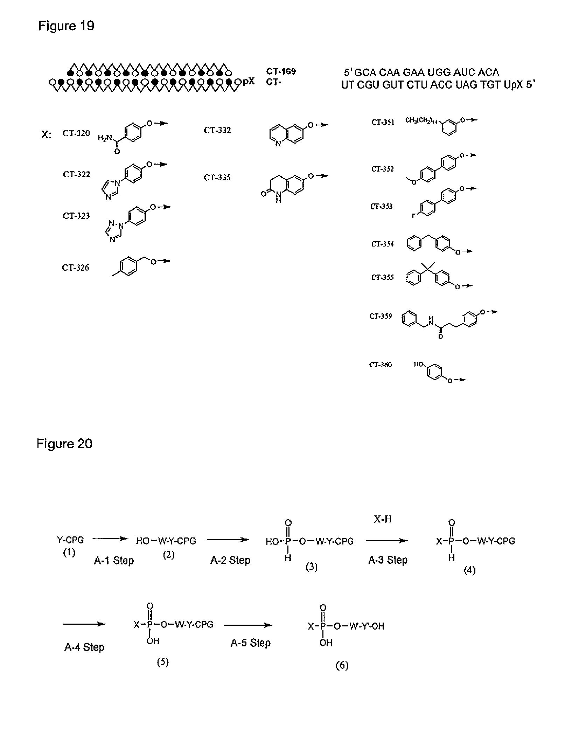

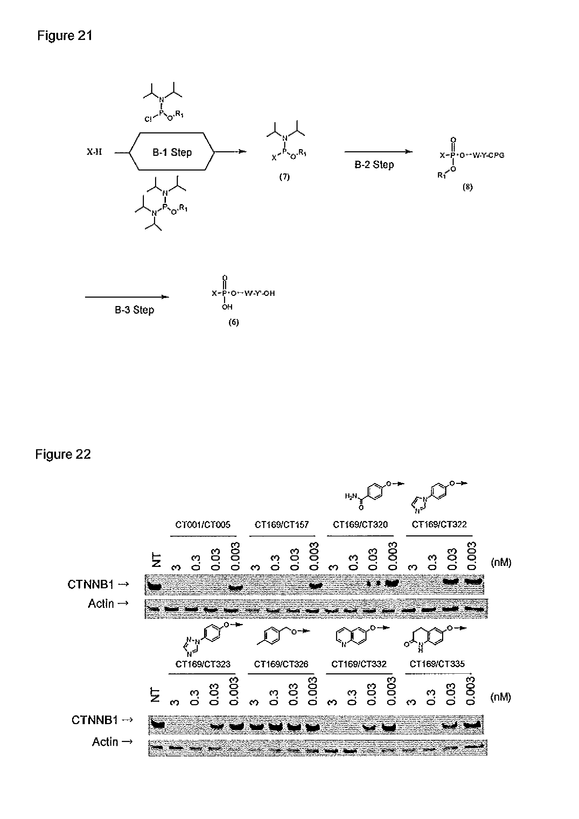

The 5'-phenylphosphate-modified antisense strand polynucleotide constituting the 5'-Ar-double-stranded polynucleotide is not particularly limited as long as the 5'-phenylphosphate-modified antisense strand can be synthesized. It can be synthesized by, for example, Method A (Figure 20) or Method B (Figure 21) shown below.

### 2-3-1 Method A

### 2-3-1-1 Step A-1

The present step is the step of producing compound 2 (referred to as HO-W-Y-CPG in Method A), which is an oligonucleotide analog consisting of the desired nucleotide sequence, using commercially available CPG (1) (controlled pore glass) bound with the desired nucleosides (referred to as Y-CPG in Method A).

Compound (2) is produced by a usual phosphoramidite method using an automatic DNA synthesizer and a phosphoramidite reagent, etc., necessary for producing compound 2. The oligonucleotide analog having the desired nucleotide sequence can be synthesized according to a method described in the literature (Nucleic Acids Research, 12, 4539 (1984)) using a DNA synthesizer, for example, model 392 based on the phosphoramidite method (manufactured by PerkinElmer Inc.).

Moreover, when the oligonucleotide analog is converted, if desired, to a thioate form, a thioate derivative can be obtained according to a method described in the literature (Tetarhedron Letters, 32, 3005 (1991); and J. Am. Chem. Soc., 112, 1253 (1990)) using sulfur or a reagent such as tetraethylthiuram disulfide (TETD, Applied Biosystems), Beaucage reagent, or a phenylacetyl disulfide/pyridine-acetonitrile (1:1 v/v) solution (Ravikumar, V.T. et al., Bioorg. Med. Chem. Lett. (2006) 16, p. 2513-2517).

### 2-3-2 Step A-2

The present step is the step of reacting the compound (2) produced in Step A-1 with tris-(1,2,4-triazolyl)phosphite or 2-chloro-4H-1,3,2-benzodioxaphosphorin-4-one in an inert solvent, and then converting the reaction product to a H-phosphonate form by the addition of water to produce compound (3).

The solvent used is not particularly limited as long as it does not influence the reaction. Preferable examples thereof can include: ethers such as tetrahydrofuran, diethyl ether, and dioxane; and halogenated hydrocarbons such as methylene chloride, chloroform, carbon tetrachloride, dichloroethane, chlorobenzene, and dichlorobenzene.

When 2-chloro-4H-1,3,2-benzodioxaphosphorin-4-one is used, a deoxidizer is used. In this case, examples of the deoxidizer used include: heterocyclic amines such as pyridine and dimethylaminopyridine; and aliphatic amines such as trimethylamine, triethylamine, and diisopropylamine. The deoxidizer is preferably an aliphatic amine (particularly, triethylamine).

The reaction temperature is not particularly limited and is usually -20 to 100°C, preferably 10 to 40°C.

The reaction time differs depending on the starting materials used, reagents, temperature, etc., and is usually 5 minutes to 30 hours, preferably 30 minutes for a reaction at room temperature.

After the completion of the reaction, the reaction solution can be separated from the CPG by filtration. After washing with an organic solvent such as pyridine or acetonitrile, an aqueous triethylamine carbonate solution is added to the reaction product, and the reaction product is washed again with acetonitrile and dried to obtain compound (3).

### 2-3-3 Step A-3

The present step is the step of condensing the compound (3) produced in Step A-2 and a compound having a hydroxy group (referred to as X-H in Method A) in the presence of a condensing agent (e.g., pivaloyl chloride) and a deoxidizer so that a H-phosphonic acid diester bond is formed to produce compound (4).

A solvent used in the present step which is not particularly limited as long as it does not inhibit the reaction. Preferably, anhydrous acetonitrile, anhydrous pyridine, or a mixture thereof is used.

Examples of the reagent used as the condensing agent can include acid chloride of carboxylic acid or phosphoric acid. Preferably, pivaloyl chloride or adamantanoic acid chloride is used.

Examples of the deoxidizer used include: heterocyclic amines such as pyridine and dimethylaminopyridine; and aliphatic amines such as trimethylamine, triethylamine, and diisopropylethylamine. The deoxidizer is preferably an aliphatic amine (particularly, triethylamine).

The reaction temperature is not particularly limited and is usually -50 to 50°C, preferably room temperature.

The reaction time differs depending on starting materials used, reagents, temperature, etc., and is usually 5 minutes to 30 hours, preferably 30 minutes for a reaction at room temperature.

After the completion of the reaction, the reaction solution can be separated from the CPG by filtration. The reaction product is washed with an organic solvent such as pyridine or acetonitrile and then dried to obtain compound (4).

### 2-3-4 Step A-4

The present step is the step of converting the H-phosphonic acid bond in the compound (4) produced in Step A-3 to a phosphodiester bond using an oxidizing agent to produce a compound (5).

The oxidizing agent used for oxidizing the H-phosphonic acid bond is not particularly limited as long as it is usually used in oxidation reactions. Examples thereof can include: inorganic metal oxidizing agents, for example, manganese oxides such as potassium permanganate and manganese dioxide; ruthenium oxides such as ruthenium tetroxide; selenium compounds such as selenium dioxide; iron compounds such as iron chloride; osmium compounds such as osmium tetroxide; silver compounds such as silver oxide; mercury compounds such as mercury acetate; lead oxide compounds such as lead oxide and lead tetroxide; chromic acid compounds such as potassium chromate, chromic acid-sulfuric acid complexes, and chromic acid-pyridine complexes, and cerium compounds such as cerium ammonium nitrate (CAN); inorganic oxidizing agents, for example, halogen molecules such as chlorine, bromine, and iodine molecules; periodic acids such as sodium periodate; ozone; aqueous hydrogen peroxide; nitrous acid compounds such as nitrous acid; chlorous acid compounds such as potassium chlorite and sodium chlorite; persulfuric acid compounds such as potassium persulfate and sodium persulfate; and organic oxidizing agents, for example, reagents used in DMSO oxidation (complexes of dimethyl sulfoxide and dicyclohexylcarbodiimide, oxalyl chloride, acetic acid anhydride, or phosphorus pentoxide, or pyridine-acetic acid anhydride complexes); peroxides such as t-butyl hydroperoxide; stable cations such as triphenylmethyl cations; succinimides such as N-bromosuccinimide; hypochlorous acid compounds such as t-butyl hypochlorite; azodicarboxylic acid compounds such as methyl azodicarboxylate; disulfides such as dimethyl disulfide, diphenyl disulfide, and dipyridyl disulfide, triphenylphosphine; nitrous acid esters such as methyl nitrite; tetrahalocarbon such as methane tetrabromide, and quinone compounds such as 2,3-dichloro-5,6-dicyano-p-benzoquinone (DDQ). The oxidizing agent is preferably an iodine molecule.

Examples of the deoxidizer used include: heterocyclic amines such as pyridine and dimethylaminopyridine; and aliphatic amines such as trimethylamine, triethylamine, and diisopropylethylamine. The deoxidizer is preferably pyridine.

The reaction temperature is not particularly limited and is usually -50 to 50°C, preferably room temperature.

The reaction time differs depending on the starting materials used, reagents, temperature, etc., and is usually 5 minutes to 30 hours, preferably 30 minutes for a reaction at room temperature.

After the completion of the reaction, the reaction solution can be separated from the CPG by filtration. The reaction product is washed with an organic solvent such as pyridine or acetonitrile and then dried to obtain compound (5).

### 2-3-5 Step A-5

The present step is the step of cleaving compound (5) produced in Step A-4 from the CPG, and removing the protective group to produce final compound (6) (in Methods A and B, -W'-Y'- represents the structure of an oligonucleotide excluding a 5'-hydroxy group and a 3'-hydroxy group).

Examples of a base used can include concentrated ammonia water, methanolic ammonia, ethanolic ammonia, and a concentrated ammonia water-ethanol (3:1 v/v) mixture. The base is preferably concentrated ammonia water or a concentrated ammonia water-ethanol (3:1 v/v) mixture.

The reaction temperature is not particularly limited and is usually -50 to 80°C, preferably room temperature to 60°C.

The reaction time differs depending on the starting materials used, reagents, temperature, etc., and is usually 5 minutes to 30 hours, preferably 5 hours for a reaction at 60°C. The reaction mixture containing compound (6) thus obtained can be purified by purification procedures used in usual nucleic acid purification, for example, various chromatography techniques such as reverse-phase and ion-exchange chromatography (including high-performance liquid chromatography) to obtain compound (6).

The outline of Method A is shown in Figure 20.

### 2-4 Step B

### 2-4-1 Step B-1

The present step is the step of reacting a compound having a hydroxy group (referred to as X-H in Method B) with mono-substituted chloro(alkoxy)phosphines or di-substituted alkoxyphosphines usually used in conversions to amidite, in an inert solvent to produce compound (7).

The solvent used is not particularly limited as long as it does not influence the reaction. Preferable examples thereof include: ethers such as tetrahydrofuran, diethyl ether, and dioxane; and halogenated hydrocarbons such as methylene chloride, chloroform, carbon tetrachloride, dichloroethane, chlorobenzene, and dichlorobenzene.

Examples of R₁ in Method B can include a cyanoethyl group, a methyl group, a methanesulfonylethyl group, and a 2,2,2-trichloroethyl group. R₁ is preferably a cyanoethyl group or a methyl group.

Examples of the mono-substituted chloro(alkoxy)phosphines used include phosphines such as chloro(morpholino)methoxyphosphine, chloro(morpholino)cyanoethoxyphosphine, chloro(dimethylamino)methoxyphosphine, chloro(dimethylamino)cyanoethoxyphosphine, chloro(diisopropylamino)methoxyphosphine, and chloro(diisopropylamino)cyanoethoxyphosphine. The mono-substituted chloro(alkoxy)phosphines are preferably chloro(morpholino)methoxyphosphine, chloro(morpholino)cyanoethoxyphosphine, chloro(diisopropylamino)methoxyphosphine, or chloro(diisopropylamino)cyanoethoxyphosphine.

When the mono-substituted chloro(alkoxy)phosphines are used, a deoxidizer is used. In this case, examples of the deoxidizer used include: heterocyclic amines such as pyridine and dimethylaminopyridine; and aliphatic amines such as trimethylamine, triethylamine, and diisopropylamine. The deoxidizer is preferably an aliphatic amine (particularly, diisopropylethylamine).

Examples of the di-substituted alkoxyphosphines used can include phosphines such as bis(diisopropylamino)cyanoethoxyphosphine, bis(diethylamino)methanesulfonylethoxyphosphine, bis(diisopropylamino)(2,2,2-trichloroethoxy)phosphine, and bis(diisopropylamino)(4-chlorophenylmethoxy)phosphine. The di-substituted alkoxyphosphines are preferably bis(diisopropylamino)cyanoethoxyphosphine.

When the di-substituted alkoxyphosphines are used, an acid is used. In this case, the acid used is preferably tetrazole, acetic acid, or p-toluenesulfonic acid.

The reaction temperature is not particularly limited and is usually 0 to 80°C, preferably room temperature.

The reaction time differs depending on the starting materials used, reagents, temperature, etc., and is usually 5 minutes to 30 hours, preferably 30 minutes to 10 hours for a reaction at room temperature.

After the completion of the reaction, the objective compound (7) of interest in the present reaction is obtained, for example, by: appropriately neutralizing the reaction mixture, or removing insoluble matter, if any, by filtration; then adding water and a water-immiscible organic solvent such as ethyl acetate; separating the organic layer containing the objective compound after washing with water; and drying the organic layer with anhydrous magnesium sulfate or the like; and then distilling off the solvent.

The objective compound obtained can be further purified, if necessary, by a standard method, for example, recrystallization, reprecipitation, or chromatography.

### 2-4-2 Step B-2

The present step is the step of reacting compound (2) produced in Step A-1 with a phosphoramidite form of compound (7) produced in step B-1 by a usual phosphoramidite method using an automatic DNA synthesizer to produce compound (8).

The desired compound (8) can be synthesized according to a method described in the literature (Nucleic Acids Research, 12, 4539 (1984)) using a DNA synthesizer, for example, model 392 based on the phosphoramidite method (manufactured by PerkinElmer Inc.).

Moreover, when the compound is converted, if desired, to a thioate form, a thioate derivative can be obtained according to a method described in the literature (Tetarhedron Letters, 32, 3005 (1991); and J. Am. Chem. Soc., 112, 1253 (1990)) using sulfur or a reagent such as tetraethylthiuram disulfide (TETD, Applied Biosystems), Beaucage reagent, or a phenylacetyl disulfide/pyridine-acetonitrile (1:1 v/v) solution (Ravikumar, V.T. et al., Bioorg. Med. Chem. Lett. (2006) 16, p. 2513-2517).

### 2-4-3 Step B-3

The present step is the step of excising the compound (8) produced in Step B-2 from CPG and removing the protective group to produce final compound (6).

The present step can be performed in the same way as Step A-5.

The outline of Method B is shown in Figure 21.

### 2-5 Preparation of double-stranded polynucleotide

A sense strand single-stranded polynucleotide and an antisense strand single-stranded polynucleotide having complementarity to each other can be synthesized separately and associated by an appropriate method to form a duplex. A specific example of the association method includes a method by which the synthesized single-stranded polynucleotides are mixed at a molar ratio of preferably at least 3:7, more preferably approximately 4:6, most preferably equimolar ratio (5:5), then heated to a dissociation temperature of the duplex, and then gradually cooled. The associated double-stranded polynucleotide is purified, if necessary, by a method usually used and known per se in the art. For example, a method can be used as the purification method, by which the association is confirmed using an agarose gel or the like, and residual single-stranded polynucleotides are removed, for example, by degradation with an appropriate enzyme. A 5'-Ar-double-stranded polynucleotide and a double-stranded polynucleotide having an unmodified 5'-terminal phosphate can be obtained by the present method.

The 5'-Ar-double-stranded polynucleotide also includes: a 5'-Ar-double-stranded polynucleotide comprising a cholesterol, lipid, or vitamin E unit introduced therein (see e.g., Lorenz, C. et al. Bioorg. Med. Chem. Lett., 14, p. 4975-4977 (2004); Soutschek, J., et al. Nature, 432, p. 173-178, (2004); Wolfrum, C. et al. Nature Biotech. 25, p. 1149-1157, (2007); Kubo, T. et al. Oligonucleotides, 17, p. 1-20, (2007); Kubo, T., et al. Biochem. Biophys. Res. Comm. 365, p. 54-61, (2008); and Nishina, K., et al., Mol. Ther. 16, p. 734-740, (2008)); and a 5'-Ar-double-stranded polynucleotide bound to an aptamer, which is a protein-binding nucleic acid molecule, at the end.

The 5'-Ar-double-stranded polynucleotide also includes a 5'-Ar-double-stranded polynucleotide bound to a monoclonal antibody (or an appropriate binding site thereof) or a protein (or an appropriate oligopeptide fragment thereof) (see e.g., Song, et al. Nature Biotech. 23, p. 709-717 (2005); Xia et al. Pharm. Res. 24, p. 2309-2316 (2007); and Kumar, et al. Nature, 448, p. 39-43 (2007)).

Moreover, the 5'-Ar-double-stranded polynucleotide also includes a positively charged complex of a 5'-Ar-double-stranded polynucleotide supplemented with a cationic polymer (see, as successful examples achieving distribution in organs and cells, Leng et al. J. Gen. Med. 7, p. 977-986 (2005); Baigude et al. 2, p.237-241, ACS Chem. Biol. (2007); and Yadava et al. Oligonucleotide 17, p. 213-222 (2007)).

The 5'-Ar-double-stranded polynucleotide includes every pharmaceutically acceptable salt or ester of the 5'-Ar-double-stranded polynucleotide, or salts of such esters.

Preferable examples of the pharmaceutically acceptable salt of the 5'-Ar-double-stranded polynucleotide can include: metal salts, for example, alkali metal salts such as a sodium salt, a potassium salt, and a lithium salt, alkaline earth metal salts such as a calcium salt and a magnesium salt, an aluminum salt, an iron salt, a zinc salt, a copper salt, a nickel salt, and a cobalt salt; amine salts including inorganic salts such as an ammonium salt and organic salts such as a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-phenethylamine salt, a piperazine salt, a tetramethylammonium salt, a tris(hydroxymethyl)aminomethane salt; inorganic acid salts, for example, a hydrohalide such as a hydrofluoride, a hydrochloride, a hydrobromide, and a hydroiodide, a nitrate, a perchlorate, a sulfate, and a phosphate; organic acid salts, for example, lower alkanesulfonates such as a methanesulfonate, a trifluoromethanesulfonate, and an ethanesulfonate, arylsulfonates such as a benzenesulfonate and a p-toluenesulfonate, an acetate, a malate, a fumarate, a succinate, a citrate, a tartrate, an oxalate, and a maleate; and amino acid salts such as a glycine salt, a lysine salt, an arginine salt, an ornithine salt, a glutamate, and an aspartate.

A composition including the 5'-Ar-double-stranded polynucleotide is mixed, enclosed, or conjugated with another molecule, molecular structure, or mixture of compounds, for example, as a liposome, a receptor-targeting molecule, an oral, rectal, or local formulation, or other formulations for assisting in uptake, distribution, and/or absorption.

When the 5'-Ar-double-stranded polynucleotide is used as a prophylactic or therapeutic drug for disease, the polynucleotide or a pharmacologically acceptable salt thereof can be administered either by itself or after mixing with an appropriate pharmacologically acceptable excipient, diluent, or the like, as an oral formulation such as tablets, capsules, granules, powders, or syrups or as a parenteral formulation such as injections, suppositories, patches, or topical preparations.

These formulations are produced by well-known methods using additives such as excipients (examples thereof can include organic excipients including: sugar derivatives such as lactose, saccharose, glucose, mannitol, and sorbitol; starch derivatives such as corn starch, potato starch, α starch, and dextrin; cellulose derivatives such as crystalline cellulose; gum arabic; dextran; and pullulan, and inorganic excipients including: silicate derivatives such as light anhydrous silicic acid, synthetic aluminum silicate, calcium silicate, and magnesium aluminometasilicate; a phosphate such as calcium hydrogen phosphate; a carbonate such as calcium carbonate; and a sulfate such as calcium sulfate), lubricants (examples thereof can include: metal salts of stearic acid such as stearic acid, calcium stearate, and magnesium stearate; talc; colloidal silica; waxes such as beeswax and spermaceti; boric acid; adipic acid; a sulfate such as sodium sulfate; glycol; fumaric acid; sodium benzoate; DL leucine; a lauryl sulfate such as sodium lauryl sulfate and magnesium lauryl sulfate; silicates such as anhydrous silicate and silicate hydrate; and the starch derivatives described above), binders (examples thereof can include hydroxypropylcellulose, hydroxypropylmethylcellulose, polyvinylpyrrolidone, macrogol, and the same compounds as the excipients described above), disintegrants (examples thereof can include: cellulose derivatives such as low substituted hydroxypropylcellulose, carboxymethylcellulose, carboxymethylcellulose calcium, and internally bridged carboxymethylcellulose sodium; and chemically modified starches/celluloses such as carboxymethyl starch, carboxymethyl starch sodium, and bridged polyvinylpyrrolidone), emulsifers (examples thereof can include: colloidal clay such as bentonite and veegum; a metal hydroxide such as magnesium hydroxide and aluminum hydroxide; anionic surfactants such as sodium lauryl sulfate and calcium stearate; cationic surfactants such as benzalkonium chloride; and nonionic surfactants such as polyoxyethylene alkyl ether, polyoxyethylene sorbitan fatty acid ester, and sucrose fatty acid ester), stabilizers (examples thereof can include: p-oxybenzoic acid esters such as methylparaben and propylparaben; alcohols such as chlorobutanol, benzyl alcohol, and phenylethyl alcohol; benzalkonium chloride; phenols such as phenol and cresols; thimerosal; dehydroacetic acid; and sorbic acid), corrigents (examples thereof can include sweeteners, acidulants, and flavors that are usually used), and diluents.

### 3. Introduction of a double-stranded polynucleotide into cells, tissues, or individuals, and regulation of expression of a target gene

Recipients to which or to whom the 5'-Ar-double-stranded polynucleotide thus prepared is introduced are not particularly limited as long as the target gene can be intracellularly transcribed into RNA or translated into a protein therein. The recipients mean cells, tissues, or individuals.

The cells for which the 5'-Ar-double-stranded polynucleotide is used may be any of germline cells, somatic cells, totipotent cells, pluripotent cells, cleaved cells, non-cleaved cells, parenchymal cells, epithelial cells, immortalized cells, transformed cells, nerve cells, and immunocytes.

The tissues include 1-cell embryos or constitutive cells, or polyploid embryos, embryonic tissues, or the like. Moreover, examples of the above differentiated cells include adipocytes, fibroblasts, myocytes, cardiomyocytes, endothelial cells, nerve cells, glial cells, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes, and endocrine or exocrine cells. For example, CHO-K1 cells (RIKEN Cell bank), *Drosophila* S2 cells (Schneider, 1. et al., J. Embryol. Exp. Morph., 27, p. 353-365 (1972)), human HeLa cells (ATCC: CCL-2), and human HEK293 cells (ATCC: CRL-1573) are preferably used as such cells.

Furthermore, specific examples of the individuals used as recipients of the 5'-Ar-double-stranded polynucleotide include plants, animals, protozoans, viruses, bacteria, and those belonging to the *Eumycetes.* The plants may be monocots, dicots, or gymnosperms. The animals may be vertebrates or invertebrates. The vertebrates are preferably mammals including mice, rats, monkeys, dogs, and humans.

When the recipients are cells or tissues, a calcium phosphate method, electroporation, a lipofection method, viral infection, immersion in a 5'-Ar-double-stranded polynucleotide solution, or transformation, or the like is used as a method for introducing the double-stranded polynucleotide into the recipients. Moreover, examples of a method for introduction into embryos include microinjection, electroporation, and viral infection. When the recipients are plants, a method involving injection or perfusion into the cavities or interstitial cells or the like of the plants or spraying thereonto is used. Moreover, for animal individuals, a method involving systemic introduction through, for example, oral, local, hypodermic, intramuscular, intravenous, parenteral, transvaginal, rectal, nasal, ocular, or transmucosal administration, or electroporation, viral infection, or the like is used. A method by which the double-stranded polynucleotide is directly mixed with a diet for the organisms can also be used as an oral introduction method.

In addition to these approaches, a colloidal dispersion system can be used as a method for introducing the 5'-Ar-double-stranded polynucleotide into patients.

The colloidal dispersion system is expected to have the effect of enhancing the in vivo stability of the compound or the effect of efficiently transporting the compound to particular organs, tissues, or cells.

The colloidal dispersion system is not limited as long as it is usually applicable. Examples thereof can include polymer complexes, nanocapsules, microspheres, beads, and oil-in-water emulsifying agents, and lipid-based dispersion systems including micelles, mixed micelles and liposomes. Preferably, the colloidal dispersion system is a plurality of liposomes or artificial membrane vesicles having the effect of efficiently transporting the compound to particular organs, tissues, or cells (Mannino et al., Biotechniques, 1988, 6, p. 682-; Blume and Cevc, Biochem.et Biophys. Acta, 1990, 1029, p. 91-; Lappalainen et al., Antiviral Res., 1994, 23, p. 119-; and Chonn and Cullis, Current Op. Biotech., 1995, 6, p. 698-).

Unilamellar liposomes of 0.2 to 0.4 µm in size range are capable of enclosing a considerable amount of an aqueous buffer containing macromolecules, and the contents are enclosed in this aqueous inner membrane and transported in a biologically active form to brain cells (Fraley et al., Trends Biochem. Sci., 1981, 6, p. 77-).

The liposome composition is usually a complex of lipid, particularly phospholipid, specifically phospholipid having a high phase transition temperature, with one or more steroids, particularly cholesterols.

Examples of the lipid useful for liposome production include phosphatidyl compounds such as phosphatidyl glycerol, phosphatidyl choline, phosphatidyl serine, sphingolipid, phosphatidyl ethanolamine, cerebroside, and ganglioside.

Diacyl phosphatidyl glycerol is particularly useful, wherein the lipid moiety contains 14 to 18 carbon atoms and is saturated (lacking any internal double bonds in the chain of 14 to 18 carbon atoms) and, preferably, contains 16 to 18 carbon atoms.

Typical phospholipids encompass phosphatidyl choline, dipalmitoyl phosphatidyl choline, and distearoyl phosphatidyl choline.

Targeting by the colloidal dispersion system including liposomes may be passive or active.

Such passive targeting is achieved by use of the fundamental tendency of liposomes to be distributed to reticuloendothelial cells in organs containing sinusoids.

On the other hand, examples of the active targeting can include liposome modification approaches involving binding particular ligands such as viral coat protein (Morishita et al., Proc. Natl. Acad. Sci. (U.S.A.), 1993, 90, p. 8474-), monoclonal antibodies (or appropriate binding sites thereof), sugars, glycolipids, or proteins (or appropriate oligopeptide fragments thereof) to liposomes or changing liposome composition to achieve distribution to organs and cell types other than naturally occurring sites of localization.

The surface of the targeted colloidal dispersion system may be modified in various ways.

In the liposomal targeted delivery system, a lipid group can be incorporated into the lipid bilayer of the liposome to maintain target ligands through tight association with the lipid bilayer.
Various linking groups may be used for linking the lipid chain with the target ligands.

The target ligands binding to particular cell surface molecules predominantly found on cells desired to receive the delivery of the 5'-Ar-double-stranded polynucleotide can be, for example, (1) hormones, growth factors, or appropriate oligopeptide fragments thereof, binding to particular cell receptors predominantly expressed by the cells desired to receive the delivery, or (2) polyclonal or monoclonal antibodies or appropriate fragments thereof (e.g., Fab or F(ab')₂) specifically binding to antigenic epitopes predominantly found on the target cells.
Two or more bioactive agents can also be compounded within a single liposome and administered.

A medicament for enhancing the intracellular stability of the contents and/or targeting may further be added to the colloidal dispersion system.

The amount of the 5'-Ar-double-stranded polynucleotide or pharmacologically acceptable salt thereof used differs depending on symptoms, age, etc. 1 mg (preferably, 30 mg) as the lower limit to 2000 mg (preferably, 1500 mg) as the upper limit of the polynucleotide or the salt thereof each time for oral administration or 0.5 mg (preferably, 5 mg) as the lower limit to 500 mg (preferably, 250 mg) as the upper limit of the polynucleotide or the salt thereof each time for intravenous administration is preferably administered to an adult once to six times a day according to symptoms.

Pharmaceutical compositions and formulations for local administration include transdermal patches, ointments, lotions, creams, gels, troches, suppositories, sprays, liquids, and powders.

### Examples

Hereinafter, the present invention will be described more specifically with reference to Examples, Reference Examples, and Test Examples. However, the present invention is not intended to be limited to them. In the Examples below, procedures of genetic engineering were performed by the methods described in "Molecular Cloning" [Sambrook, J., Fritsch, E.F. and Maniatis, T., published in 1989 by Cold Spring Harbor Laboratory Press] or according to the instructions of the commercially available reagents or kits used, unless otherwise specified. The structural formula of X in each polynucleotide synthesized in Examples and the molecular weight measured value of each polynucleotide measured with a mass spectrometer are shown in Table 1.

### (Example 1)

### Synthesis of HO-G^{p}-C^{m1p}-AP-C^{m1p}-A^{p}-A^{m1p}-G^{p}-A^{m1p}-A^{p}-U^{m1p}-G^{p}-G^{m1p}-A^{p}-U^{m1p}-C^{p}-A^{m1p}-C^{p}-A^{m1p}-H (SEQ ID NO: 1 of the Sequence Listing) (CT-169)

CT-169 was synthesized according to an RNA synthesis program on the scale of 0.2 µmol using an automatic nucleic acid synthesizer (manufactured by PerkinElmer Inc., ABI model 394 DNA/RNA synthesizer). Solvents, reagents, and phosphoramidites were used in each synthesis cycle at the same concentrations as in natural oligodeoxynucleotide synthesis.

When deoxynucleoside phosphoramidites were used, 5'-O-dimethoxytrityl-6-N-benzoyl-2'-deoxyadenosine-3'-O-(2-cyanoethyl N,N-diisopropyl)phosphoramidites, 5'-O-dimethoxytrityl-2-N-isobutyryl-2'-deoxyguanosine-3'-O-(2-cyanoethyl N,N-diisopropyl)phosphoramidites, 5'-O-dimethoxytrityl-4-N-benzoyl-2'-deoxycytidine-3'-O-(2-cyanoethyl N,N-diisopropyl)phosphoramidites, and 5'-O-dimethoxytritylthymidine-3'-O-(2-cyanoethyl N,N-diisopropyl)phosphoramidites were purchased from Proligo and appropriately adjusted for use.

When 2'-O-methyl nucleoside phosphoramidites were used, 5'-O-dimethoxytrityl-6-N-benzoyl-2'-O-methyladenosine-3'-O-(2-cyanoethyl N,N-diisopropyl)phosphoramidites, 5'-O-dimethoxytrityl-2-N-isobutyryl-2'-O-methylguanosine-3'-O-(2-cyanoethyl N,N-diisopropyl)phosphoramidites, 5'-O-dimethoxytrityl-4-N-acetyl-2'-O-methylcytidine-3'-O-(2-cyanoethyl N,N-diisopropyl)phosphoramidites, and 5'-O-dimethoxytrityl-2'-O-methyluridine-3'-O-(2-cyanoethyl N,N-diisopropyl)phosphoramidites were purchased from Glen Research Corp. and appropriately adjusted for use.

When ribonucleoside phosphoramidites were used, 5'-O-dimethoxytrityl-6-N-benzoyl-2'-O-(tertbutyldimethylsilyl)adenosine-3'-O-(2-cyanoethyl N,N-diisopropyl)phosphoramidites, 5'-O-dimethoxytrityl-2-N-dimethylformamidine-2'-O-(tertbutyldimethylsilyl)guanosine-3'-O-(2-cyanoethyl N,N-diisopropyl)phosphoramidites, 5'-O-dimethoxytrityl-4-N-acetyl-2'-O-(tert-butyldimethylsilyl)cytidine-3'-O-(2-cyanoethyl N,N-diisopropyl)phosphoramidites, and 5'-O-dimethoxytrityl-2'-O-(tert-butyldimethylsilyl)uridine-3'-O-(2-cyanoethyl N,N-diisopropyl)phosphoramidites were purchased from Proligo and appropriately adjusted for use.
When 2'-O,4'-C-ethylene nucleoside phosphoramidites were used, compounds of Example 14 (5'-O-dimethoxytrityl-2'-O,4'-C-ethylene-6-N-benzoyladenosine-3'-O-(2-cyanoethyl N,N-diisopropyl)phosphoramidites), Example 27 (5'-O-dimethoxytrityl-2'-O,4'-C-ethylene-2-N-isobutyrylguanosine-3'-O-(2-cyanoethyl N,N-diisopropyl)phosphoramidites), Example 22 (5'-O-dimethoxytrityl-2'-O,4'-C-ethylene-4-N-benzoyl-5-methylcytidine-3'-O-(2-cyanoethy N,N-diisopropyl)phosphoramidites), and Example 9 (5'-O-dimethoxytrityl-2'-O,4'-C-ethylene-5-methyluridine-3'-O-(2-cyanoethyl N,N-diisopropyl)phosphoramidites) of Japanese Patent No. 3420984 were appropriately prepared for use.

When each polynucleotide has a 5'-terminal phosphate group moiety, PHOSPHALINK (manufactured by Applied Biosystems) was appropriately adjusted for use.

The phosphoramidites were appropriately supplied to the automatic nucleic acid synthesizer to synthesize a polynucleotide having the desired sequence. 0.5 µmol of CPG (controlled pore glass; manufactured by Applied Biosystems or Glen Research Corp.) bound with the desired nucleosides was used as a solid-phase carrier to synthesize the title polynucleotide. In the final step of the automatic nucleic acid synthesizer, acid treatment was not performed (the dimethoxytrityl group was bound to the oligonucleotide). The present polynucleotide was treated with ammonia water and then purified by reverse-phase HPLC (LC-10VP manufactured by Shimadzu Corp., column (Merck, Chromolith Performance RP-18e (4.6×100 mm)), Solution A: 5% acetonitrile, 0.1 M aqueous triethylamine acetate solution (TEAA), pH 7.0, Solution B: acetonitrile, B%: 10%→60% (10 min, linear gradient); 60°C; 2 ml/min; 260 nm) to gather peaks of the product of interest having the dimethoxytrityl group. Water was added thereto, and TEAA was distilled off under reduced pressure. When the dimethoxytrityl group was bonded thereto, an 80% aqueous acetic acid solution (2 mL) was added thereto, and the mixture was left for 20 minutes to deprotect the dimethoxytrityl group. The solvent was distilled off, and the residue was dissolved in 500 µl of water, washed with ethyl acetate, and freeze-dried to obtain the oligonucleotide of interest. Moreover, if necessary, the obtained precipitates were purified by 20% polyacrylamide gel electrophoresis containing 7 M urea (1x TBE, 600 V, 4 hours). After the electrophoresis, bands were visualized using a UV lamp, and the bands of interest were excised using a knife. 1 mL of a solution containing 0.2 M NaCl and 10 mM EDTA (pH 7.2) was added thereto, and the mixture was left overnight to elute the polynucleotide from the gel slice. The oligonucleotide was precipitated by the addition of ethanol and collected by centrifugation. The molecular weight of the present polynucleotide was identified by negative ion ESI mass spectrometry.
Molecular weight calculated value: 5767.86, measured value: 5767.78
Nucleotide sequence: comprising a sequence of nucleotide Nos. 3139-3156 of the human β-catenin gene (GenBank accession No. NM_001904.3)

### (Example 2)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-259)

CT-259 was synthesized in the same way as for Example 1. In the final step of the automatic nucleic acid synthesizer, the present polynucleotide was treated with an acid, and the X moiety was manually condensed. Specifically, CPG bound with the nucleic acid moiety was removed from the automatic nucleic acid synthesizer. A 1/6 aliquot of a solution of 2-chloro-4H-1,3,2-benzodioxaphosphorin-4-one (20.2 mg, 100 µmol) dissolved in 1 mL of THF and 10 µL of triethylamine was added to the CPG and reacted at room temperature for 30 minutes. The CPG was washed with 2 mL of acetonitrile and 2 mL of a 50 mM aqueous triethylamine carbonate solution, washed again with acetonitrile, and dried. N-benzyloxycarbonyl-L-tyrosine ethyl ester (2 mg, Journal of Organic Chemistry 59; 9; 1994; 2304-2313) and pivaloyl chloride (5 µL, 40 µmol) were dissolved in pyridine:acetonitrile (1:1 v/v, 150 µL), and this solution was reacted with the CPG at room temperature for 30 minutes. After washing with acetonitrile, an iodine solution (manufactured by Proligo) as a reagent for a nucleic acid synthesizer was sent thereto for 30 seconds using a nucleic acid synthesizer, and the CPG was left for 5 minutes. The CPG was washed again with acetonitrile and dried, and deprotection and purification were performed in the same way as for Example 1.
Molecular weight calculated value: 6687.20, measured value: 6686.22
Nucleotide sequence: comprising a sequence complementary to nucleotide Nos. 3139-3156 of the human β-catenin gene (GenBank accession No. NM_001904.3)

### (Example 3)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-260)

CT-260 was synthesized in the same way as for Example 2. For the present polynucleotide, methyl 2-(2-furylcarbonylamino)-3-(4-hydroxyphenyl)propanoate (Journal of Medicinal Chemistry; 16; 1973; 281) was condensed therein in the final step.

### (Example 4)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-261)

CT-261 was synthesized in the same way as for Example 2. For the present polynucleotide, methyl (S)-3-(4-Hydroxy-phenyl)-2-[(pyridine-2-carbonyl)-amino]-propionic acid ethyl ester (Bioorganic & Medicinal Chemistry Letters; 11; 11; 2001; 1441 - 1444) was condensed therein in the final step.

### (Example 5)

### Synthesis of X-P(=O) (OH)-O-T^{p}-G^{m1p}-T^{p}-C^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-263)

CT-263 was synthesized in the same way as for Example 2. For the present polynucleotide, 4-propylphenol was condensed therein in the final step.

### (Example 6)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-268)

CT-268 was synthesized in the same way as for Example 1. For the present polynucleotide, the X moiety was condensed with an amidite reagent prepared as follows: p-cresol (20 mg) was dissolved in 2 mL of acetonitrile:methylene chloride (1:1 v/v). To this solution, 2-cyanoethyl tetraisopropylphosphorodiamidite (74 µL, 0.23 mmol) and 360 µL of a 0.45 M solution of 1H-tetrazole in acetonitrile were added, and the mixture was stirred for 2 hours. The progression of reaction was confirmed by TLC, followed by filter filtration to prepare the amidite reagent.

### (Example 7)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-269)

CT-269 was synthesized in the same way as for Example 6. The amidite reagent for the X moiety in the present polynucleotide was prepared using 4-ethylphenol (20 mg).

### (Example 8)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-270)

CT-270 was synthesized in the same way as for Example 6. The amidite reagent for the X moiety in the present polynucleotide was prepared using 4-isopropylphenol (20 mg).

### (Example 9)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-271)

CT-271 was synthesized in the same way as for Example 6. The amidite reagent for the X moiety in the present polynucleotide was prepared using 4-sec-butylphenol (20 mg).

### (Example 10)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-272)

CT-272 was synthesized in the same way as for Example 6. The amidite reagent for the X moiety in the present polynucleotide was prepared using 4-butylphenol (20 mg).

### (Example 11)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-273)

CT-273 was synthesized in the same way as for Example 6. The amidite reagent for the X moiety in the present polynucleotide was prepared using 4-amylphenol (20 mg).

### (Example 12)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-274)

CT-274 was synthesized in the same way as for Example 6. The amidite reagent for the X moiety in the present polynucleotide was prepared using 4-octylphenol (20 mg).

### (Example 13)

### Synthesis of X-P(=O) (OH) -O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-275)

CT-275 was synthesized in the same way as for Example 6. The amidite reagent for the X moiety in the present polynucleotide was prepared using 4-cyclohexylphenol (20 mg).

### (Example 14)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-276)

CT-276 was synthesized in the same way as for Example 6. The amidite reagent for the X moiety in the present polynucleotide was prepared using 5,6,7,8-tetrahydro-2-naphthol (20 mg).

### (Example 15)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-C^{p}-U^{m1p}-G^{p}-C^{m1p}-T^{p}-U^{m1t}-H (SEQ ID NO: 2 of the Sequence Listing) (CT-277)

CT-277 was synthesized in the same way as for Example 6. The amidite reagent for the X moiety in the present polynucleotide was prepared using 5-hydroxyindane (20 mg).

### (Example 16)

### Synthesis of X-P(=O) (OH) -O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-290)

CT-290 was synthesized in the same way as for Example 6. The amidite reagent for the X moiety in the present polynucleotide was prepared using 3-ethylphenol (20 mg).

### (Example 17)

### Synthesis of X-P(=O) (OH)-O-T^{p}-C^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-292)

CT-292 was synthesized in the same way as for Example 6. The amidite reagent for the X moiety in the present polynucleotide was prepared using 4-phenylphenol (20 mg).

### (Example 18)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-293)

CT-293 was synthesized in the same way as for Example 6. The amidite reagent for the X moiety in the present polynucleotide was prepared using 3-phenylphenol (20 mg).

### (Example 19)

### Synthesis of X-P(=O) (OH)-O-T^{p}-G^{m1p}-T^{p}-C^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-295)

CT-295 was synthesized in the same way as for Example 6. The amidite reagent for the X moiety in the present polynucleotide was prepared using 4-(benzyloxy)phenol (20 mg).

### (Example 20)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-296)

CT-296 was synthesized in the same way as for Example 6. The amidite reagent for the X moiety in the present polynucleotide was prepared using 3-(benzyloxy)phenol (20 mg).

### (Example 21)

### Synthesis of X-P(=O) (OH) -O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-299)

CT-299 was synthesized in the same way as for Example 6. The amidite reagent for the X moiety in the present polynucleotide was prepared using 2-naphthol (20 mg).

### (Example 22)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-300)

CT-300 was synthesized in the same way as for Example 6. The amidite reagent for the X moiety in the present polynucleotide was prepared using 4-ethylphenol (20 mg).
Nucleotide sequence: comprising a sequence complementary to nucleotide Nos. 3139-3157 of the human β-catenin gene (GenBank accession No. NM_001904.3)

### (Example 23)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-312)

CT-312 was synthesized in the same way as for Example 6. The amidite reagent for the X moiety in the present polynucleotide was prepared using 4-phenylphenol (20 mg).

### (Example 24)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-313)

CT-313 was synthesized in the same way as for Example 6. The amidite reagent for the X moiety in the present polynucleotide was prepared using 4-phenoxyphenol (20 mg).

### (Example 25)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-314)

CT-314 was synthesized in the same way as for Example 6. The amidite reagent for the X moiety in the present polynucleotide was prepared using 4-methoxyphenol (20 mg).

### (Example 26)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-T^{p}-U^{m1p}-G^{p}-C^{m1p}-T^{p}-U^{m1t}-H (SEQ ID NO: 3 of the Sequence Listing) (CT-315)

CT-315 was synthesized in the same way as for Example 6. The amidite reagent for the X moiety in the present polynucleotide was prepared using 4-(trifluoromethoxy)phenol (20 mg).

### (Example 27)

### Synthesis of X-P(=O) (OH)-O-^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-316)

CT-316 was synthesized in the same way as for Example 6. The amidite reagent for the X moiety in the present polynucleotide was prepared using 4-(trifluoromethyl)phenol (20 mg).

### (Example 28)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-317)

CT-317 was synthesized in the same way as for Example 6. The amidite reagent for the X moiety in the present polynucleotide was prepared using 4-fluorophenol (20 mg).

### (Example 29)

### Synthesis of X-P(=O) (OH)-O-U^{m1p}-T^{p}-G^{m1p}-T^{p}-G^{1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-318)

CT-318 was synthesized in the same way as for Example 6. The amidite reagent for the X moiety in the present polynucleotide was prepared using 4-chlorophenol (20 mg).
Molecular weight calculated value: 6820.28, measured value: 6820.67
Nucleotide sequence: comprising a sequence complementary to nucleotide Nos. 3139-3157 of the human β-catenin gene (GenBank accession No. NM_001904.3)

### (Example 30)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-319)

CT-319 was synthesized in the same way as for Example 6. The amidite reagent for the X moiety in the present polynucleotide was prepared using 4-acetylphenol (20 mg).

### (Example 31)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-321)

CT-321 was synthesized in the same way as for Example 6. The amidite reagent for the X moiety in the present polynucleotide was prepared using 4-(methylsulfonyl)phenol (20 mg).

### (Example 32)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-325)

CT-325 was synthesized in the same way as for Example 6. The amidite reagent for the X moiety in the present polynucleotide was prepared using 4-methylphenol (20 mg).

### (Example 33)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-331)

CT-331 was synthesized in the same way as for Example 6. The amidite reagent for the X moiety in the present polynucleotide was prepared using 2-naphthol (20 mg).

### (Example 34)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-336)

CT-336 was synthesized in the same way as for Example 6. The amidite reagent for the X moiety in the present polynucleotide was prepared using 2-hydroxy-9-fluorenone (20 mg).

### (Example 35)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-337)

CT-337 was synthesized in the same way as for Example 6. The amidite reagent for the X moiety in the present polynucleotide was prepared using 3-phenylphenol (20 mg).

### (Example 36)

### Synthesis of X-P(=O) (OH)-O-U^{m1p}-T^{p}-G^{m1p}-T^{p}-C^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-338)

CT-338 was synthesized in the same way as for Example 6. The amidite reagent for the X moiety in the present polynucleotide was prepared using 2-hydroxydibenzofuran (20 mg).

### (Example 37)

### Synthesis of X-P(=O) (OH)-O-U^{rp}-U^{rp}-G^{rp}-U^{rp}-G^{rp}-A^{rp}-U^{rp}-C^{rp}-C^{rp}-A^{rp}-U^{rp}-U^{rp}-C^{rp}-U^{rp}-U^{rp}-G^{rp}-U^{rp}-G^{rp}-C^{rp}-U^{rp}-U^{rt}-H (SEQ ID NO: 4 of the Sequence Listing) (CT-339)

CT-339 was synthesized in the same way as for Example 1. The amidite reagent for the X moiety in the present polynucleotide was prepared using 3-phenylphenol (20 mg). For the present polynucleotide, the protected polynucleotide analog having the sequence of interest was treated with 2 mL of an ammonia water:ethanol solution (3:1 v/v) at 55°C for 16 hours to cleave the oligomer from the support and to remove the cyanoethyl group acting as a protective group for the phosphate group and the protective group on the base of the nucleic acid. CPG was removed by filtration. After washing with ethanol, the filtrate and the wash were combined, and the solvent was distilled off under reduced pressure. To the residue, 0.3 mL of triethylamine trihydrofluoride was added, and the mixture was left at room temperature for 19 hours. 60 µL of H₂O and 3 mL of n-butanol were added thereto, and the mixture was left at -20°C for 1 hour. Then, precipitates were collected by centrifugation. The obtained precipitates were dissolved in 200 µL of H₂O and purified by 20% polyacrylamide gel electrophoresis containing 7 M urea (1x TBE, 600 V, 4 hours). After the electrophoresis, bands were visualized using a UV lamp, and the bands of interest were excised using a knife. 1 mL of a solution containing 0.2 M NaCl and 10 mM EDTA (pH 7.2) was added thereto, and the mixture was left overnight to elute the polynucleotide from the gel slice. The oligonucleotide was precipitated by the addition of ethanol and collected by centrifugation.
Nucleotide sequence: comprising a sequence complementary to nucleotide Nos. 3139-3157 of the human β-catenin gene (GenBank accession No. NM_001904.3)

### (Example 38)

### Synthesis of HO-G^{s}-C^{m1s}-A^{s}-C^{m1s}-A^{s}-A^{m1s}-G^{s}-A^{m1s}-A^{s}-G^{m1s}-G^{s}-G^{m1s}-A^{s}-U^{m1s}-G^{s}-A^{m1s}-C^{s}-A^{m1t}-H (SEQ ID NO: 5 of the Sequence Listing) (CT-310)

CT-310 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.
Nucleotide sequence: comprising a sequence of nucleotide Nos. 3139-3156 of the human β-catenin gene (GenBank accession No. NM_001904.3)

### (Example 39)

### Synthesis of X-P(=S) (OH)-O-U^{m1s}-T^{s}-G^{m1s}-T^{s}-G^{m1s}-A^{s}-U^{m1s}-C^{s}-C^{m1s}-A^{s}-U^{m1s}-T^{s}-C^{m1s}-T^{s}-U^{m1s}-G^{s}-U^{m1s}-G^{s}-C^{m1s}-T^{s}-U^{m1t}-H (SEQ ID NO: 6 of the Sequence Listing) (CT-330)

CT-330 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes. Moreover, the amidite reagent for the X moiety in the present polynucleotide was prepared using 4-phenylphenol (20 mg).
Nucleotide sequence: comprising a sequence complementary to nucleotide Nos. 3139-3157 of the human β-catenin gene (GenBank accession No. NM_001904.3)

### (Example 40)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-320)

CT-320 was synthesized in the same way as for Example 2. For the present polynucleotide, 4-hydroxybenzamide was condensed therein in the final step.

### (Example 41)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1p}-H (SEQ ID NO: 3 of the Sequence Listing) (CT-322)

CT-322 was synthesized in the same way as for Example 2. For the present polynucleotide, 4-(imidazol-1-yl)phenol was condensed therein in the final step.

### (Example 42)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-Tp-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-323)

CT-323 was synthesized in the same way as for Example 2. For the present polynucleotide, 4-(1,2,4-triazol-1-yl)phenol was condensed therein in the final step.

### (Example 43)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-332)

CT-332 was synthesized in the same way as for Example 2. For the present polynucleotide, 6-hydroxyquinoline was condensed therein in the final step.

### (Example 44)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-335)

CT-335 was synthesized in the same way as for Example 2. For the present polynucleotide, 3,4-dihydro-6-hydroxy-2(1H)-quinolinone was condensed therein in the final step.

### (Example 45)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-352)

CT-352 was synthesized in the same way as for Example 2. The amidite reagent for the X moiety in the present polynucleotide was prepared using 4-hydroxy-4'-methoxybiphenyl (20 mg).

### (Example 46)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-353)

CT-353 was synthesized in the same way as for Example 2. The amidite reagent for the X moiety in the present polynucleotide was prepared using 4-fluoro-4'-hydroxybiphenyl (20 mg).
Nucleotide sequence: comprising a sequence complementary to nucleotide Nos. 3139-3157 of the human β-catenin gene (GenBank accession No. NM_001904.3)

### (Example 47)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-354)

CT-354 was synthesized in the same way as for Example 2. The amidite reagent for the X moiety in the present polynucleotide was prepared using 4-benzylphenol (20 mg).

### (Example 48)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-355)

CT-355 was synthesized in the same way as for Example 2. The amidite reagent for the X moiety in the present polynucleotide was prepared using 4-α-cumylphenol (20 mg).

### (Example 49)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1p}-H (SEQ ID NO: 3 of the Sequence Listing) (CT-359)

CT-359 was synthesized in the same way as for Example 2. The amidite reagent for the X moiety in the present polynucleotide was prepared using 3-(4-hydroxyphenyl)-N-benzylpropionamide (20 mg).

### (Example 50)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-U^{m1p}-T^{p}-U^{m1t}-H (SEQ ID NO: 3 of the Sequence Listing) (CT-360)

CT-360 was synthesized in the same way as for Example 2. The amidite reagent for the X moiety in the present polynucleotide was prepared using 4-hydroxyphenyl benzoate (20 mg).

### (Example 51)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-365)

CT-365 was synthesized in the same way as for Example 6. The amidite reagent for the X moiety in the present polynucleotide was prepared using 3-fluorophenol (20 mg).
Nucleotide sequence: comprising a sequence complementary to nucleotide Nos. 3139-3157 of the human β-catenin gene (GenBank accession No. NM_001904.3)

### (Example 52)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-366)

CT-366 was synthesized in the same way as for Example 6. The amidite reagent for the X moiety in the present polynucleotide was prepared using 3-chlorophenol (20 mg).

### (Example 53)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-367)

CT-367 was synthesized in the same way as for Example 6. The amidite reagent for the X moiety in the present polynucleotide was prepared using 4-(trifluoromethyl)phenol (20 mg).

### (Example 54)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-C^{m1p}-G^{p}-C^{m1p}-T^{p}-U^{m1t}-H (SEQ ID NO: 3 of the Sequence Listing) (CT-368)

CT-368 was synthesized in the same way as for Example 6. The amidite reagent for the X moiety in the present polynucleotide was prepared using 3-acetylphenol (20 mg).

### (Example 55)

### Synthesis of X-P(=O) (OH)-O-U^{m1p}-T^{p}-G^{m1p}-T^{p}-C^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-369)

CT-369 was synthesized in the same way as for Example 6. The amidite reagent for the X moiety in the present polynucleotide was prepared using 3,4-difluorophenol (20 mg).

### (Example 56)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-370)

CT-370 was synthesized in the same way as for Example 6. The amidite reagent for the X moiety in the present polynucleotide was prepared using 3-fluoro-4-chlorophenol (20 mg).

### (Example 57)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-371)

CT-371 was synthesized in the same way as for Example 6. The amidite reagent for the X moiety in the present polynucleotide was prepared using 3-(trifluoromethyl)-4-fluorophenol (20 mg).

### (Example 58)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-373)

CT-373 was synthesized in the same way as for Example 6. The amidite reagent for the X moiety in the present polynucleotide was prepared using 3,5-dichlorophenol (20 mg).

### (Example 59)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-374)

CT-374 was synthesized in the same way as for Example 6. The amidite reagent for the X moiety in the present polynucleotide was prepared using 3-fluoro-4-acetylphenol (20 mg).

### (Example 60)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-375)

CT-375 was synthesized in the same way as for Example 6. The amidite reagent for the X moiety in the present polynucleotide was prepared using 3,4,5-trifluorophenol (20 mg).

### (Example 61)

### Synthesis of X-P(=O) (OH)-O-U^{m1p}-U^{rp}-G^{m1p}-U^{rp}-G^{m1p}-A^{rp}-U^{m1p}-C^{rp}-C^{m1p}-A^{rp}-U^{m1p}-U^{rp}-C^{m1p}-U^{rp}-U^{m1p}-G^{rp}-U^{m1p}-G^{rp}-C^{m1p}-T^{p}-T^{t}-H (SEQ ID NO: 8 of the Sequence Listing) (CT-377)

CT-377 was synthesized in the same way as for Example 37. The amidite reagent for the X moiety in the present polynucleotide was prepared using 4-(trifluoromethoxy)phenol (20 mg).

### (Example 62)

### Synthesis of HO-G^{rp}-G^{rp}-A^{rp}-C^{rp}-A^{rp}-A^{rp}-G^{rp}-G^{rp}-A^{rp}-A^{rp}-G^{rp}-C^{rp}-U^{rp}-G^{rp}-C^{rp}-A^{rp}-G^{rp}-A^{rp}-A^{rp}-T^{p}-T^{t}-H (SEQ ID NO: 13 of the Sequence Listing) (CT-165)

CT-165 was synthesized in the same way as for Example 37.
Nucleotide sequence: comprising a sequence of nucleotide Nos. 2137-2155 of the human β-catenin gene (GenBank accession No. NM_001904.3)

### (Example 63)

### Synthesis of HO-G^{p}-G^{m1p}-A^{p}-C^{m1p}-A^{p}-A^{m1p}-G^{p}-G^{m1p}-A^{p}-A^{m1p}-G^{p}-C^{m1p}-T^{p}-G^{m1p}-C^{p}-A^{m1p}-G^{p}-A^{m1t}-H (SEQ ID NO: 14 of the Sequence Listing) (CT-278)

CT-278 was synthesized in the same way as for Example 1.
Nucleotide sequence: comprising a sequence of nucleotide Nos. 2137-2154 of the human β-catenin gene (GenBank accession No. NM_001904.3)

### (Example 64)

### Synthesis of X-P(=O) (OH)-O-U^{rp}-U^{rp}-C^{rp}-U^{rp}-G^{rp}-C^{rp}-A^{rp}-G^{rp}-C^{rp}-U^{rp}-U^{rp}-C^{rp}-C^{rp}-U^{rp}-U^{rp}-G^{rp}-U^{rp}-C^{rp}-C^{rp}-T^{p}-T^{t}-H (SEQ ID NO: 15 of the Sequence Listing) (CT-378)

CT-378 was synthesized in the same way as for Example 37. The amidite reagent for the X moiety in the present polynucleotide was prepared using 4-(trifluoromethoxy)phenol (20 mg).

Nucleotide sequence: comprising a sequence complementary to nucleotide Nos. 2137-2155 of the human β-catenin gene (GenBank accession No. NM_001904.3)

### (Example 65)

### Synthesis of X-P(=O) (OH)-O-T^{p}-T^{p}-C^{m1p}-T^{p}-G^{m1p}-C^{p}-A^{m1p}-G^{p}-C^{m1p}-T^{p}-U^{m1p}-C^{p}-C^{m1p}-T^{p}-U^{m1p}-G^{p}-U^{m1p}-C^{p}-C^{m1p}-T^{p}-U^{m1t}-H (SEQ ID NO: 16 of the Sequence Listing) (CT-379)

CT-379 was synthesized in the same way as for Example 6. The amidite reagent for the X moiety in the present polynucleotide was prepared using 4-(trifluoromethoxy)phenol (20 mg).

### (Example 66)

### Synthesis of HO-C^{rp}-C^{rp}-G^{rp}-C^{rp}-C^{rp}-G^{rp}-A^{rp}-A^{rp}-U^{rp}-U^{rp}-C^{rp}-A^{rp}-U^{rp}-U^{rp}-A^{rp}-A^{rp}-U^{rp}-U^{rp}-U^{rp}-T^{p}-T^{t}-H (SEQ ID NO: 17 of the Sequence Listing) (MC-006)

MC-006 was synthesized in the same way as for Example 37.

### Nucleotide sequence: comprising a sequence of nucleotide Nos. 1544-1562 of the myeloid cell leukemia sequence 1 (MCL1) gene (GenBank accession No. NM_021960)

### (Example 67)

### Synthesis of HO-C^{p}-C^{m1p}-G^{p}-C^{m1p}-C^{p}-G^{m1p}-A^{p}-A^{m1p}-T^{p}-U^{m1p}-C^{p}-A^{m1p}-T^{p}-U^{m1p}-A^{p}-A^{m1p}-T^{p}-U^{m1t}-H (SEQ ID NO: 18 of the Sequence Listing) (MC-008)

MC-008 was synthesized in the same way as for Example 1.
Nucleotide sequence: comprising a sequence of nucleotide Nos. 1544-1561 of the MCL1 gene (GenBank accession No. NM_021960)

### (Example 68)

### Synthesis of X-P(=O) (OH) -O-A^{rp}-A^{rp}-A^{rp}-U^{rp}-U^{rp}-A^{rp}-A^{rp}-U^{rp}-G^{rp}-A^{rp}-A^{rp}-U^{rp}-U^{rp}-C^{rp}-G^{rp}-G^{rp}-C^{rp}-G^{rp}-G^{rp}-G^{p}-T^{t}-H (SEQ ID NO: 19 of the Sequence Listing) (MC-015)

MC-015 was synthesized in the same way as for Example 37. The amidite reagent for the X moiety in the present polynucleotide was prepared using 4-(trifluoromethoxy)phenol (20 mg).
Nucleotide sequence: comprising a sequence complementary to nucleotide Nos. 1544-1562 of the MCL1 gene (GenBank accession No. NM_021960)

### (Example 69)

### Synthesis of X-P(=O) (OH) -O-A^{m1p}-A^{p}-A^{m1p}-T^{p}-U^{m1p}-A^{p}-A^{m1p}-T^{p}-G^{m1p}-A^{p}-A^{m1p}-T^{p}-U^{m1p}-C^{p}-G^{m1p}-G^{p}-C^{m1p}-G^{p}-G^{m1p}-G^{p}-U^{m1t}-H (SEQ ID NO: 20 of the Sequence Listing) (MC-016)

MC-016 was synthesized in the same way as for Example 6. The amidite reagent for the X moiety in the present polynucleotide was prepared using 4-(trifluoromethoxy)phenol (20 mg).

### (Example 70)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 21 of the Sequence Listing) (CT-380)

CT-380 was synthesized in the same way as for Example 6. The amidite reagent for the X moiety in the present polynucleotide was prepared using 4-n-octylphenol (20 mg).
Nucleotide sequence: comprising a sequence complementary to nucleotide Nos. 3139-3157 of the human β-catenin gene (GenBank accession No. NM_001904.3)

### (Example 71)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 21 of the Sequence Listing) (CT-381)

CT-381 was synthesized in the same way as for Example 6. The amidite reagent for the X moiety in the present polynucleotide was prepared using 4-n-octyloxyphenol (20 mg).

### (Example 72)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 21 of the Sequence Listing) (CT-382)

CT-382 was synthesized in the same way as for Example 6. The amidite reagent for the X moiety in the present polynucleotide was prepared using 4'-hydroxyoctaphenone (20 mg).

### (Example 73)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 21 of the Sequence Listing) (CT-383)

CT-383 was synthesized in the same way as for Example 6. The amidite reagent for the X moiety in the present polynucleotide was prepared using 4'-hydroxyvalerophenone (20 mg).

### (Example 74)

### Synthesis of HO-G^{s}-C^{e2s}-A^{s}-C^{m1s}-A^{s}-A^{m1s}-G^{s}-A^{m1s}-A^{s}-A^{m1s}-G^{s}-G^{m1s}-A^{s}-U^{m1s}-C^{s}-A^{m1s}-C^{s}-A^{m1t}-H (SEQ ID NO: 22 of the Sequence Listing) (CT-342)

CT-342 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.
Nucleotide sequence: comprising a sequence of nucleotide Nos. 3139-3156 of the human β-catenin gene (GenBank accession No. NM_001904.3)

### (Example 75)

### Synthesis Of HO-G^{s}-C^{e2s}-A^{s}-C^{e2s}-A^{s}-A^{m1s}-G^{s}-A^{m1s}-A^{s}-U^{m1s}-G^{s}-G^{m1s}-A^{s}-U^{m1s}-C^{s}-A^{m1s}-C^{s}-A^{m1t}-H (SEQ ID NO: 23 of the Sequence Listing) (CT-343)

CT-343 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Example 76)

### Synthesis of HO-G^{s}-C^{e2s}-A^{s}-C^{e2s}-A^{s}-A^{e2s}-G^{s}-A^{m1s}-A^{s}-U^{m1s}-G^{s}-G^{m1s}-A^{s}-U^{m1s}-C^{s}-A^{m1s}-C^{s}-A^{m1t}-H (SEQ ID NO: 24 of the Sequence Listing) (CT-344)

CT-344 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Example 77)

### Synthesis of HO-G^{s}-C^{e2s}-A^{s}-C^{e2s}-A^{s}-A^{e2s}-G^{s}-A^{e2s}-A^{s}-U^{m1s}-G^{s}-G^{m1s}-A^{s}-U^{m1s}-C^{s}-A^{m1s}-C^{s}-A^{m1t}-H (SEQ ID NO: 25 of the Sequence Listing) (CT-345)

CT-345 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Example 78)

### Synthesis of HO-G^{s}-C^{e2s}-A^{s}-C^{e2s}-A^{s}-A^{e2s}-G^{s}-A^{e2s}-A^{s}-U^{e2s}-G^{s}-G^{m1s}-A^{s}-U^{m1s}-C^{s}-A^{m1s}-C^{s}-A^{m1t}-H (SEQ ID NO: 26 of the Sequence Listing) (CT-346)

CT-346 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Example 79)

### Synthesis of HO-G^{s}-C^{e2s}-A^{s}-C^{e2s}-A^{s}-A^{e2s}-G^{s}-A^{e2s}-A^{s}-U^{e2s}-G^{s}-G^{e2s}-A^{s}-U^{m1s}-C^{s}-A^{m1s}-C^{s}-A^{m1t}-H (SEQ ID NO: 27 of the Sequence Listing) (CT-347)

CT-347 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Example 80)

### Synthesis of HO-G^{s}-C^{e2s}-A^{s}-C^{e2s}-A^{s}-A^{e2s}-G^{s}-A^{e2s}-A^{s}-U^{e2s}-G^{s}-G^{e2s}-A^{s}-U^{e2s}-C^{s}-A^{m1s}-C^{s}-A^{m1t}-H (SEQ ID NO: 28 of the Sequence Listing) (CT-348)

CT-348 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Example 81)

### Synthesis of HO-G^{s}-C^{e2s}-A^{s}-C^{e2s}-A^{s}-A^{e2s}-G^{s}-A^{e2s}-A^{s}-U^{e2s}-G^{s}-G^{e2s}-A^{s}-U^{e2s}-C^{s}-A^{e2s}-C^{s}-A^{m1t}-H (SEQ ID NO: 29 of the Sequence Listing) (CT-349)

CT-349 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Example 82)

### Synthesis of HO-G^{s}-C^{e2s}-A^{s}-C^{e2s}-A^{s}-A^{e2s}-G^{s}-A^{e2s}-A^{s}-U^{e2s}-G^{s}-G^{e2s}-A^{s}-U^{e2s}-C^{s}-A^{e2s}-C^{s}-A^{e2t}-H (SEQ ID NO: 30 of the Sequence Listing) (CT-350)

CT-350 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Example 83)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-387)

CT-387 was synthesized in the same way as for Example 6. The amidite reagent for the X moiety in the present polynucleotide was prepared using 4'-hydroxyacetanilide (20 mg).
Nucleotide sequence: comprising a sequence complementary to nucleotide Nos. 3139-3157 of the human β-catenin gene (GenBank accession No. NM_001904.3)

### (Example 84)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-388)

CT-388 was synthesized in the same way as for Example 6. The amidite reagent for the X moiety in the present polynucleotide was prepared using methyl 4-hydroxybenzoate (20 mg).

### (Example 85)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-389)

CT-389 was synthesized in the same way as for Example 6. The amidite reagent for the X moiety in the present polynucleotide was prepared using phenol (20 mg).

### (Example 86)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-390)

CT-390 was synthesized in the same way as for Example 6. The amidite reagent for the X moiety in the present polynucleotide was prepared using resorcinol monoacetate (20 mg).

### (Example 87)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-391)

CT-391 was synthesized in the same way as for Example 6. The amidite reagent for the X moiety in the present polynucleotide was prepared using 3-bromophenol (20 mg).

### (Example 88)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-392)

CT-392 was synthesized in the same way as for Example 6. The amidite reagent for the X moiety in the present polynucleotide was prepared using 3-iodophenol (20 mg).

### (Example 89)

### Synthesis of X-P(=O) (OH)-O-C^{m1p}-T^{p}-C^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-438)

CT-438 was synthesized in the same way as for Example 6. However, acid treatment was performed in the final step. The amidite reagent for the X moiety in the present polynucleotide was prepared using a compound of Reference Example 22 (20 mg).

### (Example 90)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-439)

CT-439 was synthesized in the same way as for Example 6. The amidite reagent for the X moiety in the present polynucleotide was prepared using 3-hydroxypyridine (20 mg).

### (Example 91)

### Synthesis of X-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-457)

CT-457 was synthesized in the same way as for Example 6. The amidite reagent for the X moiety in the present polynucleotide was prepared using a compound of Reference Example 34 (20 mg).

### (Example 92)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-458)

CT-458 was synthesized in the same way as for Example 6. The amidite reagent for the X moiety in the present polynucleotide was prepared using methyl 5-hydroxy-3-pyridinecarboxylate (20 mg).

**[Table 1]**

| Example | Name | X | Molecular weight | Example | Name | X | Molecular weight |
|---|---|---|---|---|---|---|---|
| 1 | CT-169 | - | 5767.78 | 11 | CT-273 | 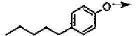 | 6536.57 |
| 2 | CT-259 | 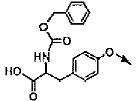 | 6686.22 | 12 | CT-274 | 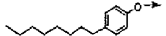 | 6578.22 |
| 3 | CT-260 | 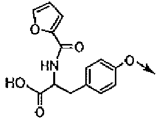 | 6646.36 | 13 | CT-275 | 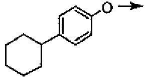 | 6548.45 |
| 4 | CT-261 | 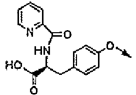 | - | 14 | CT-276 | 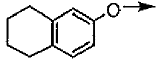 | 6520.14 |
| 5 | CT-263 | 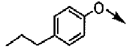 | 6508.32 | 15 | CT-277 | 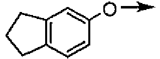 | 6506.14 |
| 6 | CT-268 | 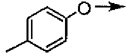 | 6480.39 | 16 | CT-290 | 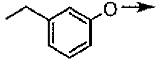 | 6494.18 |
| 7 | CT-269 | 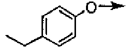 | 6494.25 | 17 | CT-292 | 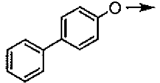 | 6542.27 |
| 8 | CT-270 | 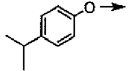 | 6508.40 | 18 | CT-293 | 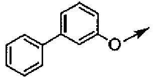 | 6542.10 |
| 9 | CT-271 | 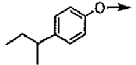 | 6522.60 | 19 | CT-295 | 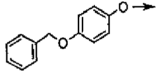 | 6572.35 |
| 10 | CT-272 | 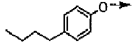 | 6522.48 | 20 | CT-296 | 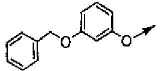 | 6572.50 |

**[Table 1-2]**

| Example | Name | X | Molecular weight | Example | Name | X | Molecular weight |
|---|---|---|---|---|---|---|---|
| 21 | CT-299 | 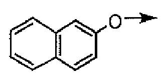 | 6516.25 | 31 | CT-321 | 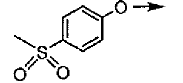 | 6864.62 |
| 22 | CT-300 | 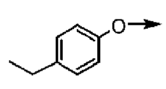 | 6814.60 | 32 | CT-322 | 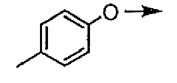 | 6800.62 |
| 23 | CT-312 | 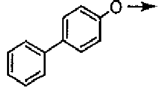 | 6862.48 | 33 | CT-331 | 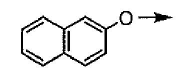 | 6836.50 |
| 24 | CT-313 | 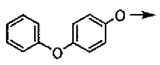 | 6878.34 | 34 | CT-332 | 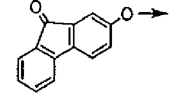 | 6888.54 |
| 25 | CT-314 | 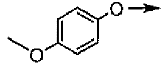 | 6816.30 | 35 | CT-333 | 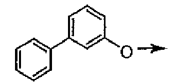 | 6862.46 |
| 26 | CT-315 | 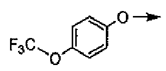 | 6870.27 | 36 | CT-334 | 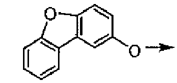 | 6876.71 |
| 27 | CT-316 | 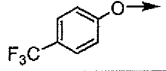 | 6854.38 | 37 | CT-335 | 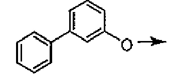 | 6798.21 |
| 28 | CT-317 | 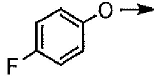 | 6804.57 | 38 | CT-336 | - | 6041.42 |
| 29 | CT-318 | 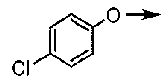 | 6820.67 | 39 | CT-337 | 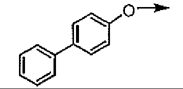 | 7199.76 |
| 30 | CT-319 | 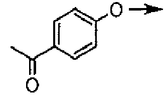 | 6828.41 | 40 | CT-338 | 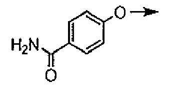 | 6830.28 |

**[Table 1-3]**

| Example | Name | X | Molecular weight | Example | Name | X | Molecular weight |
|---|---|---|---|---|---|---|---|
| 41 | CT-322 | 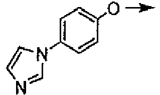 | 6852.06 | 49 | CT-359 | 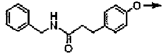 | 6947.43 |
| 42 | CT-323 | 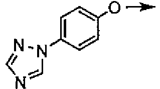 | 6852.86 | 50 | CT-360 | 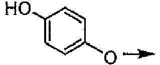 | 6802.43 |
| 43 | CT-332 | 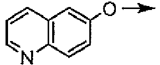 | 6837.80 | | | | |
| 44 | CT-335 | 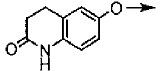 | 6855.21 | | | | |
| 45 | CT-352 | 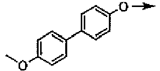 | 6892.69 | | | | |
| 46 | CT-353 | 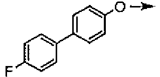 | 6880.47 | | | | |
| 47 | CT-354 | 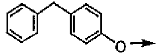 | 6876.41 | | | | |
| 48 | CT-355 | 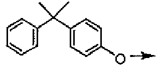 | 6904.51 | | | | |

**[Table 1-4]**

| Example | Name | X | Molecular weight | Example | Name | X | Molecular weight |
|---|---|---|---|---|---|---|---|
| 51 | CT-365 | 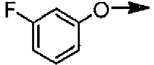 | 6804.78 | 61 | CT-377 | 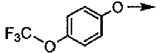 | 6942.66 |
| 52 | CT-366 | 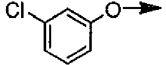 | 6820.88 | 62 | CT-165 | - | 6818.27 |
| 53 | CT-367 | 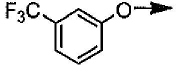 | 6854.30 | 63 | CT-278 | - | 5877.25 |
| 54 | CT-368 | 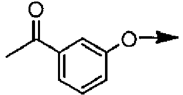 | 6828.52 | 64 | CT-378 | 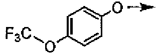 | 6737.27 |
| 55 | CT-369 | 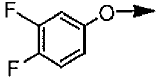 | 6822.37 | 65 | CT-379 | 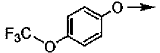 | 6789.62 |
| 56 | CT-370 | 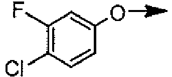 | 6838.78 | 66 | MC-006 | - | 6552.08 |
| 57 | CT-371 | 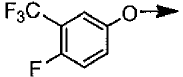 | 6873.38 | 67 | MC-008 | - | 5661.94 |
| 58 | CT-373 | 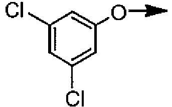 | 6855.23 | 68 | MC-015 | 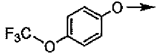 | 6983.45 |
| 59 | CT-374 | 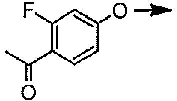 | 6846.55 | 69 | MC-016 | 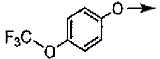 | 7038.02 |
| 60 | CT-375 | 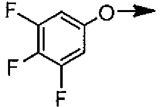 | 6840.79 | 70 | CT-380 | 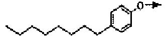 | 6898.83 |

**[Table 1-5]**

| Example | Name | X | Molecular weight | Example | Name | X | Molecular weight |
|---|---|---|---|---|---|---|---|
| 71 | CT-381 | 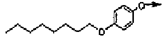 | 6914.79 | 77 | CT-345 | - | 6117.58 |
| 72 | CT-382 | 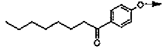 | 6912.84 | 78 | CT-346 | - | 6142.97 |
| 73 | CT-383 | 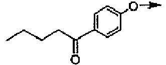 | 6870.83 | 79 | CT-347 | - | 6154.61 |
| 74 | CT-342 | - | 6066.42 | 80 | CT-348 | - | 6180.77 |
| 75 | CT-343 | - | 6092.43 | 81 | CT-349 | - | 6192.51 |
| 76 | CT-344 | - | 6103.83 | 82 | CT-350 | - | 6204.65 |

**[Table 1-6]**

| Example | Name | X | Molecular weight | Example | Name | X | Molecular weight |
|---|---|---|---|---|---|---|---|
| 83 | CT-387 | 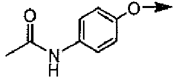 | 6843.55 | 88 | CT-392 | 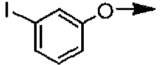 | 6912.37 |
| 84 | CT-388 | 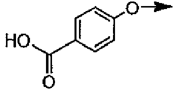 | 6829.69 | 89 | CT-438 | 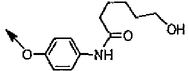 | 6915.12 |
| 85 | CT-389 | 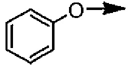 | 6786.79 | 90 | CT-439 | 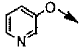 | 6786.43 |
| 86 | CT-390 | 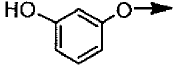 | 6802.66 | 91 | CT-457 | 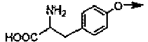 | 6871.33 |
| 87 | CT-391 | 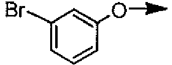 | 6865.67 | 92 | CT-458 | 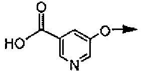 | 6829.58 |

Likewise, the structural formula of X in each polynucleotide synthesized in the Reference Examples and the molecular weight measured value of each polynucleotide, measured with a mass spectrometer, are shown in Table 2.

### (Reference Example 1)

### Synthesis of HO-G^{rp}-C^{m1p}-A^{rp}-C^{m1p}-A^{rp}-A^{m1p}-G^{rp}-A^{m1p}-A^{rp}-U^{m1p}-G^{rp}-G^{m1p}-A^{rp}-U^{m1p}-C^{rp}-A^{m1p}-C^{rp}-A^{m1p}-A^{rp}-T^{p}-T^{t}-H (SEQ ID NO: 7 of the Sequence Listing) (CT-001)

CT-001 was synthesized in the same way as for Example 1. For the present polynucleotide, the protected polynucleotide analog having the sequence of interest was treated with 2 mL of an ammonia water:ethanol solution (3:1 v/v) at 55°C for 16 hours to cleave the oligomer from the support and to remove the cyanoethyl group acting as a protective group for the phosphate group and the protective group on the base of the nucleic acid. CPG was removed by filtration. After washing with ethanol, the filtrate and the wash were combined, and the solvent was distilled off under reduced pressure. To the residue, 0.3 mL of triethylamine trihydrofluoride was added, and the mixture was left at room temperature for 19 hours. 60 µL of H₂O and 3 mL of n-butanol were added thereto, and the mixture was left at -20°C for 1 hour. Then, precipitates were collected by centrifugation. The obtained precipitates were dissolved in 200 µL of H₂O and purified by 20% polyacrylamide gel electrophoresis containing 7 M urea (1x TBE, 600 V, 4 hours). After the electrophoresis, bands were visualized using a UV lamp, and the bands of interest were excised using a knife. 1 mL of a solution containing 0.2 M NaCl and 10 mM EDTA (pH 7.2) was added thereto, and the mixture was left overnight to elute the polynucleotide from the gel slice. The oligonucleotide was precipitated by the addition of ethanol and collected by centrifugation.
Nucleotide sequence: comprising a sequence of nucleotide Nos. 3139-3157 of the human β-catenin gene (GenBank accession No. NM_001904.3)

### (Reference Example 2)

### Synthesis of HO-U^{m1p}-U^{rp}-G^{m1p}-U^{rp}-G^{m1p}-A^{rp}-U^{m1p}-C^{rp}-C^{m1p}-A^{rp}-U^{m1p}-U^{rp}-C^{m1p}-U^{rp}-U^{m1p}-G^{rp}-U^{m1p}-G^{rp}-C^{m1p}-T^{p}-T^{t}-H (SEQ ID NO: 8 of the Sequence Listing) (CT-005)

CT-005 was synthesized in the same way as for Reference Example 1.
Nucleotide sequence: comprising a sequence complementary to nucleotide Nos. 3139-3157 of the human β-catenin gene
(GenBank accession No. NM_001904.3)

### (Reference Example 3)

### Synthesis of HO-G^{rp}-C^{rp}-A^{rp}-C^{rp}-A^{rp}-A^{rp}-G^{rp}-A^{rp}-A^{rp}-U^{rp}-G^{rp}-G^{rp}-A^{rp}-U^{rp}-C^{rp}-A^{rp}-C^{rp}-A^{rp}-A^{rp}-U^{rp}-U^{rt}-H (SEQ ID NO: 9 of the Sequence Listing) (CT-106)

CT-106 was synthesized in the same way as for Reference Example 1.
Nucleotide sequence: comprising a sequence of nucleotide Nos. 3139-3157 of the human β-catenin gene (GenBank accession No. NM_001904.3)

### (Reference Example 4)

### Synthesis of HO-U^{rp}-U^{rp}-G^{rp}-U^{rp}-G^{rp}-A^{rp}-U^{rp}-C^{rp}-C^{rp}-A^{rp}-U^{rp}-U^{rp}-C^{rp}-U^{rp}-U^{rp}-G^{rp}-U^{rp}-G^{rp}-C^{rp}-U^{rp}-U^{rt}-H (SEQ ID NO: 10 of the Sequence Listing) (CT-041)

CT-041 was synthesized in the same way as for Reference Example 1.
Nucleotide sequence: comprising a sequence complementary to nucleotide Nos. 3139-3157 of the human β-catenin gene (GenBank accession No. NM_001904.3)

### (Reference Example 5)

### Synthesis of HO-P(=O) (OH) -O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 11 of the Sequence Listing) (CT-157)

CT-157 was synthesized in the same way as for Example 1.
Nucleotide sequence: comprising a sequence complementary to nucleotide Nos. 3139-3157 of the human β-catenin gene (GenBank accession No. NM_001904.3)

### (Reference Example 6)

### Synthesis of X-P(=O) (OH) -O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-225)

CT-225 was synthesized in the same way as for Example 2. For the present polynucleotide, methyl 3-(4-hydroxyphenyl)propionate was condensed therein in the final step.

### (Reference Example 7)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-291)

CT-291 was synthesized in the same way as for Example 6. The amidite reagent for the X moiety in the present polynucleotide was prepared using 2-ethylphenol (20 mg).

### (Reference Example 8)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-294)

CT-294 was synthesized in the same way as for Example 6. The amidite reagent for the X moiety in the present polynucleotide was prepared using 2-phenylphenol (20 mg).

### (Reference Example 9)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-297)

CT-297 was synthesized in the same way as for Example 6. The amidite reagent for the X moiety in the present polynucleotide was prepared using 2-(benzyloxy)phenol (20 mg).

### (Reference Example 10)

### Synthesis of X-P(=O) (OH)-O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 2 of the Sequence Listing) (CT-298)

CT-298 was synthesized in the same way as for Example 6. The amidite reagent for the X moiety in the present polynucleotide was prepared using 1-naphthol (20 mg).

### (Reference Example 11)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-186)

CT-186 was synthesized in the same way as for Example 6. For the present polynucleotide, the X moiety was prepared using a compound of Example 8a in Japanese Patent Laid-Open No. 11-246592.

### (Reference Example 12)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-327)

CT-327 was synthesized in the same way as for Example 6. The amidite reagent for the X moiety in the present polynucleotide was prepared using 4-methylphenenyl alcohol (20 mg).

### (Reference Example 13)

### Synthesis of HO-P(=O) (OH)-O-U^{m1p}-T^{s}-G^{m1s}-T^{s}-G^{m1s}-A^{s}-U^{m1s}-C^{s}-C^{m1s}-A^{s}-U^{m1s}-T^{s}-C^{m1s}-T^{s}-U^{m1s}-G^{s}-U^{m1s}-G^{s}-C^{m1s}-T^{s}-U^{m1t}-H (SEQ ID NO: 6 of the Sequence Listing) (CT-311)

CT-311 was synthesized in the same way as for Example 1. In the present polynucleotide, the phosphorothioate bond moiety was prepared by treating with a 0.2 M phenylacetyl disulfide/pyridine-acetonitrile (1:1 v/v) solution for 3 minutes.

### (Reference Example 14)

### Synthesis of HO-P(=O) (OH) -O-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 12 of the Sequence Listing) (CT-203)

CT-203 was synthesized in the same way as for Example 1.
Nucleotide sequence: comprising a sequence complementary to nucleotide Nos. 3139-3156 of the human β-catenin gene
(GenBank accession No. NM_001904.3)

### (Reference Example 15)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-351)

CT-351 was synthesized in the same way as for Example 2. The amidite reagent for the X moiety in the present polynucleotide was prepared using 3-pentadecylphenol (20 mg).

### (Reference Example 16)

### Synthesis of X-P(=O) (OH)-O-U^{m1p}-T^{p}-G^{m1p}-T^{p}-G^{m1p}-A^{p}-U^{m1p}-C^{p}-C^{m1p}-A^{p}-U^{m1p}-T^{p}-C^{m1p}-T^{p}-U^{m1p}-G^{p}-U^{m1p}-G^{p}-C^{m1p}-T^{p}-U^{m1t}-H (SEQ ID NO: 3 of the Sequence Listing) (CT-326)

CT-326 was synthesized in the same way as for Example 2. The amidite reagent for the X moiety in the present polynucleotide was prepared using 4-methylbenzyl alcohol.
Nucleotide sequence: comprising a sequence complementary to nucleotide Nos. 3139-3157 of the human β-catenin gene (GenBank accession No. NM_001904.3)

### (Reference Example 17)

### Synthesis of HO-U^{rp}-U^{rp}-C^{rp}-U^{rp}-G^{rp}-C^{rp}-A^{rp}-G^{rp}-C^{rp}-U^{rp}-U^{rp}-C^{rp}-C^{rp}-U^{rp}-U^{rP}-G^{rp}-U^{rp}-C^{rp}-C^{rp}-T^{P}-T^{t}-H (SEQ ID NO: 31 of the Sequence Listing) (CT-166)

CT-166 was synthesized in the same way as for Example 37.
Nucleotide sequence: comprising a sequence complementary to nucleotide Nos. 2137-2155 of the human β-catenin gene
(GenBank accession No. NM_001904.3)

### (Reference Example 18)

### Synthesis of HO-P(=O) (OH)-O-T^{p}-T^{p}-C^{m1p}-T^{p}-G^{m1p}-C^{p}-A^{m1p}-G^{p}-C^{m1p}-T^{p}-U^{m1p}-C^{p}-C^{m1p}-T^{p}-U^{m1p}-G^{p}-U^{m1p}-C^{p}-C^{m1p}-T^{p}-U^{m1t}-H (SEQ ID NO: 32 of the Sequence Listing) (CT-280)

CT-280 was synthesized in the same way as for Example 1.
Molecular weight calculated value: 6629.26, measured value: 6629.51

### (Reference Example 19)

### Synthesis of HO-A^{rp}-A^{rp}-A^{rp}-U-^{rP}-U-T^{p}-A^{rp}-A^{rp}-U^{rp}-G^{rp}-A^{rp}-A^{rp}-U^{rp}-U^{rp}-C^{rp}-G^{rp}-G^{rp}-C^{rp}₋G^{rp}-G^{rp}-G^{p}-T^{t}-H (SEQ ID NO: 33 of the Sequence Listing) (MC-007)

MC-007 was synthesized in the same way as for Example 37.

### Nucleotide sequence: comprising a sequence complementary to nucleotide Nos. 1544-1562 of the MCL1 gene (GenBank accession No. NM_021960)

### (Reference Example 20)

### Synthesis of HO-P(=O) (OH) -O-A^{m1p}-A^{p}-A^{m1p}-T^{p}-U^{m1p}-A^{p}-A^{m1p}-T^{p}-G^{m1p}-A^{p}-A^{m1p}-T^{p}-U^{m1p}-C^{p}-G^{m1p}-G^{p}-C^{m1p}-G^{p}-G^{m1p}-G^{p}-U^{m1t}-H (SEQ ID NO: 34 of the Sequence Listing) (MC-009)

MC-009 was synthesized in the same way as for Example 1.

### (Reference Example 22)

6-(4,4'-dimethoxytrityloxy)hexanoic acid (722 mg, 1.67 mmol; J. Org. Chem., 1995, 60, 3358-3364) was dissolved in 2 mL of methylene chloride. To the solution, 4-aminophenol (200 mg, 1.84 mmol), EDC (288 mg, 2.5 mmol), HOBT (225 mg, 2.5 mmol), and triethylamine (260 µL) were added, and the mixture was stirred overnight. The completion of the reaction was confirmed by TLC. Then, the reaction solution was separated into organic and aqueous phases using methylene chloride and a 5% aqueous sodium bicarbonate solution, and the organic phase was washed with saturated saline. The organic phase was dried over sodium sulfate, and the solvent was then concentrated under reduced pressure. The residue was purified with a silica gel column (30 g, 2% methanol/methylene chloride) to obtain the amorphous compound of Reference Example 22 (649 mg).
¹H-NMR (400MHz, CDCl₃) 7.43-6.75 (17H, m), 3.78 (6H, s), 3.05 (2H, m), 2.30 (2H, m), 1.73-1.59 (4H, m), 1.49-1.38 (2H, m)
FAB-MAS(mNBA): 525 M⁺

### (Reference Example 23)

8-hydroxyoctanoic acid (100 mg, 0.59 mmol) was dissolved in 1.5 mL of pyridine. To the solution, 4,4'-dimethoxytrityl chloride (237 mg, 0.7 mmol) was added, and the mixture was stirred overnight. The completion of the reaction was confirmed by TLC. Then, the reaction solution was separated into organic and aqueous phases using methylene chloride and water. The organic phase was dried over sodium sulfate, and the solvent was then concentrated under reduced pressure. The residue was purified with a silica gel column (4 g, methylene chloride) to obtain amorphous 8-(4,4'-dimethoxytrityloxy)octanoic acid (348 mg). The obtained 8-(4,4'-dimethoxytrityloxy)octanoic acid was dissolved in 1 mL of methylene chloride. To the solution, 4-aminophenol (70.9 mg, 0.64 mmol), EDC (101.6 mg, 0.88 mmol), HOBT (79 mg, 0.886 mmol), and triethylamine (92 µL) were added, and the mixture was stirred overnight. The completion of the reaction was confirmed by TLC. Then, the reaction solution was purified with a silica gel column (5 g, 30%→50% ethyl acetate/n-hexane) to obtain the amorphous compound of Reference Example 23 (148 mg).
¹H-NMR (400MHz, CDCl₃) 7.52-6.59 (17H, m), 3.79 (6H, s), 3.05-3.01(2H, m),2.28(2H, m), 1.71-1.58(4H, m), 1.35-1.24(6H, m)
FAB-MAS(mNBA+KI): 592 (M+K)⁺

### (Reference Example 24)

10-(4,4'-dimethoxytrityloxy)decanoic acid (0.707 g, 1.19 mmol; Tetrahedron Letters, 1994, 35, 2353-2356) was dissolved in 2 mL of methylene chloride. To the solution, 4-aminophenol (141.8 mg, 1.28 mmol), EDC (203 mg, 1.76 mmol), HOBT (158 mg, 1.76 mmol), and triethylamine (183 µL) were added, and the mixture was stirred overnight. The completion of the reaction was confirmed by TLC. Then, the reaction solution was purified with a silica gel column (7.5 g, 30%→50% ethyl acetate/n-hexane) to obtain the amorphous compound of Reference Example 24 (485 mg).
¹H-NMR (400MHz, CDCl₃) 7.52-6.59 (17H, m), 3.78(6H, s), 3.02(2H, m),2.31(2H, m), 1.74-1.56(4H, m), 1.33-1.24(10H, m)
FAB-MAS(mNBA): 580 (M-H)⁺

### (Reference Example 25)

A solution of EDC (383 mg, 2 mmol) and HOBT (67.5 mg, 0.5 mmol) dissolved in 3 mL of methylene chloride was added to 4-amino-1-butanol (160.45 mg, 1.8 mmol) and 4-hydroxybenzoic acid (207.18 mg, 1.5 mmol). Triethylamine (260 µL) was further added thereto, and the mixture was shaken overnight. The completion of the reaction was confirmed by TLC. Then, the reaction solution was purified with a silica gel column (5 g, elution with methylene chloride→ethyl acetate) to obtain an amide compound in an oil form (0.20 g). This compound was dissolved in 1.5 mL of pyridine. To the solution, 4,4'-dimethoxytrityl chloride (500 mg, 1.5 mmol) was added, and the mixture was stirred at room temperature for 3 hours. The completion of the reaction was confirmed by TLC. Then, 0.5 mL of methanol was added thereto, and the reaction solution was separated into organic and aqueous phases using ethyl acetate and a 5% aqueous sodium bicarbonate solution. The solvent in the organic phase was concentrated under reduced pressure. The residue was purified with a silica gel column (10 g, 40%→50% ethyl acetate/n-hexane) to obtain the amorphous compound of Reference Example 25 (325 mg).
¹H-NMR (400MHz, CDCl₃) 7.70-6.78 (17H, m), 6.11 (1H, brs), 5.69 (1H, s), 3.78(6H, s), 3.42(2H, m), 3.11(2H, m), 1.69(4H, m)

### FAB-MAS(mNBA): 511 M⁺

### (Reference Example 26)

The amorphous compound of Reference Example 26 was obtained (445 mg) by synthesis in the same way as for Reference Example 25 using 6-amino-1-hexanol (210.94 mg, 1.8 mmol) and 4-hydroxybenzoic acid (207.18 mg, 1.5 mmol). ¹H-NMR (400MHz, CDCl₃) 7.67-6.79 (17H, m), 5.97 (1H, brs), 5.56 (1H, s), 3.78(6H, s), 3.43-3.38(2H, m), 3.06-3.02(2H, m), 1.63-1.55(4H, m), 1.45-1.34(4H, m)
FAB-MAS(mNBA): 539 M⁺

### (Reference Example 27)

The amorphous compound of Reference Example 27 was obtained (486 mg) by synthesis in the same way as for Reference Example 25 using 8-amino-1-octanol (261.43 mg, 1.8 mmol) and 4-hydroxybenzoic acid (207.18 mg, 1.5 mmol). ¹H-NMR (400MHz, CDCl₃) 7.68-6.80 (17H, m), 5.98 (1H, brs), 5.54 (1H, s), 3.79(6H, s), 3.44-3.39(2H, m), 3.03(2H, m), 1.62-1.55(4H, m), 1.34-1.24(8H, m)
FAB-MAS(mNBA): 567 M⁺

### (Reference Example 28)

The amorphous compound of Reference Example 28 was obtained (566 mg) by synthesis in the same way as for Reference Example 25 using 4-amino-1-butanol (160.45 mg, 1.8 mmol) and 3-hydroxybenzoic acid (207.18 mg, 1.5 mmol). ¹H-NMR (400MHz, CDCl₃) 7.52-6.79 (17H, m), 6.25 (1H, brs), 6.06 (1H, s), 3.78 (5H, s), 3.47-3.42 (2H, m), 3.13 (2H, m), 1.72-1.66 (4H, m)
FAB-MAS(mNBA): 511 M⁺

### (Reference Example 29)

The amorphous compound of Reference Example 29 was obtained (580 mg) by synthesis in the same way as for Reference Example 25 using 6-amino-1-hexanol (210.94 mg, 1.8 mmol) and 3-hydroxybenzoic acid (207.18 mg, 1.5 mmol). ¹H-NMR (400MHz, CDCl₃) 7.52-6.80 (17H, m), 6.08 (1H, brs), 6.04 (1H, s), 3.78 (6H, s), 3.45-3.40 (2H, m), 3.06-3.03 (2H, m), 1.65-1.56(4H, m), 1.45-1.34(4H, m)
FAB-MAS(mNBA): 539 M⁺

### (Reference Example 30)

The amorphous compound of Reference Example 30 was obtained (675 mg) by synthesis in the same way as for Reference Example 25 using 8-amino-1-octanol (261.43 mg, 1.8 mmol) and 3-hydroxybenzoic acid (207.18 mg, 1.5 mmol). ¹H-NMR (400MHz, CDCl₃) 7.52-6.80 (17H, m), 6.21 (1H, brs), 6.11 (1H, s), 3.78 (6H, s), 3.46-3.41(2H, m), 3.04-3.01(2H, m), 1.63-1.58 (4H, m), 1.39-1.33 (8H, m)
FAB-MAS(mNBA): 566 (M-H)⁺

### (Reference Example 31)

The amorphous compound of Reference Example 31 was obtained (540 mg) by synthesis in the same way as for Reference Example 25 using 4-amino-1-butanol (160.45 mg, 1.8 mmol) and 3-(4-hydroxyphenyl)propionic acid (249.26 mg, 1.5 mmol).
¹H-NMR (400MHz, CDCl₃) 7.52-6.68 (17H, m), 5.37 (1H, brs), 4.87 (1H, s), 3.78 (6H, s), 3.18 (2H, m), 3.04(2H, m), 2.86(2H, t, J=7.56Hz), 2.35(2H, t, J=7.56Hz), 1.54-1.48 (4H, m)
FAB-MAS(mNBA): 540 (M+H)⁺

### (Reference Example 32)

The amorphous compound of Reference Example 32 was obtained (559 mg) by synthesis in the same way as for Reference Example 25 using 6-amino-1-hexanol (210.94 mg, 1.8 mmol) and 3-(4-hydroxyphenyl)propionic acid (249.26 mg, 1.5 mmol).
¹H-NMR (400MHz, CDCl₃) 7.44-6.70 (17H, m), 5.21 (1H, brs), 5.03 (1H, s), 3.79(6H, s), 3.15(2H, m), 3.03(2H, m), 2.87 (2H, t, J=7.33Hz), 2.39 (2H, t, J=7.56Hz), 1.59-1.13(8H, m)
FAB-MAS(mNBA): 568 (M+H)⁺

### (Reference Example 33)

The compound having a chewy-candy consistency of Reference Example 33 was obtained (720 mg) by synthesis in the same way as for Reference Example 25 using 8-amino-1-octanol (261.43 mg, 1.8 mmol) and 3-(4-hydroxyphenyl)propionic acid (249.26 mg, 1.5 mmol). ¹H-NMR (400MHz, CDCl₃) 7.52-6.71 (17H, m), 5.26 (1H, brs), 5.10 (1H, s), 3.78(6H, s), 3.18 (2H, m), 3.03(2H, m), 2.88 (2H, t, J=7.56Hz), 2.41 (2H, t, J=7.56Hz), 1.62-1.17 (12H, m)
FAB-MAS(mNBA): 594 (M-H)⁺

### (Reference Example 34)

### N-(4-methoxytrityl)-L-tyrosine ethyl ester

L-tyrosine ethyl (418 mg, 2 mmol) was dissolved in 5 mL of pyridine. To the solution, 4-methoxytrityl chloride (741 mg, 2.4 mmol) was added, and the mixture was stirred at room temperature for 5 hours. The completion of the reaction was confirmed by TLC. Then, the reaction solution was separated into organic and aqueous phases using ethyl acetate and a 5% aqueous sodium bicarbonate solution. The organic phase was dried over sodium sulfate, and the organic solvent was then concentrated under reduced pressure. The residue was purified with a silica gel column (30 g, 30% ethyl acetate/n-hexane) to obtain the amorphous compound of Reference Example 34 (687 mg).
¹H-NMR (400MHz, CDCl₃) 7.42-6.72 (18H, m), 4.69 (1H, s), 3.76(3H, s), 3.53-3.33(3H, m), 2.94-2.81(2H, m), 2.58 (1H, d), 0.84 (3H, m)
FAB-MAS(mNBA): 482 (M+H)⁺

### (Reference Example 35)

### 3-(4-methoxytrityloxy)-2-acetylamino-propionic acid (Ac-Ser(MMTr)-OH)

N-acetyl-D,L-serine (1.775 g, 12 mmol) was dissolved in 20 mL of pyridine. To the solution, 4-methoxytrityl chloride (4.1 g, 13.2 mmol) was added, and the mixture was stirred overnight at room temperature. The completion of the reaction was confirmed by TLC. Then, the reaction solution was separated into organic and aqueous phases using ethyl acetate and a 5% aqueous sodium bicarbonate solution. The organic phase was dried over sodium sulfate, and the solvent was then concentrated under reduced pressure. The residue was purified with a silica gel column (120 g, 30% acetone/n-hexane) to obtain the amorphous compound of Reference Example 35 (3.93 g).
¹H-NMR (400MHz, CDCl₃) 7.41-6.81 (14H, m), 6.15 (1H, d, J=7.33Hz), 4.70-4.66 (1H, m), 3.78(3H, s), 3.75-3.744 (1H, m), 3.42-3.38 (1H, m), 2.02(3H, s)
FAB-MAS(mNBA): 419 M⁺

### (Reference Example 36)

### Ac-Ser(MMTr)-Tyr-OEt

The compound of Reference Example 35 (629 mg, 1.5 mmol Ac-Ser(MMTr)-OH) was dissolved in 3 mL of methylene chloride. To the solution, L-tyrosine ethyl (334 mg, 1.6 mmol), EDC (383 mg, 2 mmol), HOBT (67.5 mg, 0.5 mmol), and triethylamine (260 µL) were added, and the mixture was stirred for 4 hours. The reaction solution was purified with a silica gel column (15 g, 40%→50% ethyl acetate/n-hexane) to obtain the amorphous compound of Reference Example 36 (460 mg).
¹H-NMR (400MHz, CDCl₃) 7.52-6.61 (18H, m), 6.08 (1H, m), 4.87-4.77 (1H, m), 4.52 (1H, m), 4.19-4.05 (2H, m), 3.79(3H, s), 3.70-3.59 (1H, m), 3.19-2.96(3H, m), 1.93,1.91 (1H, ds), 1.28-1.22 (3H, m)
FAB-MAS(mNBA): 611 (M+H)⁺

### (Reference Example 37)

### t-Boc-βAla-Tyr-OEt

The amorphous compound of Reference Example 37 was obtained (497 mg) by synthesis in the same way as for Reference Example 36 using N-t-Boc-β-alanine (283 mg, 1.5 mmol, t-Boc-βAla-OH) and L-tyrosine ethyl (376 mg, 1.8 mmol, H-Tyr-OEt).
¹H-NMR (400MHz, CDCl₃) 6.97-6.74 (4H, m), 6.03 (1H, brs), 5.11 (1H, brs), 4.80 (1H, q, J=6.72Hz), 4.22-4.15(2H, m), 3.36 (2H, m), 3.10-3.01 (2H, m), 2.38 (2H, m), 1.41 (9H, s), 1.29-1.23 (3H, m)
FAB-MAS(mNBA): 381 (M+H)⁺

### (Reference Example 38)

### t-Boc-Ala-Tyr-OEt

The amorphous compound of Reference Example 38 was obtained (490 mg) by synthesis in the same way as for Reference Example 36 using N-t-Boc-alanine (283 mg, 1.5 mmol, t-Boc-Ala-OH) and L-tyrosine ethyl (376 mg, 1.8 mmol).
¹H-NMR (400MHz, CDCl₃) 6.98-6.71 (4H, m), 6.49 (1H, d), 5.16 (1H, s), 4.95-4.76 (1H, m), 4.20-4.13(3H, m), 3.05(2H, m), 1.41 (9H, s), 1.33,1.31 (3H, ds), 1.28-1.21 (3H, m) FAB-MAS(mNBA): 381 (M+H)⁺

### (Reference Example 39)

### t-Boc-Gly-Tyr-OEt

The amorphous compound of Reference Example 39 was obtained (434 mg) by synthesis in the same way as for Reference Example 36 using N-t-Boc-glycine (263 mg, 1.5 mmol) and L-tyrosine ethyl (376 mg, 1.8 mmol). ¹H-NMR (400MHz, CDCl₃) 6.98-6.72 (4H, m), 6.46 (1H, d), 5.06 (1H, brs), 4.82 (1H, m), 4.21-4.13(2H, m), 3.85-3.72 (2H, m), 3.05 (2H, m), 1.41 (9H, s), 1.29-1.24 (3H, m), 1.28-1.21(3H, m)
FAB-MAS(mNBA): 367 (M+H)⁺

### (Reference Example 40)

### Ac-Ser(MMTr)-βAla-Tyr-OEt

The compound of Reference Example 37 (490 mg, 1.29 mmol) was dissolved in 4 mL of methylene chloride. To the solution, 4 mL of TFA was added, and the mixture was left at room temperature for 15 minutes. Then, the solvent was concentrated under reduced pressure. The residue was dissolved in methylene chloride (3 mL) and triethylamine (260 µL). To the solution, the compound of Reference Example 35 (544 mg, 1.3 mmol), EDC (383 mg, 2 mmol), HOBT (67.5 mg, 0.5 mmol), and triethylamine (260 µL) were added, and the mixture was stirred overnight. The reaction solution was purified with a silica gel column (20 g, 80% ethyl acetate/n-hexane→ethyl acetate) to obtain the amorphous compound of Reference Example 40 (469 mg).
¹H-NMR (400MHz, CDCl₃) 7.41-6.71 (18H, m), 4.81-4.75 (1H, m), 4.54-4.44 (1H, m), 4.26-4.15(2H, m), 3.78(3H, s), 3.46-2.20(8H, m), 2.02, 1.98 (3H, ds), 1.34-1.24(3H, m) FAB-MAS(mNBA): 682 (M+H)⁺

### (Reference Example 41)

### Ac-Ser(MMTr)-Ala-Tyr-OEt

The compound of Reference Example 41 in a white solid form was obtained (448 mg) by synthesis in the same way as for Reference Example 40 using the compound of Reference Example 38 (485 mg, 1.26 mmol) and the compound of Reference Example 35 (544 mg, 1.3 mmol). ¹H-NMR (400MHz, CDCl₃) 7.41-6.49 (18H, m), 4.79 (1H, m), 4.54-4.44(2H, m), 4.20-4.15(2H, m), 3.78 (3H, s), 3.46-2.85 (4H, m), 2.01,1.94(3H, ds), 1.37-1.17 (6H, m) FAB-MAS(mNBA): 682 (M+H)⁺

### (Reference Example 42)

### Ac-Ser(MMTr)-Gly-Tyr-OEt

The compound of Reference Example 42 in a white solid form was obtained (486 mg) by synthesis in the same way as for Reference Example 40 using the compound of Reference Example 39 (430 mg, 1.17 mmol) and the compound of Reference Example 35 (544 mg, 1.3 mmol). ¹H-NMR (400MHz, DMSO-d₆) 9.22 (1H, s), 8.41-8.34 (1H, m), 8.23 (1H, m), 8.08-8.05 (1H, m), 7.38-6.63(18H, m), 4.60 (1H, m), 4.36 (1H, m), 4.04-3.97 (2H, m), 3.92-3.61 (2H, m), 3.74 (3H, s), 3.09 (1H, m), 2.83 (1H, m), 1.85 (3H, s), 1.11-1.06(3H, m)
FAB-MAS(mNBA): 668 (M+H)⁺

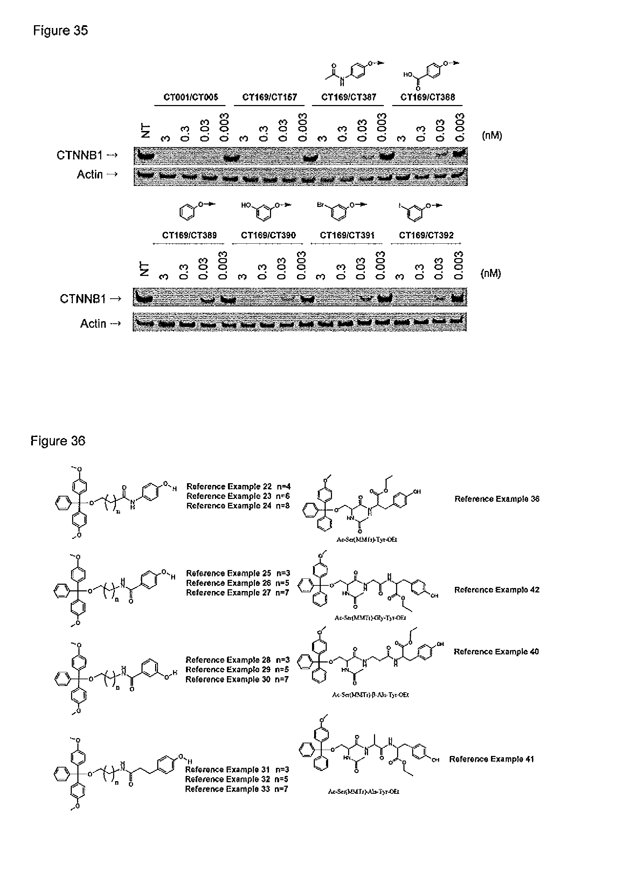

The structures of the compounds described in Reference Examples 22 to 33, 36, and 40 to 42 are shown in Figure 36.

**[Table 2]**

| Reference Example | Name | X | Molecular weight | Reference Example | Name | X | Molecular weight |
|---|---|---|---|---|---|---|---|
| 1 | CT-001 | - | 6850.8 | 11 | CT-186 | 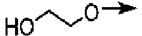 | 6754.18 |
| 2 | CT-005 | - | 6702.2 | 12 | CT-327 | 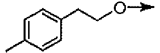 | 6828.55 |
| 3 | CT-106 | - | 6726.73 | 13 | CT-311 | - | 7031.31 |
| 4 | CT-041 | - | 6565.34 | 14 | CT-203 | - | 6390.72 |
| 5 | CT-157 | - | 6710.39 | 15 | CT-351 | 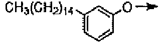 | 6996.54 |
| 6 | CT-225 | 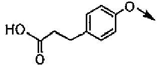 | 6537.23 | 16 | CT-326 | 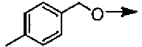 | 6814.24 |
| 7 | CT-291 | 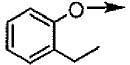 | 6494.28 | 17 | CT-166 | - | 6496.99 |
| 8 | CT-294 | 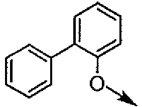 | 6542.19 | 18 | CT-280 | - | 6629.51 |
| 9 | CT-297 | 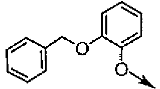 | 6572.22 | 19 | MC-007 | - | 6743.19 |
| 10 | CT-298 | 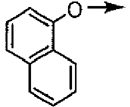 | 6516.29 | 20 | MC-009 | - | 6877.70 |

### (Example 93) Annealing for formation of double-stranded polynucleotide

The polynucleotides synthesized in the above Examples and Reference Examples were placed in the combinations shown in Tables 3 to 6 into one tube at concentrations of 300 pmol of each sense and antisense strand and dried under reduced pressure. 30 µL of an siRNA suspension buffer (QIAGEN) was added thereto, and the mixture was heated at 65°C for 1 minute and then left at room temperature for 5 minutes for annealing of the sense and antisense strands to obtain a 10 µM double-stranded polynucleotide solution.

Each double-stranded polynucleotide may be indicated only in the combination of sense and antisense strands, i.e., for example, the double-stranded polynucleotide consisting of the combination CT-001/CT-0005 may be simply referred to as "CT-001/CT-005".
Double-stranded polynucleotides and 5'-Ar-double-stranded polynucleotide having an aromatic substituent added to 5'-phosphate in the antisense strand, shown in Tables 3 to 8, can be obtained by the present method.

**[Table 3]**

| Sense strand | Antisense strand | Sense strand | Antisense strand |
|---|---|---|---|
| CT-001 | CT-005 | CT-169 | CT-296 |
| CT-106 | CT-041 | CT-169 | CT-297 |
| CT-169 | CT-157 | CT-169 | CT-298 |
| CT-169 | CT-300 | CT-169 | CT-299 |
| CT-169 | CT-186 | CT-169 | CT-225 |
| CT-169 | CT-203 | CT-169 | CT-259 |
| CT-169 | CT-269 | CT-169 | CT-260 |
| CT-169 | CT-292 | CT-169 | CT-261 |
| CT-169 | CT-263 | CT-169 | CT-262 |
| CT-169 | CT-268 | CT-169 | CT-263 |
| CT-169 | CT-269 | CT-169 | CT-312 |
| CT-169 | CT-267 | CT-169 | CT-313 |
| CT-169 | CT-268 | CT-169 | CT-314 |
| CT-169 | CT-269 | CT-169 | CT-315 |
| CT-169 | CT-270 | CT-169 | CT-316 |
| CT-169 | CT-271 | CT-169 | CT-317 |
| CT-169 | CT-272 | CT-169 | CT-318 |
| CT-169 | CT-273 | CT-169 | CT-319 |
| CT-169 | CT-274 | CT-310 | CT-311 |
| CT-169 | CT-275 | CT-310 | CT-330 |
| CT-169 | CT-276 | CT-169 | CT-321 |
| CT-169 | CT-277 | CT-169 | CT-325 |
| CT-169 | CT-290 | CT-169 | CT-327 |
| CT-169 | CT-291 | CT-169 | CT-331 |
| CT-169 | CT-292 | CT-169 | CT-336 |
| CT-169 | CT-293 | CT-169 | CT-337 |
| CT-169 | CT-294 | CT-169 | CT-338 |
| CT-169 | CT-295 | | |

**[Table 4]**

| Sense strand | Antisense strand |
|---|---|
| CT-169 | CT-320 |
| CT-169 | CT-322 |
| CT-169 | CT-323 |
| CT-169 | CT-326 |
| CT-169 | CT-332 |
| CT-169 | CT-335 |
| CT-169 | CT-351 |
| CT-169 | CT-352 |
| CT-169 | CT-353 |
| CT-169 | CT-354 |
| CT-169 | CT-355 |
| CT-169 | CT-359 |
| CT-169 | CT-360 |

**[Table 5]**

| Sense strand | Antisense strand |
|---|---|
| CT-169 | CT-365 |
| CT-169 | CT-366 |
| CT-169 | CT-367 |
| CT-169 | CT-368 |
| CT-169 | CT-369 |
| CT-169 | CT-370 |
| CT-169 | CT-371 |
| CT-169 | CT-373 |
| CT-169 | CT-374 |
| CT-169 | CT-375 |

**[Table 6]**

| Sense strand | Antisense strand | Sense strand | Antisense strand |
|---|---|---|---|
| CT-106 | CT-339 | CT-342 | CT-330 |
| CT-001 | CT-377 | CT-343 | CT-330 |
| CT-165 | CT-166 | CT-348 | CT-330 |
| CT-165 | CT-378 | CT-349 | CT-330 |
| CT-278 | CT-280 | CT-350 | CT-330 |
| CT-278 | CT-379 | CT-169 | CT-387 |
| MC-006 | MC-007 | CT-169 | CT-388 |
| MC-008 | MC-009 | CT-169 | CT-389 |
| MC-008 | MC-016 | CT-169 | CT-390 |
| CT-310 | CT-330 | CT-169 | CT-391 |
| CT-344 | CT-330 | CT-169 | CT-392 |
| CT-345 | CT-330 | CT-169 | CT-438 |
| CT-346 | CT-330 | CT-169 | CT-439 |
| CT-347 | CT-330 | CT-169 | CT-457 |
| CT-169 | CT-380 | CT-169 | CT-458 |
| CT-169 | CT-381 | | |
| CT-169 | CT-382 | | |
| CT-169 | CT-383 | | |

### (Test Example 1)

### (1) Transfection

A human colon cancer SW480 cell strain (derived from human adenocarcinoma of the large intestine) was cultured in an RPMI1640 medium (Invitrogen Corp.) containing 10% fetal bovine serum. The culture solution of SW480 was seeded at a concentration of 100000 cells/well onto a 12-well plate and cultured overnight. Next, a lipofection reagent, HiPerFect Transfection Reagent (QIAGEN), at a final concentration of 0.5% and a double-stranded polynucleotide solution at a final concentration of 30, 3, 0.3, or 0.03 nM (or 3, 1, 0.3, 0.1, or 0.03 nM and the like: shown in the diagrams) were added to each well, and the culture was further continued for 3 days. Then, the medium was removed, and the cells were washed with PBS (phosphate buffered saline) and then lysed by the direct addition of 100 µL of Laemmli Sample Buffer containing 5% 2-mercaptoethanol. The cell lysate was collected into a tube and then heated at 100°C for 5 minutes to effect protein denaturation. The structures and nucleotide sequences of the double-stranded polynucleotides are shown in Figures 1, 2, 4, 6, 8, 10, 16, 18, 19, 24, 26, 27, 29, 31, 32, 34, 37 and 39.

### (2) Western blot analysis

Each sample (1 µg in terms of protein amount) was separated by polyacrylamide gel electrophoresis (5-20% gradient gel) and electrically transferred to a nitrocellulose membrane. The membrane was blocked with a 5% skim milk solution. Then, β-catenin proteins were detected using rabbit anti-β-catenin antibodies (Cell Signaling Technology, Inc.) as primary antibodies and HRP-labeled anti-rabbit IgG antibodies (GE Healthcare Life Sciences) as secondary antibodies. β-actin proteins were detected as a negative control using anti-β-actin monoclonal antibodies (GE Healthcare Life Sciences) and HRP-labeled anti-mouse IgG antibodies (GE Healthcare Life Sciences). Each protein detection was performed by visualization based on High Performance Chemiluminescence Film (GE Healthcare Life Sciences) photosensitized with chemiluminescence generated with Western Lightning (PerkinElmer Life Sciences) as a substrate.

### (3) Results of Western blot analysis

### (a) Gene expression inhibitory activities of double-stranded polynucleotides synthesized as Reference Examples

The experiment was carried out on a double-stranded polynucleotide consisting of the combination CT-106/CT-041 in which all nucleotides in the double-stranded polynucleotide consisted of RNAs (hereinafter, each double-stranded polynucleotide may be indicated only in the combination of sense and antisense strands, i.e., for example, the double-stranded polynucleotide consisting of the combination CT-106/CT-041 may be simply referred to as "CT-106/CT-041"), and on a double-stranded polynucleotide CT-001/CT-005 comprising RNAs alternated with 2'-O-methyl RNAs, and DNAs in the overhang moiety. The structures of these double-stranded polynucleotides are shown in Figure 1. CT-001/CT-005 inhibited the expression of the human β-catenin gene at a level equivalent to CT-106/CT-041 in which all nucleotides consisted of RNAs (Figure 1). In the subsequent experiments, CT-001/CT-005 or CT-106/CT-041 was used as a control.

### (b) Analysis of gene inhibitory activities of double-stranded polynucleotides - 1 -

CT-169/CT-300 in which a 4-ethylphenylphosphate group was bound to the 5'-end of the antisense strand in the double-stranded polynucleotide CT-169/CT-157, and CT-169/CT-186 in which a 2-hydroxyethylphosphate group was bound thereto (for their structures, see Figure 2) were examined for their gene expression inhibitory activities. As shown in Figure 3, CT-169/CT-300 strongly inhibited the expression of the human β-catenin gene at a level equivalent to CT-169/CT-157. However, no inhibition of the expression of the human β-catenin gene was seen in CT-169/CT-186. Moreover, CT-169/CT-269 in which a 4-ethylphenylphosphate group was bound to the 5'-end of the antisense strand in the double-stranded polynucleotide CT-169/CT-203, and CT-169/CT-292 in which a 4-phenylphenylphosphate group was bound thereto (see Figure 2) were examined for their gene expression inhibitory activities. As shown in Figure 3, CT-169/CT-269 and CT-169/CT-292 strongly inhibited the expression of the human β-catenin gene. This shows that a double-stranded polynucleotide in which a phenylphosphate group having a substituent is bound to the 5'-end of the antisense strand has strong gene expression inhibitory activity.

### (c) Analysis of gene inhibitory activities of double-stranded polynucleotides - 2 -

CT-169/CT-263, CT-169/CT-268, CT-169/CT-269, CT-169/CT-270, CT-169/CT-271, CT-169/CT-272, CT-169/CT-273, CT-169/CT-274, CT-169/CT-275, CT-169/CT-276, and CT-169/CT-277 in which a phenylphosphate group having a substituent was bound to the 5'-end of the antisense strand in the double-stranded polynucleotide CT-169/CT-203 (for their structures, see Figure 4) were examined for their β-catenin gene expression inhibitory activities.

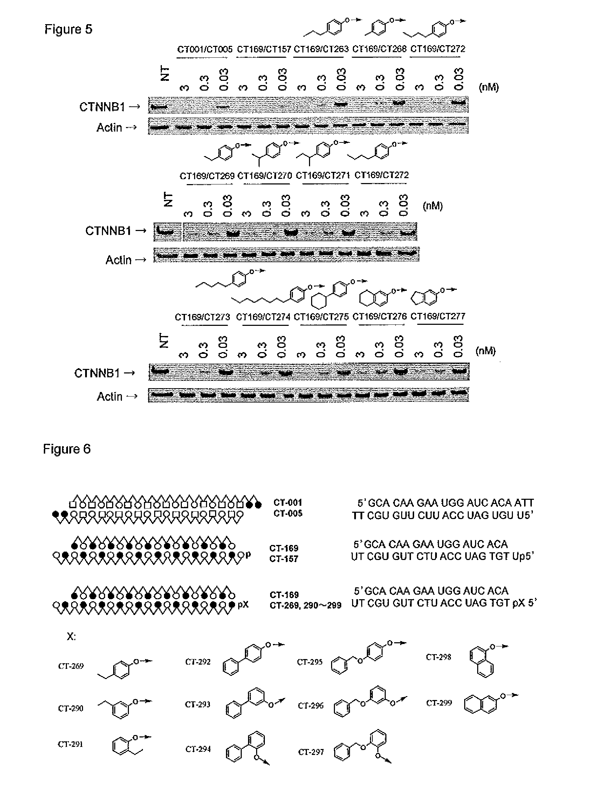

As shown in Figure 5, the double-stranded polynucleotides CT-169/CT-263, CT-169/CT-268, CT-169/CT-269, CT-169/CT-270, CT-169/CT-271, CT-169/CT-272, CT-169/CT-273, CT-169/CT-274, CT-169/CT-275, CT-169/CT-276, and CT-169/CT-277 strongly inhibited the expression of the β-catenin gene. This shows that a double-stranded polynucleotide having a phenylphosphate group having a substituent, at the 5'-end of the antisense strand has strong gene expression inhibitory activity.

### (d) Analysis of gene inhibitory activities of double-stranded polynucleotides - 3 -

CT-169/CT-269, CT-169/CT-290, CT-169/CT-291, CT-169/CT-292, CT-169/CT-293, CT-169/CT-294, CT-169/CT-295, CT-169/CT-296, CT-169/CT-297, CT-169/CT-298, and CT-169/CT-299 in which a phenylphosphate group having a substituent was bound to the 5'-end of the antisense strand in the double-stranded polynucleotide CT-169/CT-203 (for their structures, see Figure 6) were examined for their β-catenin gene expression inhibitory activities.

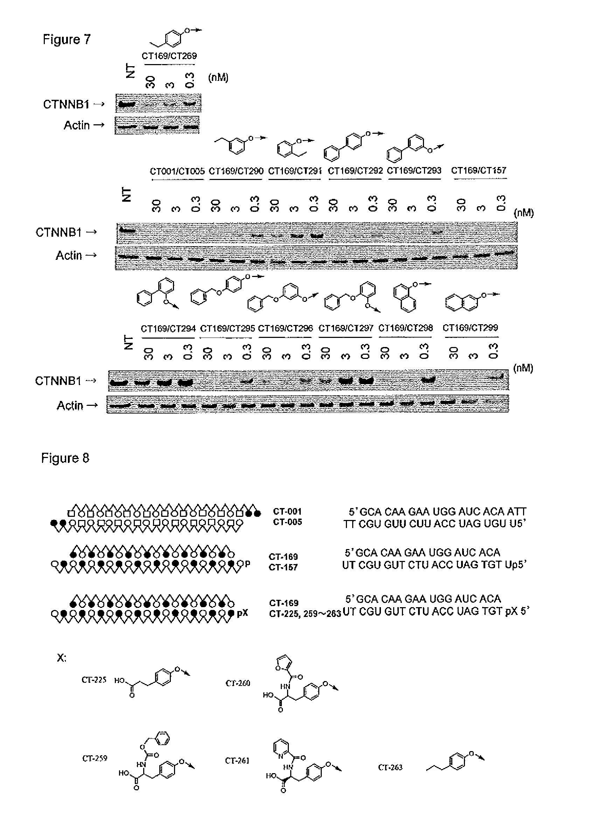

As shown in Figure 7, the double-stranded polynucleotides CT-169/CT-269, CT-169/CT-290, CT-169/CT-292, CT-169/CT-293, CT-169/CT-295, CT-169/CT-296, and CT-169/CT-299 strongly inhibited the expression of the β-catenin gene. By contrast, CT-169/CT-291, CT-169/CT-294, CT-169/CT-297, and CT-169/CT-298 had low β-catenin gene expression inhibitory activity. This shows that: the meta or para position rather than the ortho position is preferable as a substitution position on the phenylphosphate group at the 5'-end of the antisense strand; and a double-stranded polynucleotide having an ethyl group, a phenyl group, or a benzyloxy group has strong gene expression inhibitory activity.

### (e) Analysis of gene inhibitory activities of double-stranded polynucleotides - 4 -

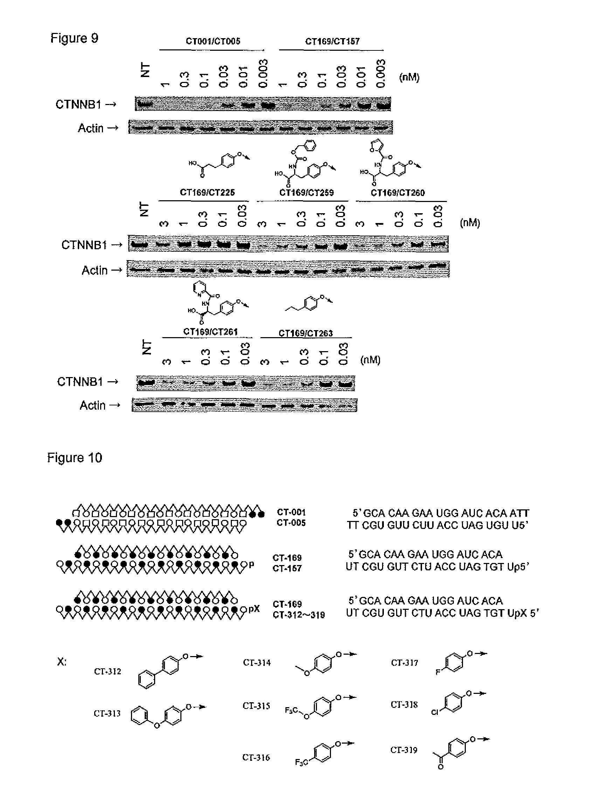

CT-169/CT-225, CT-169/CT-259, CT-169/CT-260, CT-169/CT-261, and CT-169/CT-263 in which a phenylphosphate group having a substitution was bound to the 5'-end of the antisense strand in the double-stranded polynucleotide CT-169/CT-203 (for their structures, see Figure 8) were examined for their β-catenin gene expression inhibitory activities.
As shown in Figure 9, the double-stranded polynucleotides CT-169/CT-259, CT-169/CT-260, CT-169/CT-261, and CT-169/CT-263 strongly inhibited the expression of the β-catenin gene. By contrast, CT-169/CT-225 had low β-catenin gene expression inhibitory activity. The double-stranded polynucleotide CT-169/CT-263 having a 4-propylphenylphosphate group at the 5'-end of the antisense strand had strong gene expression inhibitory activity, whereas CT-169/CT-225 in which carboxylic acid was introduced in CT-169/CT-263 had lower β-catenin gene expression inhibitory activity. However, CT-169/CT-259, CT-169/CT-260, and CT-169/CT-261 in which a substituent was further introduced in CT-169/CT-225 were shown to have strong gene expression inhibitory activity.

### (f) Analysis of gene inhibitory activities of double-stranded polynucleotides - 5 -

CT-169/CT-312, CT-169/CT-313, CT-169/CT-314, CT-169/CT-315, CT-169/CT-316, CT-169/CT-317, CT-169/CT-318, and CT-169/CT-319 in which a phenylphosphate group having a substitution was bound to the 5'-end of the antisense strand in the double-stranded polynucleotide CT-169/CT-157 (for their structures, see Figure 10) were examined for their β-catenin gene expression inhibitory activities.

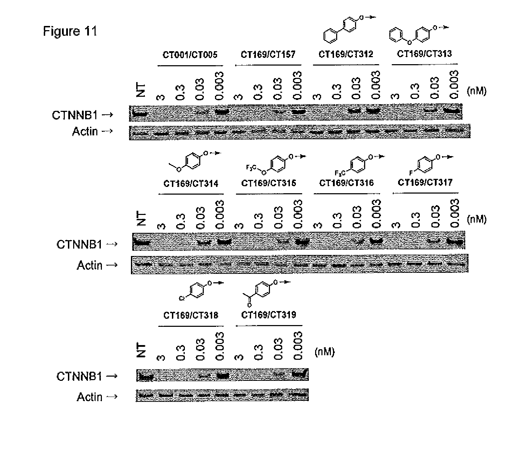

As shown in Figure 11, the double-stranded polynucleotides CT-169/CT-312, CT-169/CT-313, CT-169/CT-314, CT-169/CT-315, CT-169/CT-316, CT-169/CT-317, CT-169/CT-318, and CT-169/CT-319 strongly inhibited the expression of the β-catenin gene at a level equivalent to CT-001/CT-005 and CT-169/CT-157.

### (g) Analysis of gene inhibitory activities of double-stranded polynucleotides - 6 -

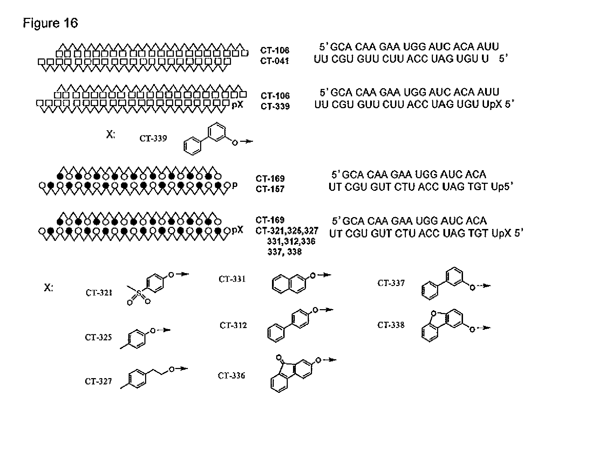

CT-106/CT-339 in which a 3-phenylphenylphosphate group was bound to the 5'-end of the antisense strand in the natural double-stranded polynucleotide CT-106/CT-041 (for its structure, see Figure 16) was examined for its β-catenin gene expression inhibitory activity.

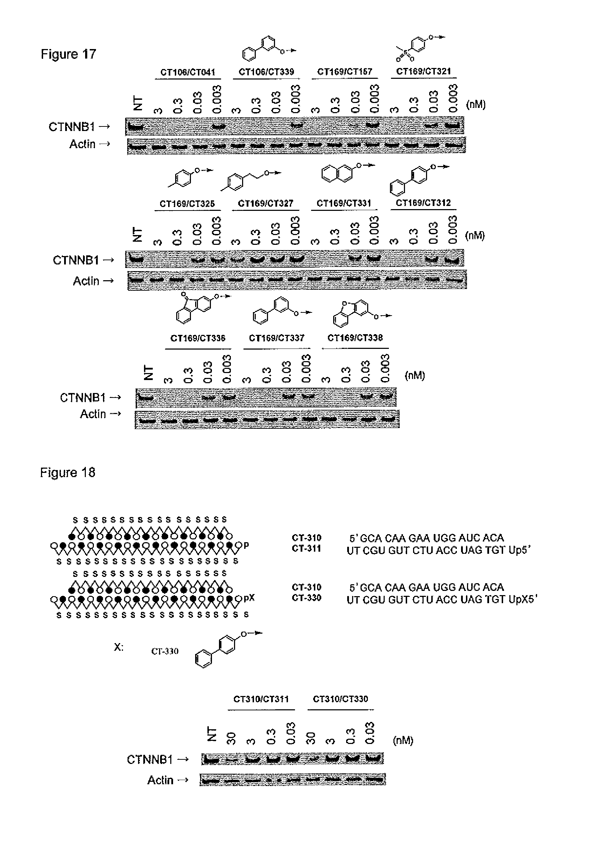

As shown in Figure 17, the double-stranded polynucleotide CT-106/CT-339 strongly inhibited the expression of the β-catenin gene at a level equivalent to the natural double-stranded polynucleotide CT-106/CT-041. This shows that a natural double-stranded polynucleotide having a phenylphosphate group having a substituent, at the 5'-end of the antisense strand has strong gene expression inhibitory activity.

### (h) Analysis of gene inhibitory activities of double-stranded polynucleotides - 7 -

CT-169/CT-321, CT-169/CT-325, CT-169/CT-327, CT-169/CT-331, CT-169/CT-312, CT-169/CT-336, CT-169/CT-337, and CT-169/CT-338 in which a phenylphosphate group having a substitution or a 4-methylphenenylphosphate group was bound to the 5'-end of the antisense strand in the double-stranded polynucleotide CT-169/CT-157 (for their structures, see Figure 16) were examined for their β-catenin gene expression inhibitory activities.

As shown in Figure 17, the double-stranded polynucleotides CT-169/CT-321, CT-169/CT-325, CT-169/CT-331, CT-169/CT-312, CT-169/CT-336, CT-169/CT-337, and CT-169/CT-338 strongly inhibited the expression of the β-catenin gene at a level equivalent to CT-169/CT-157. By contrast, CT-169/CT-327 had low β-catenin gene expression inhibitory activity. This shows that a natural double-stranded polynucleotide having a phenylphosphate group having a substituent, at the 5'-end of the antisense strand has strong gene expression inhibitory activity.

### (i) Analysis of gene inhibitory activities of double-stranded polynucleotides - 8 -

CT-310/CT-330 in which a 3-phenylphenylthiophosphate group was bound to the 5'-end of the antisense strand in a double-stranded polynucleotide CT-310/CT-311 having a phosphorothioate bond instead of the phosphodiester bond of the double-stranded polynucleotide CT-169/CT-157 (for its structure, see Figure 18) was examined for its β-catenin gene expression inhibitory activity.

As shown in Figure 18, the double-stranded polynucleotide CT-310/CT-330 inhibited the expression of the β-catenin gene at a level equivalent to CT-310/CT-311. This shows that a double-stranded polynucleotide having a phenylthiophosphate group having a substituent, at the 5'-end of the antisense strand has strong gene expression inhibitory activity.

### (Test Example 2) Test on resistance to phosphatase

400 pmol of each of the polynucleotides CT-203, and CT-292 (see Figure 2) was brought to the total volume of 49 µL with Shrimp Alkaline phosphatase buffer (50 mM Tris-HCl pH 9.0, 5 mM MgCl₂, TAKARA BIO INC.). 1 U Shrimp Alkaline phosphatase (SAP, 1 µL, TAKARA BIO INC.) was added thereto, and the mixture was heated at 37°C for 15 minutes. After further heating at 60°C for 15 minutes, each reaction solution was analyzed by reverse-phase HPLC (LC-10VP manufactured by Shimadzu Corp., column (Merck, Chromolith Performance RP-18e (4.6×100 mm)), Solution A: 5% acetonitrile, 0.1 M aqueous triethylamine acetate solution (TEAA), pH 7.0, Solution B: acetonitrile, B%: 0%→10% (10 min, linear gradient for CT-203) or B%: 0%→30% (10 min, linear gradient for CT-292); 60°C; 2 ml/min; 260 nm).

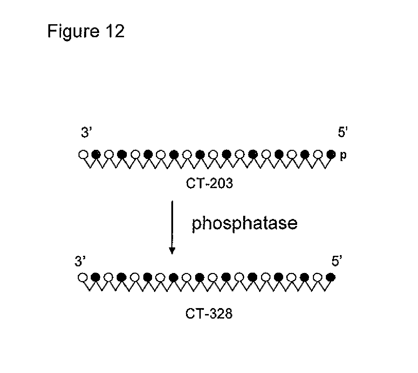

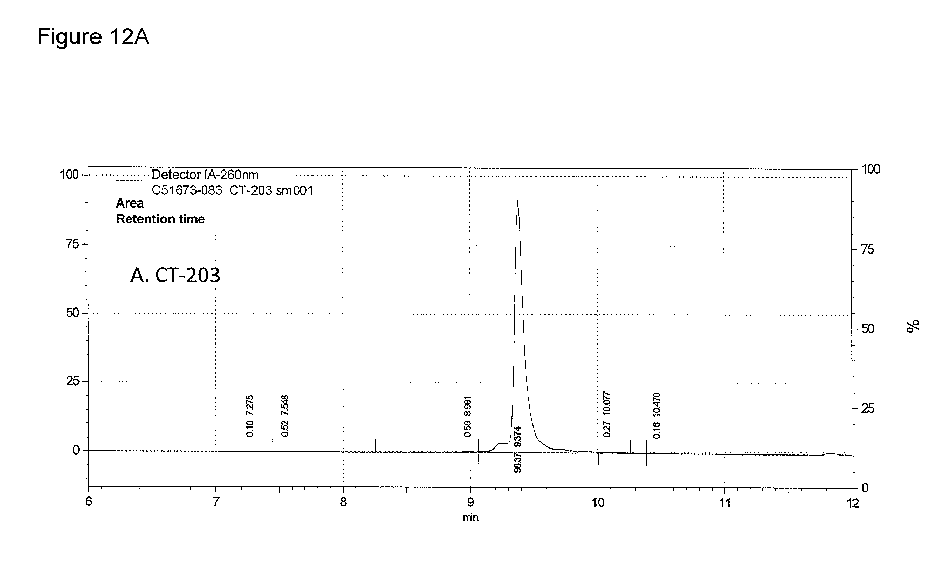

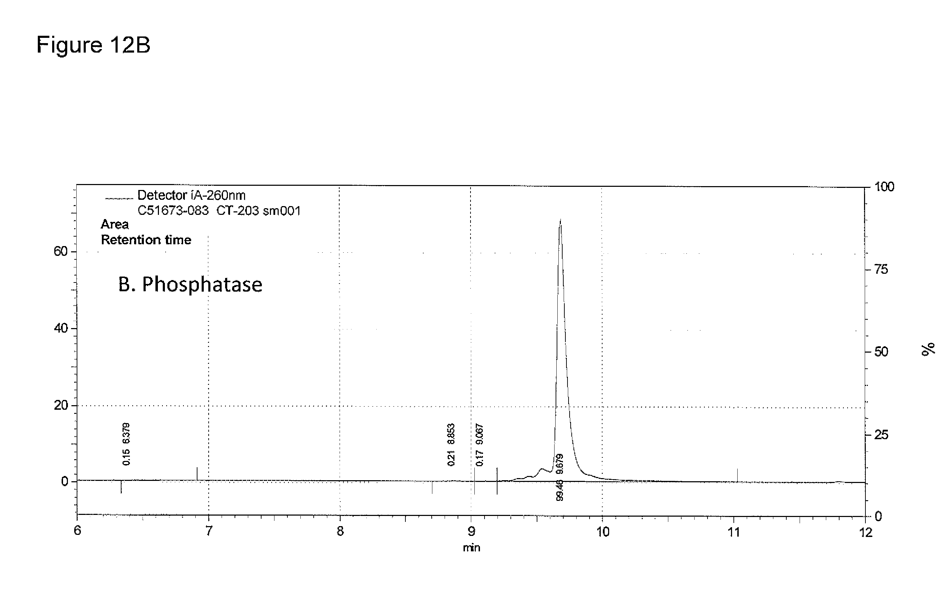

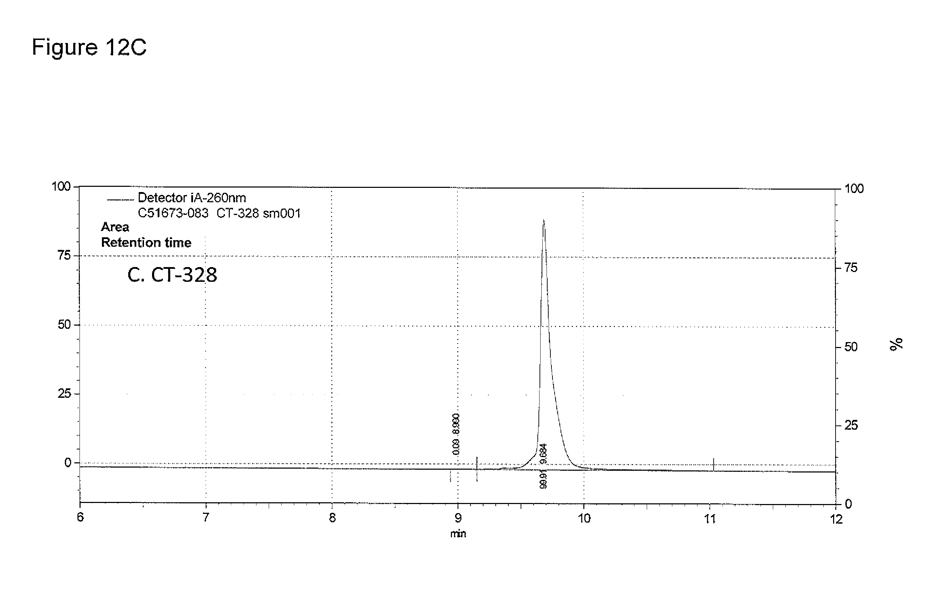

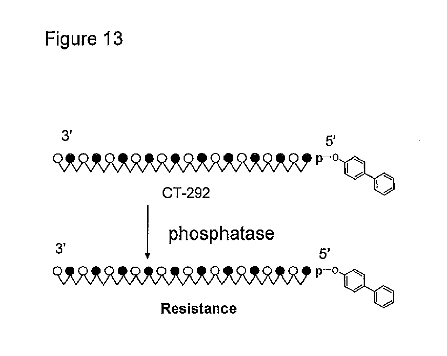

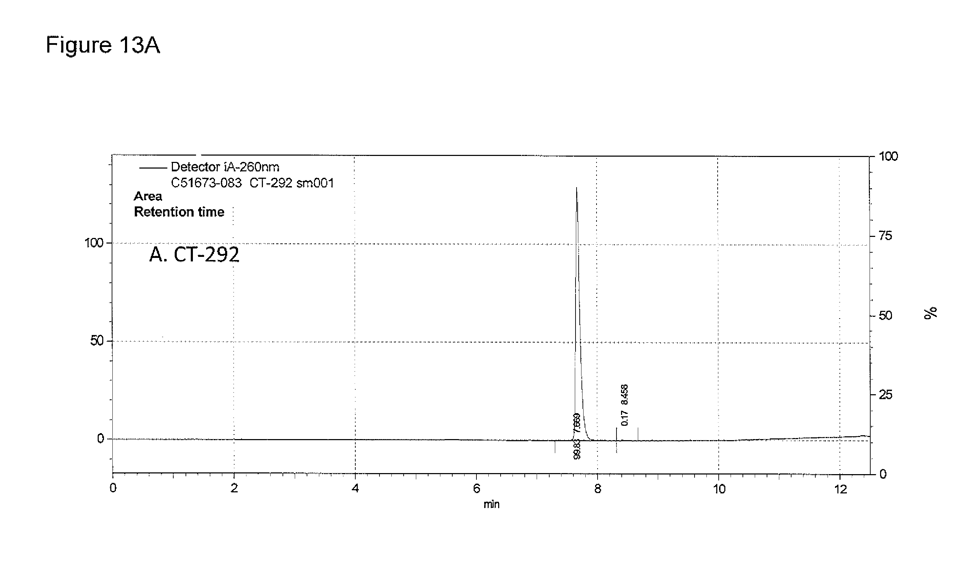

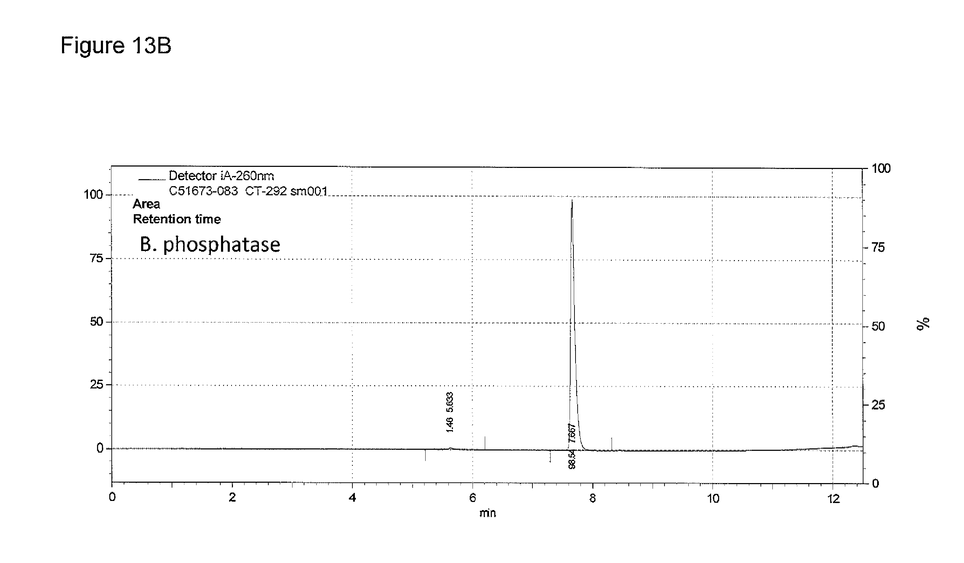

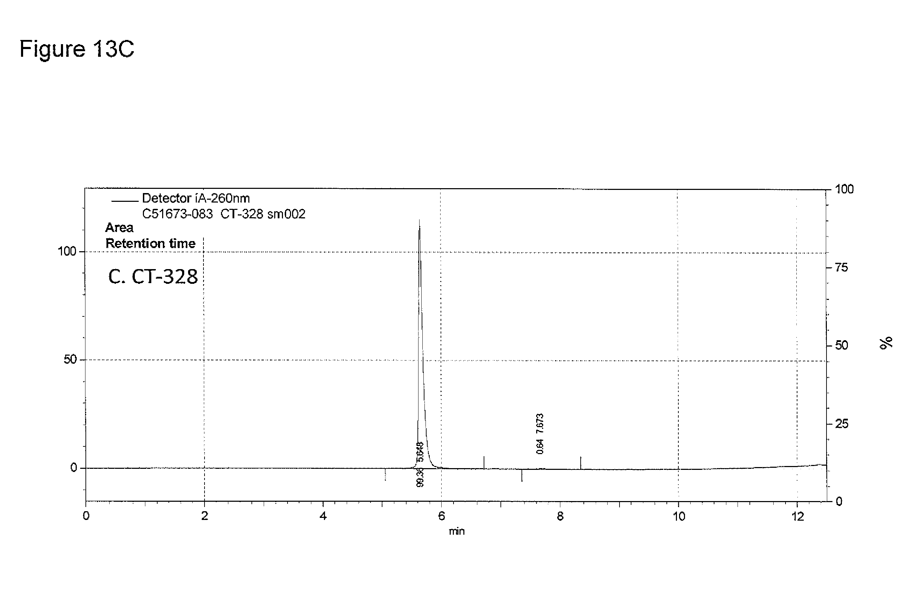

The polynucleotide CT-203 was rapidly dephosphorylated by SAP and gave the same retention time as that of the dephosphorylated form CT-328 (see Figures 12A, 12B, and 12C). On the other hand, the polynucleotide CT-292 having a 4-phenylphenylphosphate group was resistant to SAP even without being dephosphorylated by the SAP treatment (see Figures 13A, 13B, and 13C).

### (Test Example 3) Test on resistance to exonuclease

400 pmol of each of the polynucleotides CT-203, and CT-292 (see Figure 2) was brought to the total volume of 19 µL with a buffer (50 mM Tris-HCl pH 8.0, 2 mM MgCl₂, 0.1 M NaCl, Epicentre). 1 U 5'-phosphate-dependent exonuclease (1 µL, Epicentre) was added thereto, and the mixture was heated at 30°C. 15, 30, and 45 minutes later, a 5 µl aliquot of each sample was added to a stop solution (0.2 µL of 120 mM EDTA, 7 µL of H₂O). Each reaction solution was analyzed by reverse-phase HPLC (LC-10VP manufactured by Shimadzu Corp., column (Merck, Chromolith Performance RP-18e (4.6×100 mm)), Solution A: 5% acetonitrile, 0.1 M aqueous triethylamine acetate solution (TEAA), pH 7.0, Solution B: acetonitrile, B%: 0%→10% (10 min, linear gradient for CT-203) or B%:0%→30% (10 min, linear gradient for CT-292); 60°C; 2 ml/min; 260 nm).

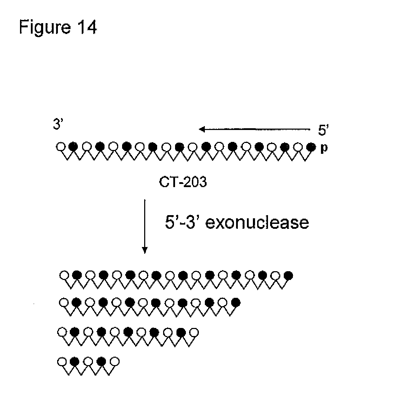

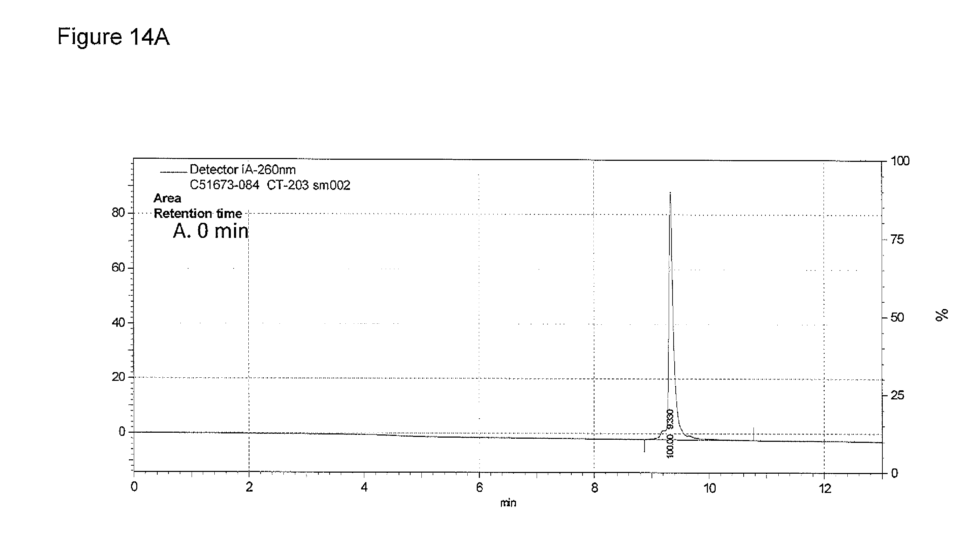

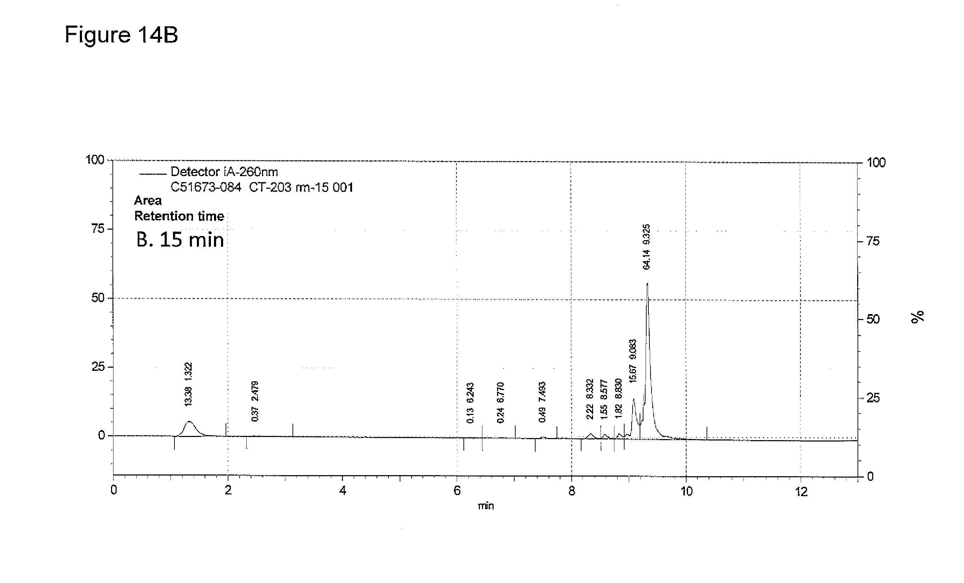

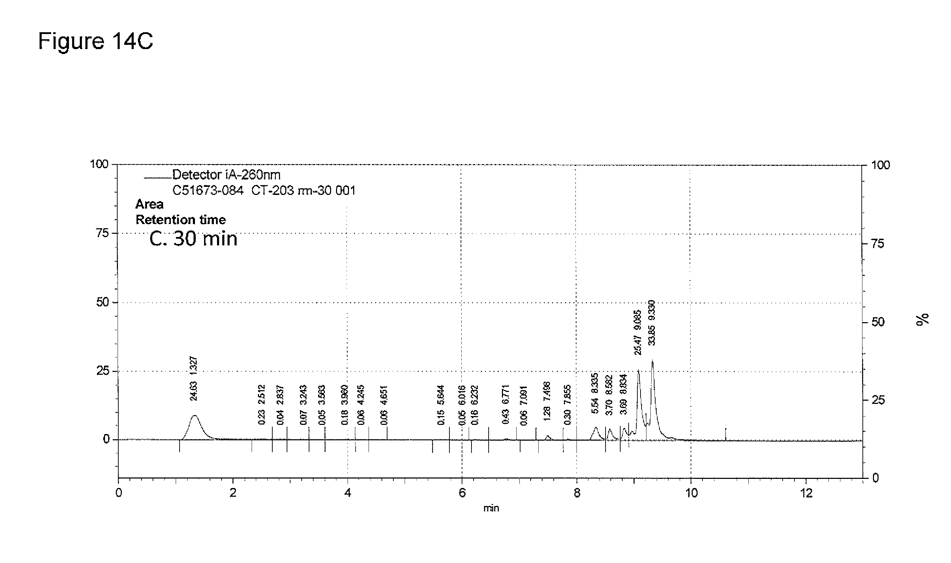

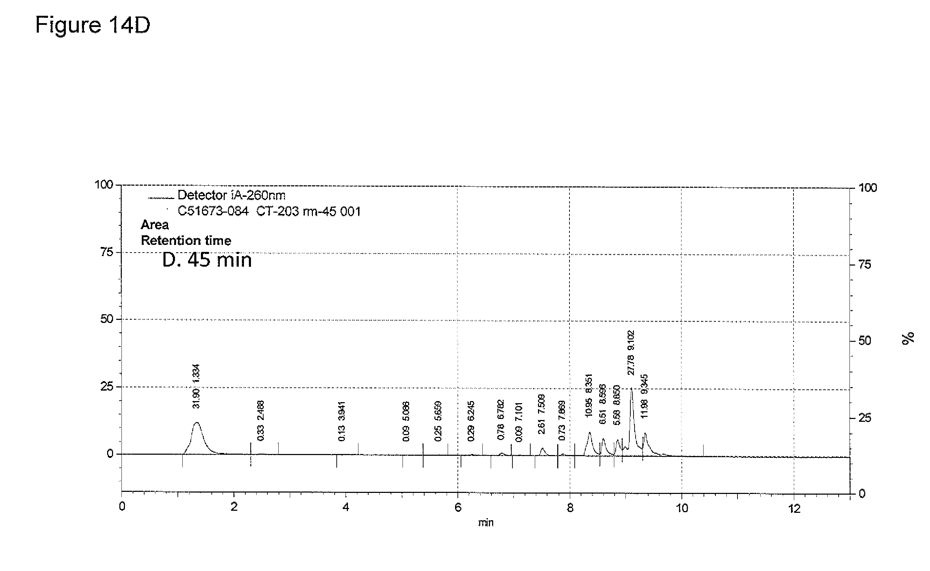

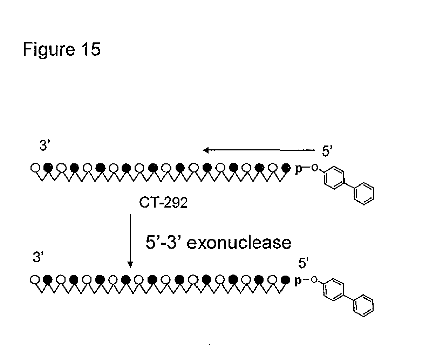

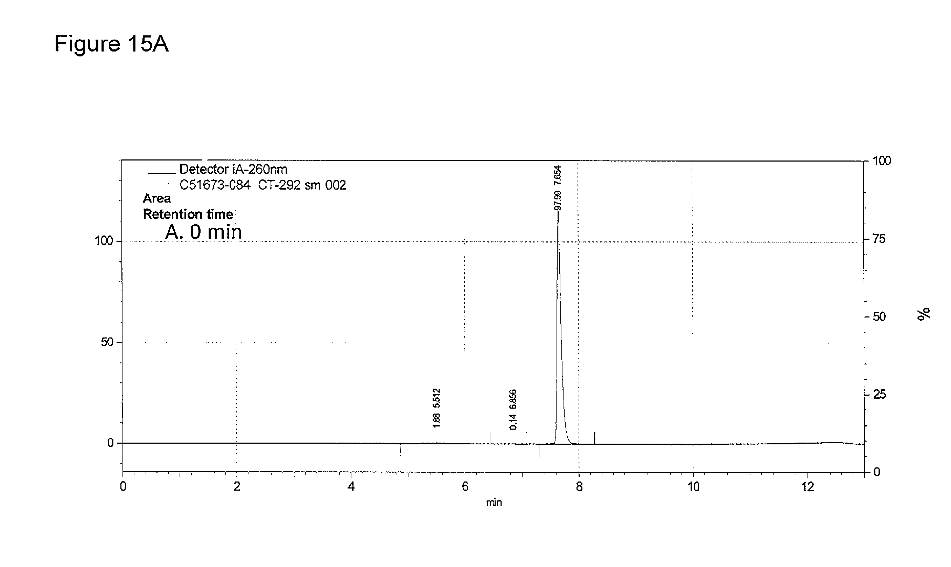

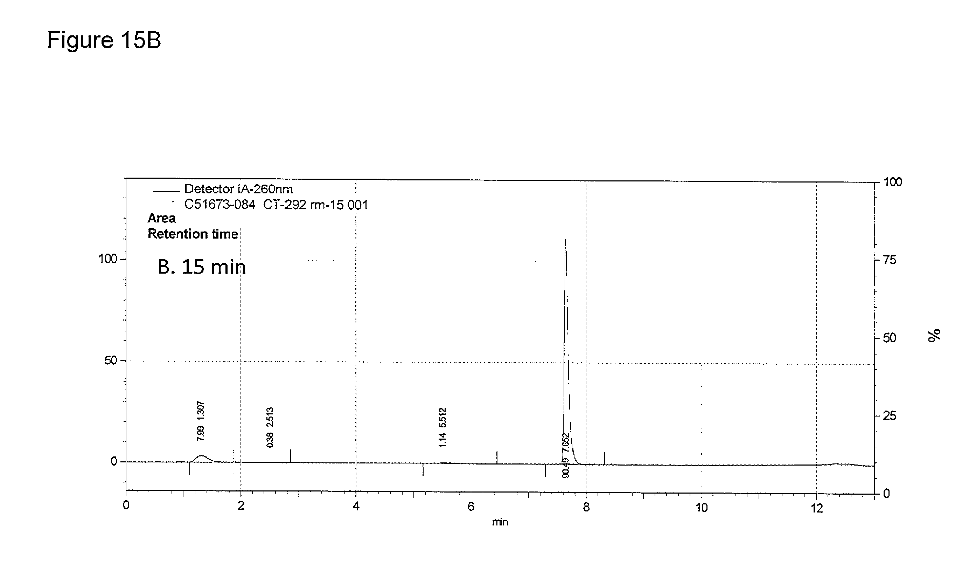

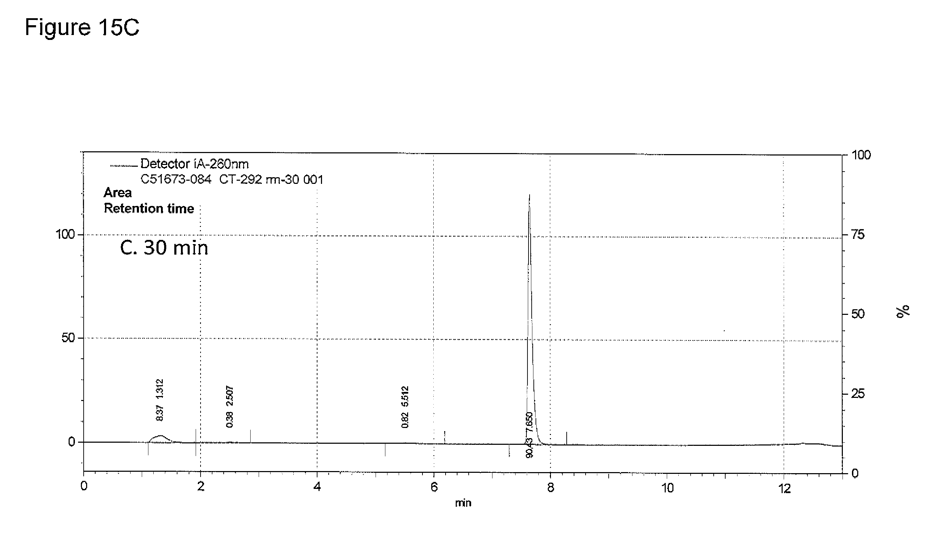

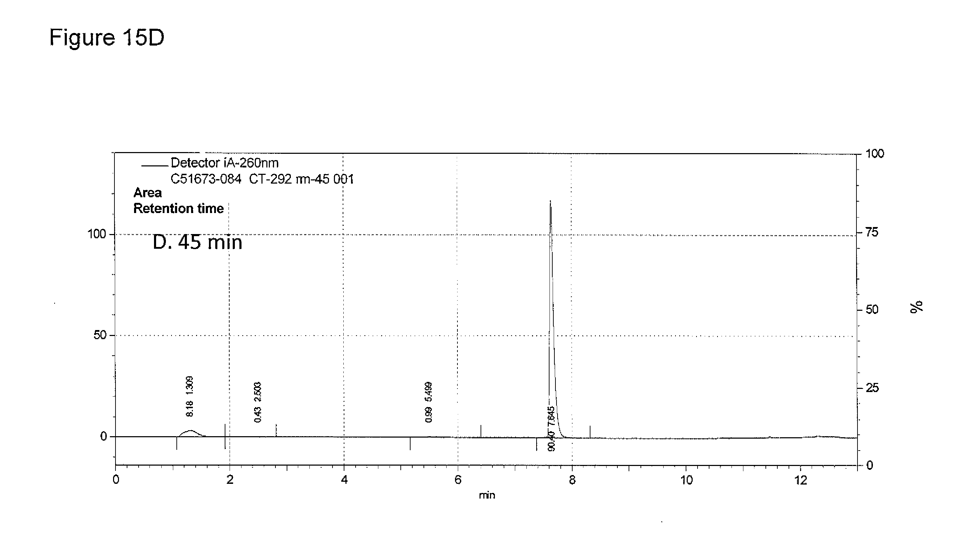

A peak probably derived from a strand shortened by exonuclease degradation was observed in the polynucleotide CT-203 (see Figure 14). On the other hand, no change in peak was seen in the polynucleotide CT-292 having a 4-phenylphenylphosphate group even after the exonuclease treatment. This shows that CT-292 is very resistant to exonuclease without being degraded (see Figure 15).

### (Test Example 4)

The intensity of gene expression inhibitory activity was compared among double-stranded polynucleotides as follows in the same way as in Test Example 1.

### (a) Analysis of gene expression inhibitory activities of double-stranded polynucleotides - 1 -

CT-169/CT-320, CT-169/CT-322, CT-169/CT-323, CT-169/CT-326, CT-169/CT-332, and CT-169/CT-335 in which a phenylphosphate group having a substitution or a 4-methylbenzylphosphate group is bound to the 5'-end of the antisense strand in the double-stranded polynucleotide CT-169/CT-157 (for their structures, see Figure 19) were examined for their β-catenin gene expression inhibitory activities.

As shown in Figure 22, the double-stranded polynucleotides CT-169/CT-320, CT-169/CT-322, CT-169/CT-323, CT-169/CT-332, and CT-169/CT-335 strongly inhibited the expression of the β-catenin gene, as in CT-169/CT-157. By contrast, CT-169/CT-326 had low β-catenin gene expression inhibitory activity. This shows that a natural double-stranded polynucleotide having a phenylphosphate group having a substituent, at the 5'-end of the antisense strand has strong gene expression inhibitory activity.

### (b) Analysis of gene expression inhibitory activities of double-stranded polynucleotides - 2 -

CT-169/CT-351, CT-169/CT-352, CT-169/CT-353, CT-169/CT-354, CT-169/CT-355, CT-169/CT-359, and CT-169/CT-360 in which a phenylphosphate group having a substitution was bound to the 5'-end of the antisense strand in the double-stranded polynucleotide CT-169/CT-157 (for their structures, see Figure 19) were examined for their β-catenin gene expression inhibitory activities.

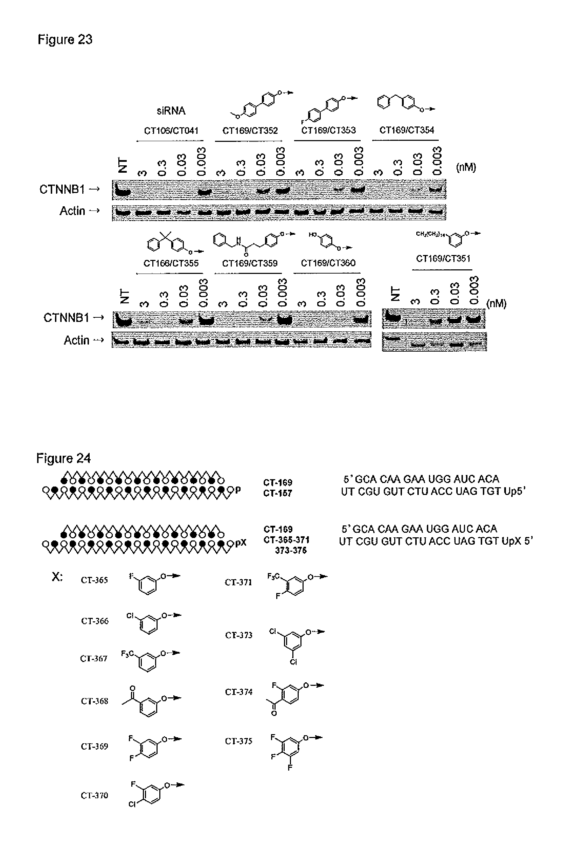

As shown in Figure 23, the double-stranded polynucleotides CT-169/CT-352, CT-169/CT-353, CT-169/CT-354, CT-169/CT-355, CT-169/CT-359, and CT-169/CT-360 strongly inhibited the expression of the β-catenin gene, as in CT-106/CT-041. By contrast, CT-169/CT-351 had low β-catenin gene expression inhibitory activity. This shows that a natural double-stranded polynucleotide having a phenylphosphate group having a substituent, at the 5'-end of the antisense strand has strong gene expression inhibitory activity.

### (c) Analysis of gene expression inhibitory activities of double-stranded polynucleotides - 3 -

CT-169/CT-365, CT-169/CT-366, CT-169/CT-367, CT-169/CT-368, CT-169/CT-369, CT-169/CT-370, CT-169/CT-371, CT-169/CT-373, CT-169/CT-374, and CT-169/CT-375 in which a phenylphosphate group having a substitution was bound to the 5'-end of the antisense strand in the double-stranded polynucleotide CT-169/CT-157 (for their structures, see Figure 24) were examined for their β-catenin gene expression inhibitory activities.

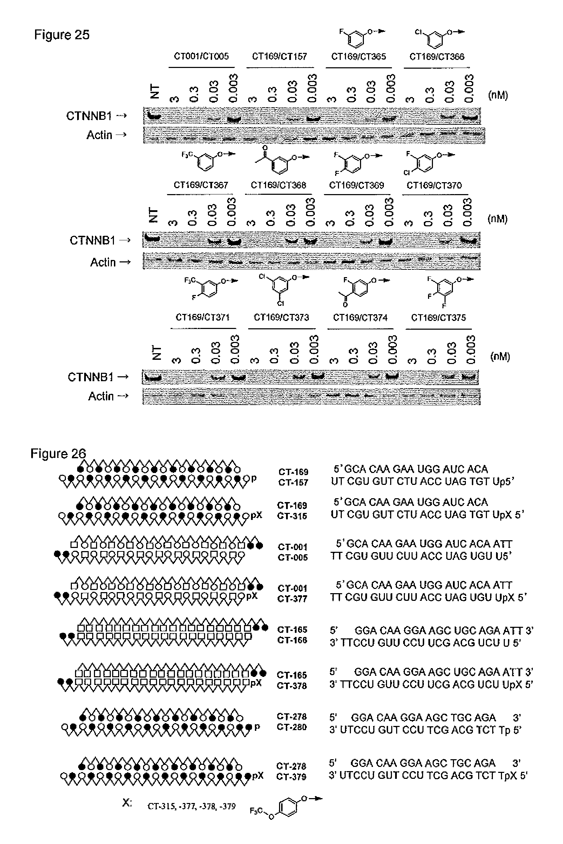

As shown in Figure 25, the double-stranded polynucleotides CT-169/CT-365, CT-169/CT-366, CT-169/CT-367, CT-169/CT-368, CT-169/CT-369, CT-169/CT-370, CT-169/CT-371, CT-169/CT-373, CT-169/CT-374, and CT-169/CT-375 strongly inhibited the expression of the β-catenin gene, as in CT-169/CT-157. This shows that a natural double-stranded polynucleotide having a phenylphosphate group having a substituent, at the 5'-end of the antisense strand has strong gene expression inhibitory activity.

### (d) Analysis of gene inhibitory activities of double-stranded polynucleotides - 4 -

CT-169/CT-315 in which a 4-(trifluoromethoxy)phenylphosphate group was bound to the 5'-end of the antisense strand in the double-stranded polynucleotide CT-169/CT-157 consisting of DNAs and 2'-OMeRNAs, CT-001/CT-377 in which a 4-(trifluoromethoxy)phenylphosphate group was bound to the 5'-end of the antisense strand in the double-stranded polynucleotide CT-001/CT-005 consisting of RNAs, DNAs, and 2'-OMeRNAs, CT-165/CT-378 in which a 4-(trifluoromethoxy)phenylphosphate group was bound to the 5'-end of the antisense strand in the double-stranded polynucleotide CT-165/CT-166 consisting of RNAs and DNAs, and CT-278/CT-379 in which a 4-(trifluoromethoxy)phenylphosphate group was bound to the 5'-end of the antisense strand in the double-stranded polynucleotide CT-278/CT-280 consisting of DNAs and 2'-OMeRNAs (for their structures, see Figure 26) were examined for their β-catenin gene expression inhibitory activities.

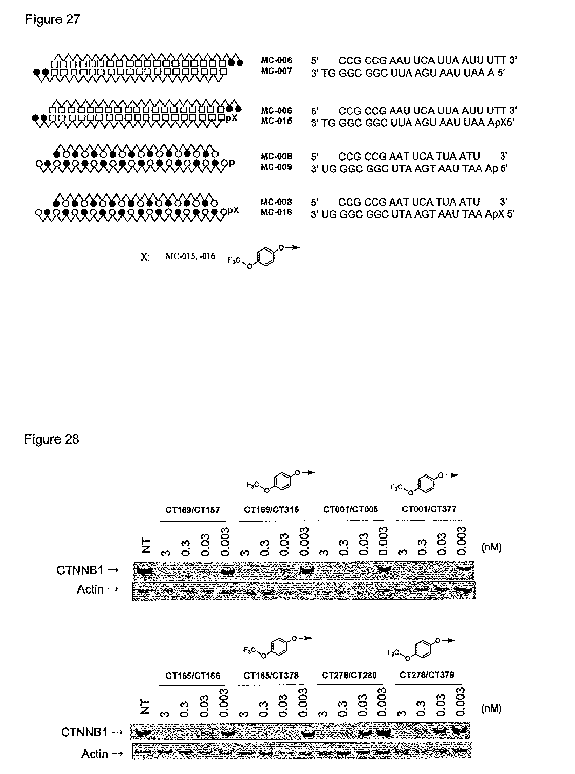

As shown in Figure 28, the double-stranded polynucleotide CT-169/CT-315 strongly inhibited the expression of the β-catenin gene, as in CT-169/CT-157. The double-stranded polynucleotide CT-001/CT-377 strongly inhibited the expression of the β-catenin gene, as in CT-001/CT-005. The double-stranded polynucleotide CT-165/CT-378 strongly inhibited the expression of the β-catenin gene, as in CT-165/CT-166. The double-stranded polynucleotide CT-278/CT-379 strongly inhibited the expression of the β-catenin gene, as in CT-278/CT-280. This shows that a double-stranded polynucleotide consisting of DNAs and 2'-OMeRNAs, consisting of RNAs, or consisting of RNAs and 2'-OMeRNAs and having a phenylphosphate group having a substituent, at the 5'-end of the antisense strand has strong gene expression inhibitory activity.

### (Test Example 5)

The intensity of gene expression inhibitory activity was compared among double-stranded polynucleotides as follows in the same way as in Test Example 1. However, Western blot analysis was conducted as follows.

### (1) Western blot analysis

Each sample (5 µg in terms of protein amount) was separated by polyacrylamide gel electrophoresis (5-20% gradient gel) and electrically transferred to a nitrocellulose membrane. The membrane was blocked with a 5% skim milk solution. Then, MCL1 proteins were detected using rabbit anti-MCL1 antibodies (Santa Cruz Biotechnology, Inc.) as primary antibodies and HRP-labeled anti-rabbit IgG antibodies (GE Healthcare Life Sciences) as secondary antibodies. β-catenin proteins were detected as a negative control using anti-β-catenin antibodies (Cell Signaling Technology, Inc.) and HRP-labeled anti-rabbit IgG antibodies (GE Healthcare Life Sciences). Each protein detection was performed by visualization based on High Performance Chemiluminescence Film (GE Healthcare Life Sciences) photosensitized with chemiluminescence generated with Western Lightning (PerkinElmer Life Sciences) as a substrate.

### (2) Analysis of gene inhibitory activities of double-stranded polynucleotides - 1 -

MC-006/MC-015 in which a 4-(trifluoromethoxy)phenylphosphate group was bound to the 5'-end of the antisense strand in the double-stranded polynucleotide MC-006/MC-007 consisting of RNAs and DNAs, and MC-008/MC-016 in which a 4-(trifluoromethoxy)phenylphosphate group was bound to the 5'-end of the antisense strand in the double-stranded polynucleotide MC-008/MC-009 consisting of DNAs and 2'-OMeRNAs (for their structures, see Figure 27) were examined for their MCL1 gene expression inhibitory activities.

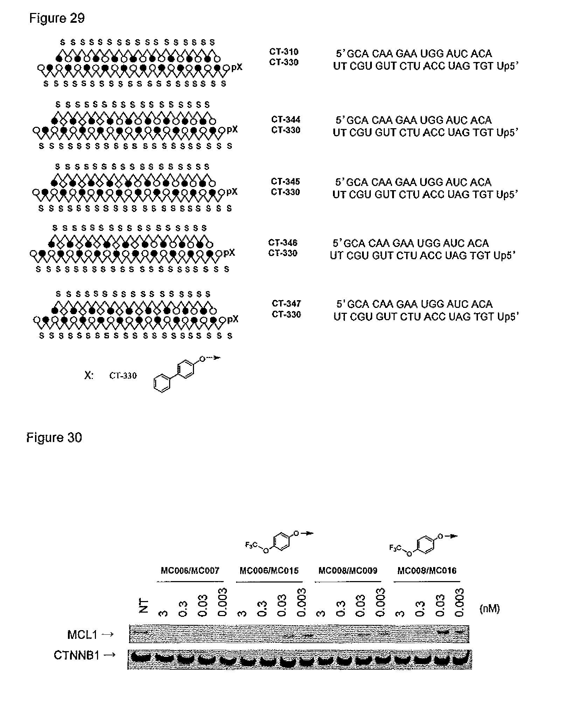

As shown in Figure 30, the double-stranded polynucleotide MC-006/MC-015 strongly inhibited the expression of the β-catenin gene, as in MC-006/MC-007. The double-stranded polynucleotide MC-008/MC-016 strongly inhibited the expression of the β-catenin gene, as in MC-008/MC-009. This shows that a double-stranded polynucleotide consisting of RNAs or consisting of DNAs and 2'-OMeRNAs and having a phenylphosphate group having a substituent, at the 5'-end of the antisense strand has strong gene expression inhibitory activity.

### (Test Example 6)

The intensity of gene expression inhibitory activity was compared among double-stranded polynucleotides as follows in the same way as in Test Example 1.

### (a) Analysis of gene inhibitory activities of double-stranded polynucleotides - 1 -

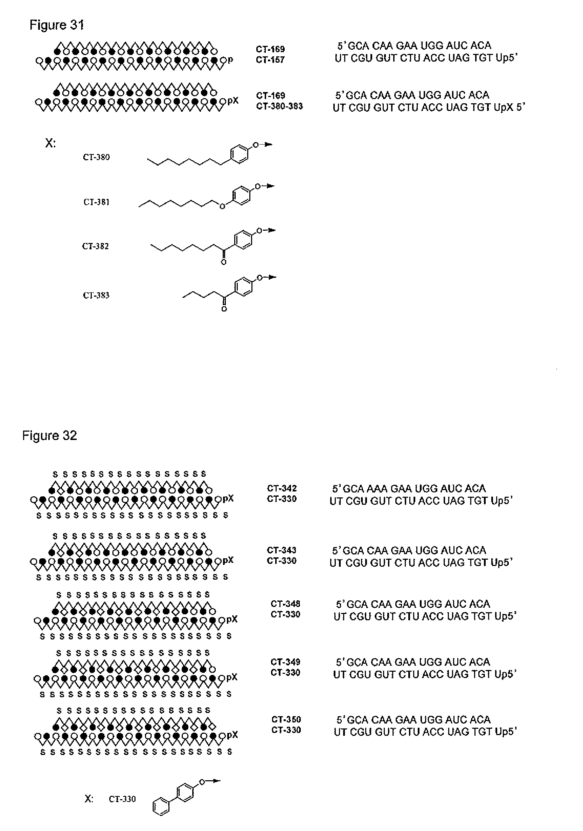

CT-169/CT-380, CT-169/CT-381, CT-169/CT-382, and CT-169/CT-383 in which a phenylphosphate group having a substitution was bound to the 5'-end of the antisense strand in the double-stranded polynucleotide CT-169/CT-157 (for their structures, see Figure 31) were examined for their β-catenin gene expression inhibitory activities.

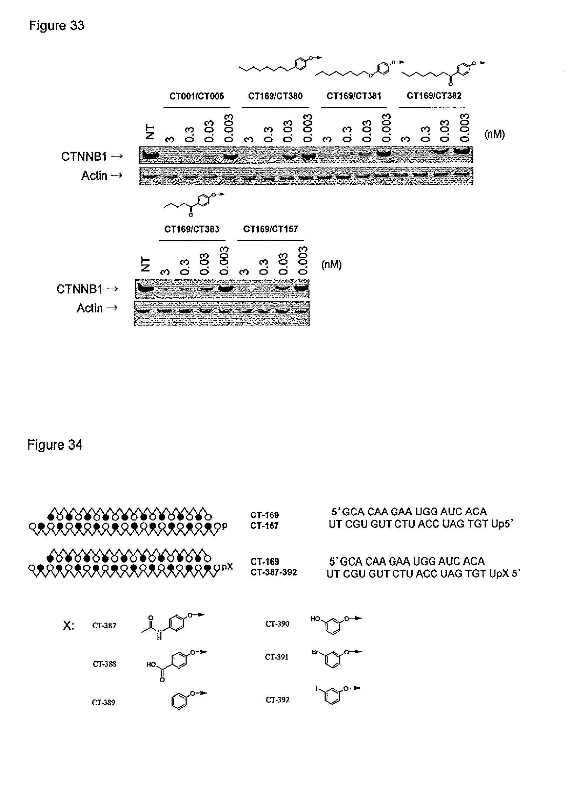

As shown in Figure 33, the double-stranded polynucleotides CT-169/CT-380, CT-169/CT-381, CT-169/CT-382, and CT-169/CT-383 strongly inhibited the expression of the β-catenin gene, as in CT-169/CT-157. This shows that a double-stranded polynucleotide having a phenylphosphate group having a substituent, at the 5'-end of the antisense strand has strong gene expression inhibitory activity.

### (b) Analysis of gene inhibitory activities of double-stranded polynucleotides - 2 -

CT-169/CT-387, CT-169/CT-388, CT-169/CT-389, CT-169/CT-390, CT-169/CT-391, and CT-169/CT-392 in which a phenylphosphate group having a substitution was bound to the 5'-end of the antisense strand in the double-stranded polynucleotide CT-169/CT-157 (for their structures, see Figure 34) were examined for their β-catenin gene expression inhibitory activities.

As shown in Figure 35, the double-stranded polynucleotides CT-169/CT-387, CT-169/CT-388, CT-169/CT-389, CT-169/CT-390, CT-169/CT-391, and CT-169/CT-392 strongly inhibited the expression of the β-catenin gene, as in CT-169/CT-157. This shows that a double-stranded polynucleotide having a phenylphosphate group having a substituent, at the 5'-end of the antisense strand has strong gene expression inhibitory activity.

### (Test Example 7)

The intensity of human β-catenin gene expression inhibitory activity was compared among single-stranded or double-stranded polynucleotides as follows.

### (1) Transfection

A human colon cancer SW480 cell strain (derived from human adenocarcinoma of the large intestine) was adjusted to a concentration of 100000 cells/mL in an RPMI1640 medium (manufactured by Invitrogen Corp.) containing 10% fetal bovine serum. Then, the solution was seeded at a concentration of 1 mL/well onto a 12-well flat-bottomed plate (manufactured by Corning Inc.) and cultured at 37°C for 1 day under 5.0% CO₂ gas. A lipofection reagent Lipofectamine RNAiMAX (manufactured by Invitrogen Corp.) at a final concentration of 7.5 µL and a single-stranded or double-stranded polynucleotide solution at a final concentration of 0.3, 0.03, or 0.003 nM were mixed in an OPTI-MEM medium and left standing at room temperature for 20 minutes. The mixture was added to each well, and the culture was further continued for 3 days.

### (2) Real-time PCR

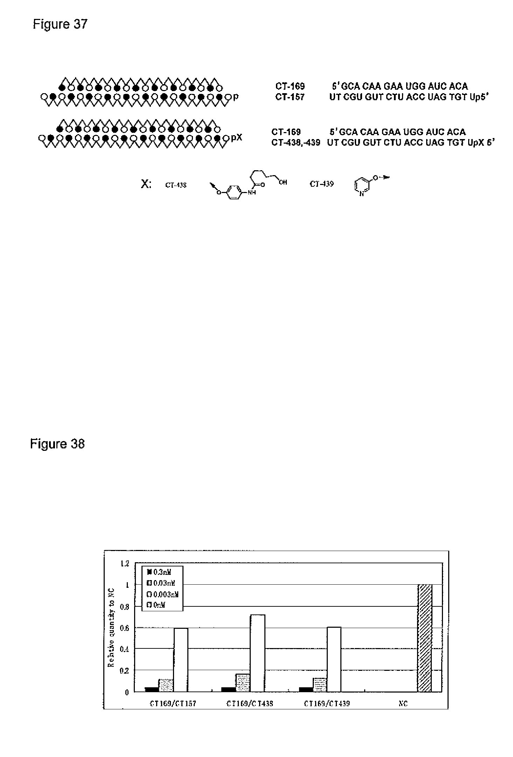

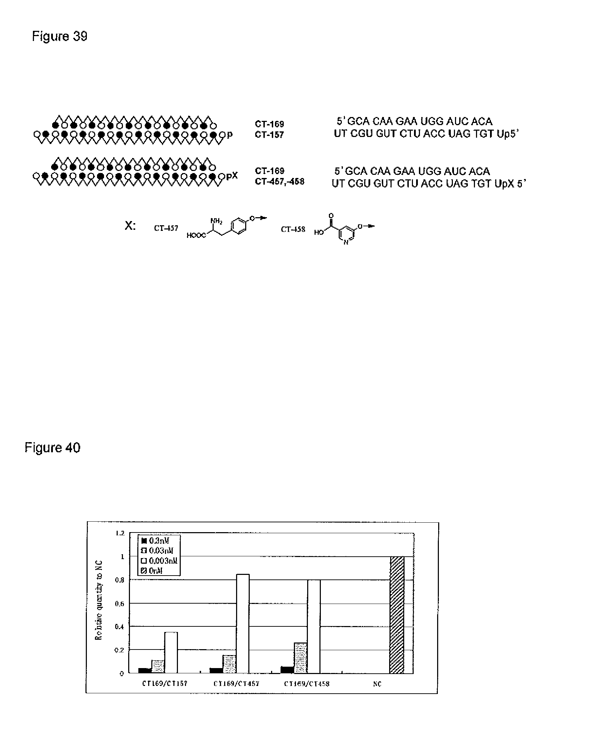

After the transfection, the culture supernatant was removed from each well, and mRNA was extracted using RNeasy Mini kit (manufactured by QIAGEN). cDNA was prepared from 0.5 µg of RNA using the obtained mRNA and iScript (TM) cDNA Synthesis kit (manufactured by QIAGEN) according to the description of the instruction. Next, the mRNA was quantified by real-time PCR as follows using PCR primers for the human β-catenin gene (primer set ID: HA135664, manufactured by TAKARA BIO INC.), PCR primers for the human GAPDH gene (primer set ID: HA067812, manufactured by TAKARA BIO INC.) as an internal standard, and a real-time PCR kit (manufactured by QIAGEN) containing reagents necessary for PCR. 25 µL of 2xQuantiTect SYBR GREEN PCR Master Mix included in the real-time PCR kit, 18 µL of RNase-Free Water, 5 µL each PCR primer (final concentration: 0.3 µM), and 2 µL of the prepared cDNA solution per well of a 96-well PCR plate (manufactured by Applied Biosystems) were added to bring the solution to the total volume of 50 µL. The plate was loaded in Mx3000P (manufactured by STRATAGENE), followed by PCR under the following conditions:
PCR initial activation at 95°C for 15 minutes
PCR at 94°C for 15 seconds
56°C for 30 seconds
72°C for 30 seconds
This PCR cycle was repeated 40 times. A calibration curve was prepared using 5-fold dilution series of cDNA prepared from mRNA extracted from cells (= NC) treated only with the lipofection reagent. Based on the calibration curve, human β-catenin and human GAPDH in each transfectant were quantified, and a relative amount determined by dividing the amount of the human catenin gene by the amount of human GAPDH was plotted in a graph. Real-time PCR was conducted on N=2, and average thereof is shown in the graph (the structures and nucleotide sequences of the double-stranded polynucleotides are shown in Figures 37 and 39).

### (3) Real-time PCR analysis

### (a) Analysis of gene inhibitory activities of double-stranded polynucleotides - 1 -

CT-169/CT-439 and CT-169/CT-458 in which a pyridylphosphate group having a substitution was bound to the 5'-end of the antisense strand in the double-stranded polynucleotide CT-169/CT-157, and CT-169/CT-457 in which tyrosine was bound to the 5'-end of the antisense strand in the double-stranded polynucleotide CT-169/CT-157 (for their structures, see Figures 37 and 39) were examined for their β-catenin gene expression inhibitory activities.

As shown in Figures 38 and 40, the double-stranded polynucleotides CT-169/CT-439, CT-169/CT-457, and CT-169/CT-458 strongly inhibited the expression of the β-catenin gene, as in CT-169/CT-157. This shows that a double-stranded polynucleotide having a pyridylphosphate group having a substituent, or tyrosine, at the 5'-end of the antisense strand has strong gene expression inhibitory activity.

### (b) Analysis of gene inhibitory activities of double-stranded polynucleotides - 2 -

A double-stranded polynucleotide CT-169/CT-438 in which 5'-end of the antisense strand in the double-stranded polynucleotide CT-169/CT-157 was modified with an aromatic substituent (for its structure, see Figure 37) was examined for its β-catenin gene expression inhibitory activity.

As shown in Figure 38, the double-stranded polynucleotide CT-169/CT-438 strongly inhibited the expression of the β-catenin gene, as in CT-169/CT-157.

### Industrial Applicability

The present invention could provide a double-stranded polynucleotide that has RNA interference effect and/or gene expression inhibitory effect. The present invention could also provide a double-stranded polynucleotide that is resistant to RNase and has RNA interference effect and/or gene expression inhibitory effect.

The double-stranded polynucleotide can be used in the functional analysis of genes, pharmaceutical compositions, etc. However, the industrial field of the present double-stranded polynucleotide is not limited as long as it can be used therein.

### Free Text of Sequence Listing

SEQ ID NO: 1: Sense strand
SEQ ID NO: 2: Antisense strand
SEQ ID NO: 3: Antisense strand
SEQ ID NO: 4: Antisense strand
SEQ ID NO: 5: Sense strand
SEQ ID NO: 6: Antisense strand
SEQ ID NO: 7: Sense strand
SEQ ID NO: 8: Antisense strand
SEQ ID NO: 9: Sense strand
SEQ ID NO: 10: Antisense strand
SEQ ID NO: 11: Antisense strand
SEQ ID NO: 12: Antisense strand
SEQ ID NO: 13: Sense strand
SEQ ID NO: 14: Sense strand
SEQ ID NO: 15: Antisense strand
SEQ ID NO: 16: Antisense strand
SEQ ID NO: 17: Sense strand
SEQ ID NO: 18: Sense strand
SEQ ID NO: 19: Antisense strand
SEQ ID NO: 20: Antisense strand
SEQ ID NO: 21: Antisense strand
SEQ ID NO: 22: Sense strand
SEQ ID NO: 23: Sense strand
SEQ ID NO: 24: Sense strand
SEQ ID NO: 25: Sense strand
SEQ ID NO: 26: Sense strand
SEQ ID NO: 27: Sense strand
SEQ ID NO: 28: Sense strand
SEQ ID NO: 29: Sense strand
SEQ ID NO: 30: Sense strand
SEQ ID NO: 31: Antisense strand
SEQ ID NO: 32: Antisense strand
SEQ ID NO: 33: Antisense strand
SEQ ID NO: 34: Antisense strand

## Claims

1. A double-stranded polynucleotide, or a salt thereof, the double-stranded polynucleotide having a sense strand polynucleotide consisting of a nucleotide sequence complementary to a target sequence in a target gene, and an antisense strand polynucleotide having a nucleotide sequence complementary to the sense strand polynucleotide, wherein a substituent represented by X is bound to a phosphate group at the 5'-end of the antisense strand polynucleotide to form a phosphodiester structure,
wherein
X represents:
(a) a substituent represented by the formula (I): 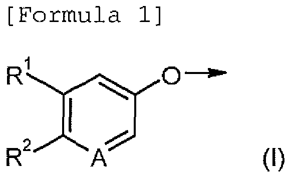
wherein A represents a nitrogen atom or C-R³,
R¹ and R² each independently represent
a hydrogen atom,
an alkyl group having 1 to 8 carbon atoms and optionally having a substituent,
an alkoxy group having 1 to 8 carbon atoms and optionally having a substituent,
a cycloalkyl group having 3 to 6 carbon atoms and
optionally having a substituent,
a halogen atom,
an alkylcarbonyl group containing an alkyl group having 1 to 8 carbon atoms and optionally having a substituent,
a phenyl group optionally having a substituent,
a phenyloxy group optionally having a substituent,
a saturated or unsaturated 5- or 6-membered heterocyclic group containing 1 to 3 heteroatoms selected from the heteroatom group consisting of a nitrogen atom, an oxygen atom, and a sulfur atom and optionally having a substituent,
an aralkyl group optionally having a substituent in the phenyl group moiety,
an aralkyloxy group optionally having a substituent in the phenyl group moiety,
an alkylsulfonyl group having 1 to 6 carbon atoms,
a hydroxy group,
an alkylcarbonylamino group having an alkyl group having 1 to 9 carbon atoms,
a hydroxyalkylcarbonylamino group having an alkyl group having 1 to 9 carbon atoms wherein the alkyl group is substituted by a hydroxy group on the terminal carbon atom thereof,
an N-alkylcarbamoyl group having an alkyl group having 1 to 8 carbon atoms,
an N-(hydroxyalkyl)carbamoyl group having an alkyl group having 1 to 9 carbon atoms wherein the alkyl group is substituted by a hydroxy group on the terminal carbon atom thereof,
an N-alkylcarbamoylalkylene group having an alkyl group having 1 to 8 carbon atoms and being bonded to the aromatic ring via an alkylene group containing 1 to 4 carbon atoms,
an N-(hydroxyalkyl)carbamoylalkylene group having an alkyl group having 1 to 9 carbon atoms wherein the alkyl group is substituted by a hydroxy group on the terminal carbon atom thereof, and being bonded to the aromatic ring via an alkylene group containing 1 to 4 carbon atoms,
a carboxy group, or
a group represented by the formula: -(CH₂)ₖ-CONR⁴R⁵,
wherein R⁴ and R⁵ each independently represent a hydrogen atom, an alkyl group having 1 to 6 carbon atoms and
optionally having a substituent, or an aralkyl group optionally having a substituent in the phenyl group moiety, and k represents an integer of 0 to 3, or
R¹ and R² are optionally combined to form, together with the aromatic ring bonded to R¹ and R², a saturated or
unsaturated bicyclic or tricyclic structure optionally having a substituent, optionally further containing 1 or 1 or more heteroatoms as constituent atoms in the ring, and optionally having an oxo group, and
R³ represents
a halogen atom,
an alkyl group having 1 to 6 carbon atoms,
an alkoxy group having 1 to 6 carbon atoms,
a halogenomethyl group,
a hydroxy group, or
a hydrogen atom; or
(b) a tyrosine residue optionally having a substituent on the amino group wherein a hydroxy group on the phenyl group is the phosphate group binding site.

2. The double-stranded polynucleotide or salt thereof according to claim 1, wherein in formula (I), A is C-R³.

3. The double-stranded polynucleotide or salt thereof according to claim 2, wherein R³ is a hydrogen atom.

4. The double-stranded polynucleotide or salt thereof according to any one of claims 1 to 3, wherein the sense strand polynucleotide consists of a polynucleotide represented by the following formula (II), the antisense strand polynucleotide consists of a polynucleotide represented by the following formula (III), and the double-stranded polynucleotide further has the following features (a) to (d):
5'₋(γ-β)₉-γ-λₘ-3' (II)
5'-X-β-(γ-β)₉-υₙ-3' (III),
(a) γ represents an RNA, β represents a 2'-OMeRNA, and λ and u each represent a DNA;
(b) m and n identically or differently represent any integer from 0 to 5;
(c) (γ-β)₉-γ in the polynucleotide represented by formula (II) has a nucleotide sequence identical to the target gene; and
(d) (γ-β)₉-γ in formula (II) and β-(γ-β)₉ in formula (III) have nucleotide sequences complementary to each other.

5. The double-stranded polynucleotide or salt thereof according to any one of claims 1 to 3, wherein the sense strand polynucleotide consists of a polynucleotide represented by the following formula (IV), the antisense strand polynucleotide consists of a polynucleotide represented by the following formula (V), and the double-stranded polynucleotide further has the following features (a) to (d):
5'-(α-β)₉-αₚ-λₘ-3' (IV)
5'-X-δₛ-(α-β)₉-υₙ-3' (V),
(a) α and β differently represent a DNA or a 2'-OMeRNA, δ and λ identically or differently represent a DNA or a 2'-OMeRNA, and u identically or differently represents any nucleotide selected from a DNA, an RNA, and a 2'-OMeRNA;
(b) p represents an integer of 0 or 1, m is 0 when p is 0 and represents any integer from 0 to 5 when p is 1, s represents an integer of 0 or 1, and n represents any integer from 0 to 5;
(c) (α-β)₉-αₚ in the polynucleotide represented by formula (IV) has a nucleotide sequence identical to the target gene; and
(d) (α-β)₉ in formula (IV) and (α-β)₉ in formula (V) have nucleotide sequences complementary to each other.

6. The double-stranded polynucleotide or a salt thereof according to any one of claims 1 to 3, wherein the sense strand polynucleotide consists of a polynucleotide represented by the following formula (VI), the antisense strand polynucleotide consists of a polynucleotide represented by the following formula (VII), and the double-stranded polynucleotide further has the following features (a) to (d):
5'-β-(α-β)₈-αₚ-λₘ-3' (VI)
5'-X-δₛ-(α-β)₈-(α-β)-υₙ-3' (VII),
(a) α and β differently represent a DNA or a 2'-OMeRNA, δ and λ identically or differently represent a DNA or a 2'-OMeRNA, and u identically or differently represents any nucleotide selected from a DNA, an RNA, and a 2'-OMeRNA;
(b) p represents an integer of 0 or 1, m is 0 when p is 0 and represents any integer from 0 to 5 when p is 1, s represents an integer of 0 or 1, and n represents any integer from 0 to 5;
(c) β-(α-β)₈-αₚ in the polynucleotide represented by formula (VI) has a nucleotide sequence identical to the target gene; and
(d) (α-β)₈ in formula (VI) and (α-β)₈ in formula (VII) have nucleotide sequences complementary to each other.

7. The double-stranded polynucleotide or salt thereof according to claim 5 or 6, wherein α is a DNA, and β is a 2'-OMeRNA.

8. The double-stranded polynucleotide or salt thereof according to any one of claims 4 to 7, wherein λₘ and υₙ are identically or differently any of: DNAs having a thymine base, an adenine base, or a guanine base; or 2'-OMeRNAs having a uracil base, an adenine base, or a guanine base.

9. The double-stranded polynucleotide or salt thereof according to any one of claims 4 to 8, wherein m is 0, and n is 2.

10. The double-stranded polynucleotide or salt thereof according to any one of claims 5 to 9, wherein p and m are 0, s is 1, and n is 2.

11. The double-stranded polynucleotide or salt thereof according to any one of claims 5 to 9, wherein p and m are 0, s is 0 or 1, n is 2, and υ₂ is a DNA or a 2'-OMeRNA.

12. The double-stranded polynucleotide or salt thereof according to any one of claims 4 to 11, wherein between 1 and 4 2'-OMeRNA nucleotides are substituted by an ENA or a 2',4'-BNA/LNA.

13. The double-stranded polynucleotide or salt thereof according to any one of claims 4 to 12, wherein between 1 and 4 DNA nucleotides are substituted by an RNA, an ENA or a 2',4'-BNA/LNA.

14. The double-stranded polynucleotide or salt thereof according to any one of claims 1 to 13, wherein the nucleotides are bonded to each other via a phosphodiester or phosphorothioate bond.

15. A pharmaceutical composition comprising a double-stranded polynucleotide or salt thereof according to any one of claims 1 to 14 as an active ingredient.

16. A method for inhibiting the expression of a target gene, comprising administering a double-stranded polynucleotide or salt thereof selected from claims 1 to 15 to a mammal.


SEQUENCE LISTING

<110>  DAIICHI SANKYO COMPANY, LTD.

<120>  modified double strand polynucleotides

<130>  DSPCT-FP1040

<150>  JP 2009-249998
<151>  2009-10-30

<150>  JP 2009-298719
<151>  2009-12-28

<160>  34

<170>  PatentIn version 3.4

<210>  1
<211>  18
<212>  DNA
<213>  Artificial Sequence

<220>
<223>  sense strand



<220>
<221>  modified_base
<222>  (2)..(2)
<223>  cm

<220>
<221>  modified_base
<222>  (4)..(4)
<223>  cm

<220>
<221>  modified_base
<222>  (6)..(6)
<223>  2'-O-methyladenosine

<220>
<221>  modified_base
<222>  (8)..(8)
<223>  2'-O-methyladenosine

<220>
<221>  modified_base
<222>  (10)..(10)
<223>  um

<220>
<221>  modified_base
<222>  (12)..(12)
<223>  gm

<220>
<221>  modified_base
<222>  (14)..(14)
<223>  um

<220>
<221>  modified_base
<222>  (16)..(16)

<223>  2'-O-methyladenosine

<220>
<221>  modified_base
<222>  (18)..(18)
<223>  2'-O-methyladenosine

<400>  1
gcacaagaau ggaucaca                                                    18


<210>  2
<211>  20
<212>  DNA
<213>  Artificial Sequence

<220>
<223>  antisense strand



<220>
<221>  modified_base
<222>  (2)..(2)
<223>  gm

<220>
<221>  modified_base
<222>  (4)..(4)
<223>  gm

<220>
<221>  modified_base
<222>  (6)..(6)
<223>  um

<220>
<221>  modified_base
<222>  (8)..(8)
<223>  cm

<220>
<221>  modified_base
<222>  (10)..(10)
<223>  um

<220>
<221>  modified_base
<222>  (12)..(12)
<223>  cm

<220>
<221>  modified_base
<222>  (14)..(14)
<223>  um

<220>
<221>  modified_base
<222>  (16)..(16)
<223>  um

<220>
<221>  modified_base
<222>  (18)..(18)
<223>  cm

<220>
<221>  modified_base
<222>  (20)..(20)
<223>  um

<400>  2
tgtgauccau tctugugctu                                                    20


<210>  3
<211>  21
<212>  DNA
<213>  Artificial Sequence

<220>
<223>  antisense strand


<220>
<221>  modified_base
<222>  (1)..(1)
<223>  um

<220>
<221>  modified_base
<222>  (3)..(3)
<223>  gm

<220>
<221>  modified_base
<222>  (5)..(5)
<223>  gm

<220>
<221>  modified_base
<222>  (7)..(7)
<223>  um

<220>
<221>  modified_base
<222>  (9)..(9)
<223>  cm

<220>
<221>  modified_base
<222>  (11)..(11)
<223>  um

<220>
<221>  modified_base
<222>  (13)..(13)
<223>  cm

<220>
<221>  modified_base
<222>  (15)..(15)
<223>  um

<220>
<221>  modified_base
<222>  (17)..(17)
<223>  um

<220>
<221>  modified_base

<222>  (19)..(19)
<223>  cm

<220>
<221>  modified_base
<222>  (21)..(21)
<223>  um

<400>  3
utgtgaucca utctugugct u                                                21


<210>  4
<211>  21
<212>  RNA
<213>  Artificial Sequence

<220>
<223>  antisense strand

<400>  4
uugugaucca uucuugugcu u                                                21


<210>  5
<211>  18
<212>  DNA
<213>  Artificial Sequence

<220>
<223>  sense strand


<220>
<221>  modified_base
<222>  (2)..(2)
<223>  cm

<220>
<221>  modified_base
<222>  (4)..(4)
<223>  cm

<220>
<221>  modified_base
<222>  (6)..(6)
<223>  2'-O-methyladenosine

<220>
<221>  modified_base
<222>  (8)..(8)
<223>  2'-O-methyladenosine

<220>
<221>  modified_base
<222>  (10)..(10)
<223>  um

<220>
<221>  modified_base
<222>  (12)..(12)
<223>  gm

<220>
<221>  modified_base

<222>  (14)..(14)
<223>  um

<220>
<221>  modified_base
<222>  (16)..(16)
<223>  2'-O-methyladenosine

<220>
<221>  modified_base
<222>  (18)..(18)
<223>  2'-O-methyladenosine

<400>  5
gcacaagaau ggaucaca                                                    18


<210>  6
<211>  21
<212>  DNA
<213>  Artificial Sequence

<220>
<223>  antisense strand


<220>
<221>  modified_base
<222>  (1)..(1)
<223>  um

<220>
<221>  modified_base
<222>  (3)..(3)
<223>  gm

<220>
<221>  modified_base
<222>  (5)..(5)
<223>  gm

<220>
<221>  modified_base
<222>  (7)..(7)
<223>  um

<220>
<221>  modified_base
<222>  (9)..(9)
<223>  cm

<220>
<221>  modified_base
<222>  (11)..(11)
<223>  um

<220>
<221>  modified_base
<222>  (13)..(13)
<223>  cm

<220>
<221>  modified_base
<222>  (15)..(15)
<223>  um

<220>
<221>  modified_base
<222>  (17)..(17)
<223>  um

<220>
<221>  modified_base
<222>  (19)..(19)
<223>  cm

<220>
<221>  modified_base
<222>  (21)..(21)
<223>  um

<400>  6
utgtgaucca utctugugct u                                                21


<210>  7
<211>  21
<212>  DNA
<213>  Artificial Sequence

<220>
<223>  sense strand


<220>
<221>  modified_base
<222>  (2)..(2)
<223>  cm

<220>
<221>  modified_base
<222>  (4)..(4)
<223>  cm

<220>
<221>  modified_base
<222>  (6)..(6)
<223>  2'-O-methyladenosine

<220>
<221>  modified_base
<222>  (8)..(8)
<223>  2'-O-methyladenosine

<220>
<221>  modified_base
<222>  (10)..(10)
<223>  um

<220>
<221>  modified_base
<222>  (12)..(12)
<223>  gm

<220>
<221>  modified_base
<222>  (14)..(14)
<223>  um

<220>

<221>  modified_base
<222>  (16)..(16)
<223>  2'-O-methyladenosine

<220>
<221>  modified_base
<222>  (18)..(18)
<223>  2'-O-methyladenosine

<400>  7
gcacaagaau ggaucacaat t                                                21


<210>  8
<211>  21
<212>  DNA
<213>  Artificial Sequence

<220>
<223>  antisense strand


<220>
<221>  modified_base
<222>  (1)..(1)
<223>  um

<220>
<221>  modified_base
<222>  (3)..(3)
<223>  gm

<220>
<221>  modified_base
<222>  (5)..(5)
<223>  gm

<220>
<221>  modified_base
<222>  (7)..(7)
<223>  um

<220>
<221>  modified_base
<222>  (9)..(9)
<223>  cm

<220>
<221>  modified_base
<222>  (11)..(11)
<223>  um

<220>
<221>  modified_base
<222>  (13)..(13)
<223>  cm

<220>
<221>  modified_base
<222>  (15)..(15)
<223>  um

<220>
<221>  modified_base
<222>  (17)..(17)

<223>  um

<220>
<221>  modified_base
<222>  (19)..(19)
<223>  cm

<400>  8
uugugaucca uucuugugct t                                                21


<210>  9
<211>  21
<212>  RNA
<213>  Artificial Sequence

<220>
<223>  sense strand

<400>  9
gcacaagaau ggaucacaau u                                                21


<210>  10
<211>  21
<212>  RNA
<213>  Artificial Sequence

<220>
<223>  antisense strand

<400>  10
uugugaucca uucuugugcu u                                                21


<210>  11
<211>  21
<212>  DNA
<213>  Artificial Sequence

<220>
<223>  antisense strand


<220>
<221>  modified_base
<222>  (1)..(1)
<223>  um

<220>
<221>  modified_base
<222>  (3)..(3)
<223>  gm

<220>
<221>  modified_base
<222>  (5)..(5)
<223>  gm

<220>
<221>  modified_base
<222>  (7)..(7)
<223>  um

<220>

<221>  modified_base
<222>  (9)..(9)
<223>  cm

<220>
<221>  modified_base
<222>  (11)..(11)
<223>  um

<220>
<221>  modified_base
<222>  (13)..(13)
<223>  cm

<220>
<221>  modified_base
<222>  (15)..(15)
<223>  um

<220>
<221>  modified_base
<222>  (17)..(17)
<223>  um

<220>
<221>  modified_base
<222>  (19)..(19)
<223>  cm

<220>
<221>  modified_base
<222>  (21)..(21)
<223>  um

<400>  11
utgtgaucca utctugugct u                                                21


<210>  12
<211>  20
<212>  DNA
<213>  Artificial Sequence

<220>
<223>  antisense strand


<220>
<221>  modified_base
<222>  (2)..(2)
<223>  gm

<220>
<221>  modified_base
<222>  (4)..(4)
<223>  gm

<220>
<221>  modified_base
<222>  (6)..(6)
<223>  um

<220>
<221>  modified_base
<222>  (8)..(8)

<223>  cm

<220>
<221>  modified_base
<222>  (10)..(10)
<223>  um

<220>
<221>  modified_base
<222>  (12)..(12)
<223>  cm

<220>
<221>  modified_base
<222>  (14)..(14)
<223>  um

<220>
<221>  modified_base
<222>  (16)..(16)
<223>  um

<220>
<221>  modified_base
<222>  (18)..(18)
<223>  cm

<220>
<221>  modified_base
<222>  (20)..(20)
<223>  um

<400>  12
tgtgauccau tctugugctu                                                    20


<210>  13
<211>  21
<212>  DNA
<213>  Artificial Sequence

<220>
<223>  sense strand

<400>  13
ggacaaggaa gcugcagaat t                                                  21


<210>  14
<211>  18
<212>  DNA
<213>  Artificial Sequence

<220>
<223>  sense strand


<220>
<221>  modified_base
<222>  (2)..(2)
<223>  gm

<220>
<221>  modified_base
<222>  (4)..(4)

<223>  cm

<220>
<221>  modified_base
<222>  (6)..(6)
<223>  2'-O-methyladenosine

<220>
<221>  modified_base
<222>  (8)..(8)
<223>  gm

<220>
<221>  modified_base
<222>  (10)..(10)
<223>  2'-O-methyladenosine

<220>
<221>  modified_base
<222>  (12)..(12)
<223>  cm

<220>
<221>  modified_base
<222>  (14)..(14)
<223>  gm

<220>
<221>  modified_base
<222>  (16)..(16)
<223>  2'-O-methyladenosine

<220>
<221>  modified_base
<222>  (18)..(18)
<223>  2'-O-methyladenosine

<400>  14
ggacaaggaa gctgcaga                                                    18


<210>  15
<211>  21
<212>  DNA
<213>  Artificial Sequence

<220>
<223>  antisense strand

<400>  15
uucugcagcu uccuugucct t                                                21


<210>  16
<211>  21
<212>  DNA
<213>  Artificial Sequence

<220>
<223>  antisense strand


<220>
<221>  modified_base
<222>  (3)..(3)

<223>  cm

<220>
<221>  modified_base
<222>  (5)..(5)
<223>  gm

<220>
<221>  modified_base
<222>  (7)..(7)
<223>  2'-O-methyladenosine

<220>
<221>  modified_base
<222>  (9)..(9)
<223>  cm

<220>
<221>  modified_base
<222>  (11)..(11)
<223>  um

<220>
<221>  modified_base
<222>  (13)..(13)
<223>  cm

<220>
<221>  modified_base
<222>  (15)..(15)
<223>  um

<220>
<221>  modified_base
<222>  (17)..(17)
<223>  um

<220>
<221>  modified_base
<222>  (19)..(19)
<223>  cm

<220>
<221>  modified_base
<222>  (21)..(21)
<223>  um

<400>  16
ttctgcagct ucctugucct u                                                21


<210>  17
<211>  21
<212>  DNA
<213>  Artificial Sequence

<220>
<223>  sense strand

<400>  17
ccgccgaauu cauuaauuut t                                                21


<210>  18
<211>  18

<212>  DNA
<213>  Artificial Sequence

<220>
<223>  sense strand



<220>
<221>  modified_base
<222>  (2)..(2)
<223>  cm

<220>
<221>  modified_base
<222>  (4)..(4)
<223>  cm

<220>
<221>  modified_base
<222>  (6)..(6)
<223>  gm

<220>
<221>  modified_base
<222>  (8)..(8)
<223>  2'-O-methyladenosine

<220>
<221>  modified_base
<222>  (10)..(10)
<223>  um

<220>
<221>  modified_base
<222>  (12)..(12)
<223>  2'-O-methyladenosine

<220>
<221>  modified_base
<222>  (14)..(14)
<223>  um

<220>
<221>  modified_base
<222>  (16)..(16)
<223>  2'-O-methyladenosine

<220>
<221>  modified_base
<222>  (18)..(18)
<223>  um

<400>  18
ccgccgaatu catuaatu                                                    18


<210>  19
<211>  21
<212>  DNA
<213>  Artificial Sequence

<220>
<223>  antisense strand

<400>  19

aaauuaauga auucggcggg t                                              21

<210>  20
<211>  21
<212>  DNA
<213>  Artificial Sequence

<220>
<223>  antisense strand

<220>
<221>  modified_base
<222>  (1)..(1)
<223>  2'-O-methyladenosine

<220>
<221>  modified_base
<222>  (3)..(3)
<223>  2'-O-methyladenosine

<220>
<221>  modified_base
<222>  (5)..(5)
<223>  um

<220>
<221>  modified_base
<222>  (7)..(7)
<223>  2'-O-methyladenosine

<220>
<221>  modified_base
<222>  (9)..(9)
<223>  gm

<220>
<221>  modified_base
<222>  (11)..(11)
<223>  2'-O-methyladenosine

<220>
<221>  modified_base
<222>  (13)..(13)
<223>  um

<220>
<221>  modified_base
<222>  (15)..(15)
<223>  gm

<220>
<221>  modified_base
<222>  (17)..(17)
<223>  cm

<220>
<221>  modified_base
<222>  (19)..(19)
<223>  gm

<220>
<221>  modified_base
<222>  (21)..(21)

<223>  um

<400>  20
aaatuaatga atucggcggg u                                                21


<210>  21
<211>  21
<212>  DNA
<213>  Artificial Sequence

<220>
<223>  antisense strand


<220>
<221>  modified_base
<222>  (1)..(1)
<223>  um

<220>
<221>  modified_base
<222>  (3)..(3)
<223>  gm

<220>
<221>  modified_base
<222>  (5)..(5)
<223>  gm

<220>
<221>  modified_base
<222>  (7)..(7)
<223>  um

<220>
<221>  modified_base
<222>  (9)..(9)
<223>  cm

<220>
<221>  modified_base
<222>  (11)..(11)
<223>  um

<220>
<221>  modified_base
<222>  (13)..(13)
<223>  cm

<220>
<221>  modified_base
<222>  (15)..(15)
<223>  um

<220>
<221>  modified_base
<222>  (17)..(17)
<223>  um

<220>
<221>  modified_base
<222>  (19)..(19)
<223>  cm

<220>
<221>  modified_base
<222>  (21)..(21)
<223>  um

<400>  21
utgtgaucca utctugugct u                                                21


<210>  22
<211>  18
<212>  DNA
<213>  Artificial Sequence

<220>
<223>  sense strand



<220>
<221>  modified_base
<222>  (2)..(2)
<223>  2'-O,4'-C-ethylenecytidine

<220>
<221>  modified_base
<222>  (4)..(4)
<223>  2'-O-methyladenosine

<220>
<221>  modified_base
<222>  (6)..(6)
<223>  2'-O-methyladenosine

<220>
<221>  modified_base
<222>  (8)..(8)
<223>  2'-O-methyladenosine

<220>
<221>  modified_base
<222>  (10)..(10)
<223>  um

<220>
<221>  modified_base
<222>  (12)..(12)
<223>  gm

<220>
<221>  modified_base
<222>  (14)..(14)
<223>  um

<220>
<221>  modified_base
<222>  (16)..(16)
<223>  2'-O-methyladenosine

<220>
<221>  modified_base
<222>  (18)..(18)
<223>  2'-O-methyladenosine

<400>  22
gcaaaagaau ggaucaca                                                    18

<210>  23
<211>  18
<212>  DNA
<213>  Artificial Sequence

<220>
<223>  sense strand

<220>
<221>  modified_base
<222>  (2)..(2)
<223>  2'-O,4'-C-ethylenecytidine

<220>
<221>  modified_base
<222>  (4)..(4)
<223>  2'-O,4'-C-ethylenecytidine

<220>
<221>  modified_base
<222>  (6)..(6)
<223>  2'-O-methyladenosine

<220>
<221>  modified_base
<222>  (8)..(8)
<223>  2'-O-methyladenosine

<220>
<221>  modified_base
<222>  (10)..(10)
<223>  um

<220>
<221>  modified_base
<222>  (12)..(12)
<223>  gm

<220>
<221>  modified_base
<222>  (14)..(14)
<223>  um

<220>
<221>  modified_base
<222>  (16)..(16)
<223>  2'-O-methyladenosine

<220>
<221>  modified_base
<222>  (18)..(18)
<223>  2'-O-methyladenosine

<400>  23
gcacaagaau ggaucaca                                                    18

<210>  24
<211>  18
<212>  DNA
<213>  Artificial Sequence

<220>
<223>  sense strand


<220>
<221>  modified_base
<222>  (2)..(2)
<223>  2'-O,4'-C-ethylenecytidine

<220>
<221>  modified_base
<222>  (4)..(4)
<223>  2'-O,4'-C-ethylenecytidine

<220>
<221>  modified_base
<222>  (6)..(6)
<223>  2'-O,4'-C-ethyleneadenosine

<220>
<221>  modified_base
<222>  (8)..(8)
<223>  2'-O-methyladenosine

<220>
<221>  modified_base
<222>  (10)..(10)
<223>  um

<220>
<221>  modified_base
<222>  (12)..(12)
<223>  gm

<220>
<221>  modified_base
<222>  (14)..(14)
<223>  um

<220>
<221>  modified_base
<222>  (16)..(16)
<223>  2'-O-methyladenosine

<220>
<221>  modified_base
<222>  (18)..(18)
<223>  2'-O-methyladenosine

<400>  24
gcacaagaau ggaucaca                                                    18


<210>  25
<211>  18
<212>  DNA
<213>  Artificial Sequence

<220>
<223>  sense strand


<220>
<221>  modified_base
<222>  (2)..(2)

<223>  2'-O,4'-C-ethylenecytidine

<220>
<221>  modified_base
<222>  (4)..(4)
<223>  2'-O,4'-C-ethylenecytidine

<220>
<221>  modified_base
<222>  (6)..(6)
<223>  2'-O,4'-C-ethyleneadenosine

<220>
<221>  modified_base
<222>  (8)..(8)
<223>  2'-O,4'-C-ethyleneadenosine

<220>
<221>  modified_base
<222>  (10)..(10)
<223>  um

<220>
<221>  modified_base
<222>  (12)..(12)
<223>  gm

<220>
<221>  modified_base
<222>  (14)..(14)
<223>  um

<220>
<221>  modified_base
<222>  (16)..(16)
<223>  2'-O-methyladenosine

<220>
<221>  modified_base
<222>  (18)..(18)
<223>  2'-O-methyladenosine

<400>  25
gcacaagaau ggaucaca                                                    18


<210>  26
<211>  18
<212>  DNA
<213>  Artificial Sequence

<220>
<223>  sense strand



<220>
<221>  modified_base
<222>  (2)..(2)
<223>  2'-O,4'-C-ethylenecytidine

<220>
<221>  modified_base
<222>  (4)..(4)
<223>  2'-O,4'-C-ethylenecytidine

<220>
<221>  modified_base
<222>  (6)..(6)
<223>  2'-O,4'-C-ethyleneadenosine

<220>
<221>  modified_base
<222>  (8)..(8)
<223>  2'-O,4'-C-ethyleneadenosine

<220>
<221>  modified_base
<222>  (10)..(10)
<223>  2'-O,4'-C-ethyleneuridine

<220>
<221>  modified_base
<222>  (12)..(12)
<223>  gm

<220>
<221>  modified_base
<222>  (14)..(14)
<223>  um

<220>
<221>  modified_base
<222>  (16)..(16)
<223>  2'-O-methyladenosine

<220>
<221>  modified_base
<222>  (18)..(18)
<223>  2'-O-methyladenosine

<400>  26
gcacaagaau ggaucaca                                                    18


<210>  27
<211>  18
<212>  DNA
<213>  Artificial Sequence

<220>
<223>  sense strand


<220>
<221>  modified_base
<222>  (2)..(2)
<223>  2'-O,4'-C-ethylenecytidine

<220>
<221>  modified_base
<222>  (4)..(4)
<223>  2'-O,4'-C-ethylenecytidine

<220>
<221>  modified_base
<222>  (6)..(6)
<223>  2'-O,4'-C-ethyleneadenosine

<220>
<221>  modified_base

<222>  (8)..(8)
<223>  2'-O,4'-C-ethyleneadenosine

<220>
<221>  modified_base
<222>  (10)..(10)
<223>  2'-O,4'-C-ethyleneuridine

<220>
<221>  modified_base
<222>  (12)..(12)
<223>  2'-O,4'-C-ethyleneur

<220>
<221>  modified_base
<222>  (12)..(12)
<223>  2'-O,4'-C-ethyleneguanosine

<220>
<221>  modified_base
<222>  (14)..(14)
<223>  um

<220>
<221>  modified_base
<222>  (16)..(16)
<223>  2'-O-methyladenosine

<220>
<221>  modified_base
<222>  (18)..(18)
<223>  2'-O-methyladenosine

<400>  27
gcacaagaau ggaucaca                                                    18


<210>  28
<211>  18
<212>  DNA
<213>  Artificial Sequence

<220>
<223>  sense strand


<220>
<221>  modified_base
<222>  (2)..(2)
<223>  2'-O,4'-C-ethylenecytidine

<220>
<221>  modified_base
<222>  (4)..(4)
<223>  2'-O,4'-C-ethylenecytidine

<220>
<221>  modified_base
<222>  (6)..(6)
<223>  2'-O,4'-C-ethyleneadenosine

<220>
<221>  modified_base
<222>  (8)..(8)
<223>  2'-O,4'-C-ethyleneadenosine

<220>
<221>  modified_base
<222>  (10)..(10)
<223>  2'-O,4'-C-ethyleneuridine

<220>
<221>  modified_base
<222>  (12)..(12)
<223>  2'-O,4'-C-ethyleneguanosine

<220>
<221>  modified_base
<222>  (14)..(14)
<223>  2'-O,4'-C-ethyleneuridine

<220>
<221>  modified_base
<222>  (16)..(16)
<223>  2'-O-methyladenosine

<220>
<221>  modified_base
<222>  (18)..(18)
<223>  2'-O-methyladenosine

<400>  28
gcacaagaau ggaucaca                                                    18


<210>  29
<211>  18
<212>  DNA
<213>  Artificial Sequence

<220>
<223>  sense strand



<220>
<221>  modified_base
<222>  (2)..(2)
<223>  2'-O,4'-C-ethylenecytidine

<220>
<221>  modified_base
<222>  (4)..(4)
<223>  2'-O,4'-C-ethylenecytidine

<220>
<221>  modified_base
<222>  (6)..(6)
<223>  2'-O,4'-C-ethyleneadenosine

<220>
<221>  modified_base
<222>  (8)..(8)
<223>  2'-O,4'-C-ethyleneadenosine

<220>
<221>  modified_base
<222>  (10)..(10)
<223>  2'-O,4'-C-ethyleneuridine

<220>

<221>  modified_base
<222>  (12)..(12)
<223>  2'-O,4'-C-ethyleneguanosine

<220>
<221>  modified_base
<222>  (14)..(14)
<223>  2'-O,4'-C-ethyleneuridine

<220>
<221>  modified_base
<222>  (16)..(16)
<223>  2'-O,4'-C-ethyleneadenosine

<220>
<221>  modified_base
<222>  (18)..(18)
<223>  2'-O-methyladenosine

<400>  29
gcacaagaau ggaucaca                                                    18


<210>  30
<211>  18
<212>  DNA
<213>  Artificial Sequence

<220>
<223>  sense strand



<220>
<221>  modified_base
<222>  (2)..(2)
<223>  2'-O,4'-C-ethylenecytidine

<220>
<221>  modified_base
<222>  (4)..(4)
<223>  2'-O,4'-C-ethylenecytidine

<220>
<221>  modified_base
<222>  (6)..(6)
<223>  2'-O,4'-C-ethyleadenosine

<220>
<221>  modified_base
<222>  (8)..(8)
<223>  2'-O,4'-C-ethyleadenosine

<220>
<221>  modified_base
<222>  (10)..(10)
<223>  2'-O,4'-C-ethyleuridine

<220>
<221>  modified_base
<222>  (12)..(12)
<223>  2'-O,4'-C-ethyleneguanosine

<220>
<221>  modified_base
<222>  (14)..(14)

<223>  2'-O,4'-C-ethyleneuridine

<220>
<221>  modified_base
<222>  (16)..(16)
<223>  2'-O,4'-C-ethyleneadenosine

<220>
<221>  modified_base
<222>  (18)..(18)
<223>  2'-O,4'-C-ethyleneadenosine

<400>  30
gcacaagaau ggaucaca                                                    18


<210>  31
<211>  21
<212>  DNA
<213>  Artificial Sequence

<220>
<223>  antisense strand

<400>  31
uucugcagcu uccuugucct t                                                21


<210>  32
<211>  21
<212>  DNA
<213>  Artificial Sequence

<220>
<223>  antisense strand


<220>
<221>  modified_base
<222>  (3)..(3)
<223>  cm

<220>
<221>  modified_base
<222>  (5)..(5)
<223>  gm

<220>
<221>  modified_base
<222>  (7)..(7)
<223>  2'-O-methyladenosine

<220>
<221>  modified_base
<222>  (9)..(9)
<223>  cm

<220>
<221>  modified_base
<222>  (11)..(11)
<223>  um

<220>
<221>  modified_base
<222>  (13)..(13)

<223>  cm

<220>
<221>  modified_base
<222>  (15)..(15)
<223>  um

<220>
<221>  modified_base
<222>  (17)..(17)
<223>  um

<220>
<221>  modified_base
<222>  (19)..(19)
<223>  cm

<220>
<221>  modified_base
<222>  (21)..(21)
<223>  um

<400>  32
ttctgcagct ucctugucct u                                                21


<210>  33
<211>  21
<212>  DNA
<213>  Artificial Sequence

<220>
<223>  antisense atrand

<400>  33
aaauuaauga auucggcggg t                                                21


<210>  34
<211>  21
<212>  DNA
<213>  Artificial Sequence

<220>
<223>  antisense strand


<220>
<221>  modified_base
<222>  (1)..(1)
<223>  2'-O-methyladenosine

<220>
<221>  modified_base
<222>  (3)..(3)
<223>  2'-O-methyladenosine

<220>
<221>  modified_base
<222>  (5)..(5)
<223>  um

<220>
<221>  modified_base
<222>  (7)..(7)

<223>  2′-O-methyladenosine

<220>
<221>  modified_base
<222>  (9)..(9)
<223>  gm

<220>
<221>  modified_base
<222>  (11)..(11)
<223>  2′-O-methyladenosine

<220>
<221>  modified_base
<222>  (13)..(13)
<223>  um

<220>
<221>  modified_base
<222>  (15)..(15)
<223>  gm

<220>
<221>  modified_base
<222>  (17)..(17)
<223>  cm

<220>
<221>  modified_base
<222>  (19)..(19)
<223>  gm

<220>
<221>  modified_base
<222>  (21)..(21)
<223>  um

<400>  34
aaatuaatga atucggcggg u                                                    21